(19) Europäisches Patentamt
European Patent Office
Office européen des brevets

(11) EP 1 872 173 B1

(12) **EUROPEAN PATENT SPECIFICATION**

(45) Date of publication and mention of the grant of the patent:
**14.04.2010 Bulletin 2010/15**

(21) Application number: **06735605.5**

(22) Date of filing: **21.02.2006**

(51) Int Cl.:
***G03C 1/73*** *(2006.01)* ***C09K 9/02*** *(2006.01)*
***G02B 5/23*** *(2006.01)* ***C07D 279/06*** *(2006.01)*
***C07D 311/92*** *(2006.01)* ***C07D 319/04*** *(2006.01)*
***C07D 339/06*** *(2006.01)* ***C07D 339/08*** *(2006.01)*

(86) International application number:
**PCT/US2006/006016**

(87) International publication number:
**WO 2006/110221 (19.10.2006 Gazette 2006/42)**

(54) **PHOTOCHROMIC MATERIALS HAVING EXTENDED PI-CONJUGATED SYSTEMS AND COMPOSITIONS AND ARTICLES INCLUDING THE SAME**

FOTOCHROME MATERIALIEN MIT ERWEITERTEN PI-KONJUGIERTEN SYSTEMEN UND ZUSAMMENSETZUNGEN UND ARTIKEL DAMIT

MATERIAUX PHOTOCHROMIQUES PRESENTANT DES SYSTEMES $G(P)-CONJUGUES ETENDUS ET COMPOSITIONS ET ARTICLES LES COMPRENANT

(84) Designated Contracting States:
**DE ES FR GB IT**

(30) Priority: **08.04.2005 US 102279**

(43) Date of publication of application:
**02.01.2008 Bulletin 2008/01**

(73) Proprietor: **TRANSITIONS OPTICAL, INC.**
**Pinellas Park, FL 33782 (US)**

(72) Inventors:
- **KIM, Beon-kyu**
  **Gibsonia, Pennsylvania 15044 (US)**
- **DENG, Jun**
  **Murrysville, Pennsylvania 15668 (US)**
- **XIAO, Wenjing**
  **Murrysville, Pennsylvania 15668 (US)**
- **VAN GEMERT, Barry**
  **Pitcairn, Pennsylvania 15140 (US)**
- **CHOPRA, Anu**
  **Pittsburgh, Pennsylvania 15239 (US)**

(74) Representative: **Lüdcke, Joachim Moritz et al**
**Fleischer, Godemeyer, Kierdorf**
**& Partner, Patentanwälte**
**polypatent**
**Braunsberger Feld 29**
**D-51429 Bergisch Gladbach (DE)**

(56) References cited:
**WO-A-01/19813** **WO-A-97/37254**
**WO-A-97/40409** **WO-A-97/48762**
**WO-A-99/15518** **WO-A-03/056390**
**JP-A- 2004 131 593** **US-A- 5 645 767**
**US-A- 5 961 892** **US-A1- 2001 025 948**
**US-A1- 2003 071 247** **US-A1- 2004 185 255**
**US-A1- 2004 185 268** **US-A1- 2004 186 241**
**US-A1- 2004 191 520** **US-B1- 6 225 466**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 872 173 B1

## Description

BACKGROUND

Various non-limiting embodiments disclosed herein relate to photochromic materials having an extended pi-conjugated system. Other non-limiting embodiments relate to photochromic compositions and articles, such as optical elements, incorporating the same.

Many conventional photochromic materials, such as indeno-fused naphthopyrans, can undergo a transformation in response to certain wavelengths of electromagnetic radiation (or "actinic radiation") from one form (or state) to another, with each form having a characteristic absorption spectrum. As used herein the term "actinic radiation" refers to electromagnetic radiation that is capable of causing a photochromic material to transform from one form or state to another. For example, many conventional photochromic materials are capable of transforming from a closed-form, corresponding to a "bleached" or "unactivated" state of the photochromic material, to an open-form, corresponding to a "colored" or "activated" state of the photochromic material, in response to actinic radiation, and reverting back to the closed-form in the absence of the actinic radiation in response to thermal energy. Photochromic compositions and articles that contain one or more photochromic materials, for example photochromic lenses for eyewear applications, may display clear and colored states that generally correspond to the states of the photochromic material(s) that they contain.

Typically, the amount of a photochromic material needed to achieve a desired optical effect when incorporated into a composition or article will depend, in part, on the amount of actinic radiation that the photochromic material absorbs on a per molecule basis. That is, the more actinic radiation that the photochromic material absorbs on a per molecule basis, the more likely (i.e., the higher the probability) the photochromic material will transform from the closed-form to the open-form. Photochromic compositions and articles that are made using photochromic materials having a relatively high molar absorption coefficient (or "extinction coefficient for actinic radiation may generally be used in lower concentrations than photochromic materials having lower molar absorption coefficients, while still achieving the desired optical effect.

For some applications, the amount of photochromic material that can be incorporated into the article may be limited due to the physical dimensions of the article. Accordingly, the use of conventional photochromic materials that have a relatively low molar absorption coefficient in such articles may be impractical because the amount photochromic material needed to achieve the desired optical effects cannot be physically accommodated in the article. Further, in other applications, the size or solubility of the photochromic material itself may limit the amount of the photochromic material that can be incorporated into the article. Additionally, since photochromic materials may be expensive, in still other applications, the amount of photochromic material be used may be limited due to economic considerations.

Accordingly, for some applications, it may be advantageous to develop photochromic materials that can display hyperchromic absorption of actinic radiation, which may enable the use of lower concentrations of the photochromic material while still achieving the desired optical effects. As used herein, the term "hyperchromic absorption" refers to an increase in the absorption of electromagnetic radiation by a photochromic material having an extended pi-conjugated system on a per molecule basis as compared to a comparable photochromic material that does not have an extended pi-conjugated system.

Additionally, as mentioned above, typically the transformation between the closed-form and the open-form requires that the photochromic material be exposed to certain wavelengths of electromagnetic radiation. For many conventional photochromic materials, the wavelengths of electromagnetic radiation that may cause this transformation typically range from 320 nanometers ("nm") to 390 nm. Accordingly, conventional photochromic materials may not be optimal for use in applications that are shielded from a substantial amount of electromagnetic radiation in the range of 320 nm to 390 nm. For example, lenses for eyewear applications that are made using conventional photochromic materials may not reach their fully-colored state when used in an automobile. This is because a large portion of electromagnetic radiation in the range of 320 nm to 390 nm can be absorbed by the windshield of the automobile before it can be absorbed by the photochromic material(s) in the lenses. Therefore, for some applications, it may be advantageous to develop photochromic materials that can have a closed-form absorption spectrum for electromagnetic radiation that is shifted to longer wavelengths, that is "bathochromically shifted." As used herein the term "closed-form absorption spectrum" refers to the absorption spectrum of the photochromic material in the closed-form or unactivated state. For example, in applications involving behind the windshield use of photochromic materials, it may be advantageous if the closed-form absorption spectrum of the photochromic material were shifted such that the photochromic material may absorb sufficient electromagnetic radiation having a wavelength greater than 390 nm to permit the photochromic material to transform from the closed-form to an open-form.

BRIEF SUMMARY OF THE DISCLOSURE

Various non-limiting embodiments disclosed herein relate to photochromic materials comprising: (i) an indeno-fused naphthopyran; and (ii) a group that extends the pi-conjugated system of the indeno-fused naphthopyran bonded at the 11-position thereof, provided that if the group bonded at the 11-position of the indeno-fused naphthopyran and a group bonded at the 10-position or 12-position of the indeno-fused naphthopyran together form a fused group, said fused group is not a benzo-fused group; and wherein the 13-position of the indeno-fused naphthopyran is unsubstituted, mono-substituted or di-substituted, provided that if the 13-position of the indeno-fused naphthopyran is di-substituted, the substituents do not together form norbornyl.

Other non-limiting embodiments relate to photochromic materials comprising an indeno-fused naphthopyran, wherein the 13-position of the indeno-fused naphthopyran is unsubstituted, mono-substituted or di-substituted, provided that if the 13-position of the indeno-fused naphthopyran is di-substituted, the substituents do not together form norbornyl, and wherein the photochromic material has an integrated extinction coefficient greater than $1.0 \times 10^6$ nm x $mol^{-1}$ x $cm^{-1}$ as determined by integration of a plot of extinction coefficient of the photochromic material vs. wavelength over a range of wavelengths ranging from 320 nm to 420 nm, inclusive.

Still other non-limiting embodiments relate to photochromic materials comprising: an indeno-fused naphthopyran chosen from an indeno[2',3':3,4]naphtho[1,2-b]pyran, an indeno[1',2':4,3]naphtho[2,1-b]pyran, and mixtures thereof, wherein the 13-position of the indeno-fused naphthopyran is unsubstituted, mono-substituted or di-substituted, provided that if the 13-position of the indeno-fused naphthopyran is di-substituted, the substituent groups do not together form norbornyl; and a group that extends the pi-conjugated system of the indeno-fused naphthopyran bonded at the 11-position thereof, where said group is a substituted or unsubstituted aryl, a substituted or unsubstituted heteroaryl, or a group represented by -X=Y or -X'≡Y', wherein X, X', Y and Y' are as described herein below and as set forth in the claims; or the group that extends the pi-conjugated system of the indeno-fused naphthopyran bonded at the 11-position of the indeno-fused naphthopyran together with a group bonded at the 12-position of the indeno-fused naphthopyran or together with a group bonded at the 10-position of the indeno-fused naphthopyran form a fused group, said fused group being indeno, dihydronaphthalene, indole, benzofuran, benzopyran or thianaphthene.

Yet other non-limiting embodiments relate to photochromic materials represented by:

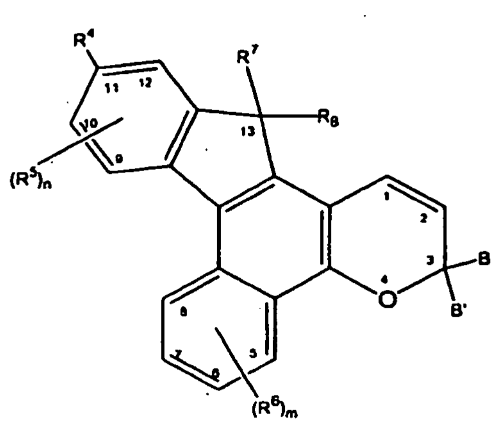

,

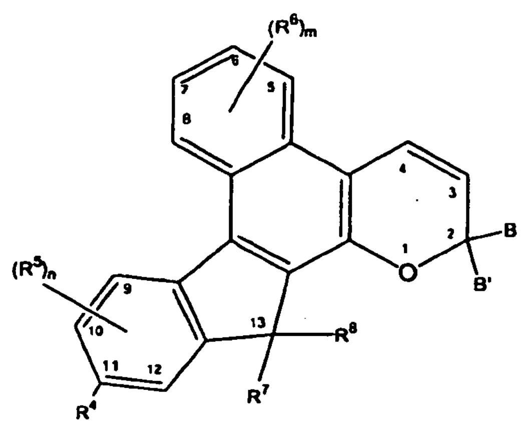

or a mixture thereof, wherein $R^4$, $R^5$, $R^6$, $R^7$, $R^8$, B and B' represent groups as described herein below and as set forth in the claims.

Still other non-limiting embodiments relate to photochromic compositions, photochromic articles, such as optical elements, and methods of making the same, wherein the photochromic compositions and photochromic articles comprise a photochromic material according to various non-limiting embodiments disclosed herein. For example, one specific non-limiting embodiment relates to an optical element adapted for use behind a substrate that blocks a substantial portion of electromagnetic radiation in the range of 320 nm to 390 nm, the optical element comprising a photochromic material comprising an indeno-fused naphthopyran and a group that extends the pi-conjugated system of the indeno-fused naphthopyran bonded at the 11-position thereof connected to at least a portion of the optical element, wherein the at least a portion of the optical element absorbs a sufficient amount of electromagnetic radiation having a wavelength greater than 390 nm passing through the substrate that blocks a substantial portion of electromagnetic radiation in the range of 320 nm to 390 nm such that the at least a portion of the optical element transforms from a first state to a second state.

BRIEF DESCRIPTION OF THE DRAWINGS

Various non-limiting embodiments disclosed herein may be better understood when read in conjunction with the drawings, in which:

Fig. 1 shows the absorption spectra obtained for a photochromic material according to one non-limiting embodiment disclosed herein at two different concentrations and the absorption spectra of a conventional photochromic material;
Figs. 2a, 2b, 3a and 3b are representations of photochromic materials according to various non-limiting embodiments disclosed herein;
Fig. 4 is a schematic diagram of a reaction scheme for making an intermediate material that may be used in forming photochromic materials according to various non-limiting embodiments disclosed herein; and
Figs. 5-8 are schematic diagrams of reaction schemes that may be used in making photochromic materials according to various non-limiting embodiments disclosed herein.

DETAILED DESCRIPTION

As used in this specification and the appended claims, the articles "a," "an," and "the" include plural referents unless expressly and unequivocally limited to one referent.

Additionally, for the purposes of this specification, unless otherwise indicated, all numbers expressing quantities of ingredients, reaction conditions, and other properties or parameters used in the specification are to be understood as being modified in all instances by the term "about." Accordingly, unless otherwise indicated, it should be understood that the numerical parameters set forth in the following specification and attached claims are approximations. At the very least, and not as an attempt to limit the application of the doctrine of equivalents to the scope of the claims, numerical parameters should be read in light of the number of reported significant digits and the application of ordinary rounding techniques.

Further, while the numerical ranges and parameters setting forth the broad scope of the invention are approximations as discussed above, the numerical values set forth in the Examples section are reported as precisely as possible. It should be understood, however, that such numerical values inherently contain certain errors resulting from the measurement equipment and/or measurement technique.

Photochromic materials according to various non-limiting embodiments of the invention will now be discussed. As used herein, the term "photochromic" means having an absorption spectrum for at least visible radiation that varies in response to absorption of at least actinic radiation. Further, as used herein the term "photochromic material" means any substance that is adapted to display photochromic properties, i.e. adapted to have an absorption spectrum for at least visible radiation that varies in response to absorption of at least actinic radiation. As previously discussed, as used herein the term "actinic radiation" refers to electromagnetic radiation that is capable of causing a photochromic material transform from one form or state to another.

Various non-limiting embodiments disclosed herein relate to photochromic materials comprising: (i) an indeno-fused naphthopyran; and (ii) a group that extends the pi-conjugated system of the indeno-fused naphthopyran bonded at the 11-position thereof, provided that if the group bonded at the 11-position of the indeno-fused naphthopyran and a group bonded at the 10-position or 12-position of the indeno-fused naphthopyran together form a fused group, said fused group is not a benzo-fused group; and wherein the 13-position of the indeno-fused naphthopyran is unsubstituted, mono-substituted or di-substituted, provided that if the 13-position of the indeno-fused naphthopyran is di-substituted, the substituent groups do not together form norbornyl (also known as bicyclo[2.2.1]heptyl or 8,9,10-trinorbornyl). As used herein, the term "fused" means covalently bonded in at least two positions.

As used herein, the terms "10-position," "11-position," "12-position," "13-position," etc. refer to the 10-, 11-, 12- and 13- position, etc. of the ring atoms of the indeno-fused naphthopyran, respectively. For example, according to one non-limiting embodiment wherein the indeno-fused naphthopyran is an indeno[2',3':3,4]naphtho[1,2-b]pyran, the ring atoms of the indeno-fused naphthopyran are numbered as shown below in (I). According to another non-limiting embodiment wherein the indeno-fused naphthopyran is an indeno[1',2':4,3]naphtho[2,1-b]pyran, the ring atoms of the indeno-fused naphthopyran are numbered shown below in (II).

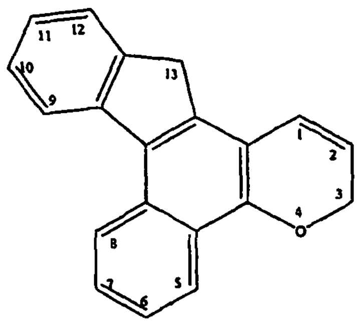

(I)

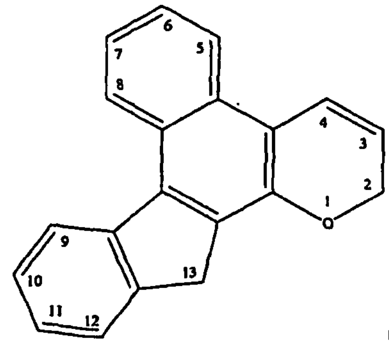

(II)

Further, according to various non-limiting embodiments disclosed herein, the indeno-fused naphthopyrans may have group(s) that can stabilize the open-form of the indeno-fused naphthopyran bonded to the pyran ring at an available position adjacent the oxygen atom (i.e., the 3-position in (I) above, or the 2-position in (II) above). For example, according to one non-limiting embodiment, the indeno-fused naphthopyrans may have a group that can extend the pi-conjugated system of the open-form of the indeno-fused naphthopyran bonded to the pyran ring adjacent the oxygen atom. Non-limiting examples of groups that may be bonded to the pyran ring as discussed above are described in more detail herein below with reference to B and B'.

Further, as discussed in more detail herein below, in addition to the group that extends the pi-conjugated system of the indeno-fused naphthopyran bonded at the 11-position of the indeno-fused naphthopyran, the photochromic materials according to various non-limiting embodiments disclosed may include additional groups bonded or fused at various positions on the indeno-fused naphthopyran other than the 11-position.

As used herein, the terms "group" or "groups" mean an arrangement of one or more atoms. As used herein, the phrase "group that extends the pi-conjugated system of the indeno-fused naphthopyran" means a group having at least one pi-bond ($\pi$-bond) in conjugation with the pi-conjugated system of the indeno-fused naphthopyran. It will be appreciated by those skilled in the art that in such system, the pi-electrons in the pi-conjugated system of the indeno-fused naphthopyran can be de-localized over the combined pi-system of the indeno-fused naphthopyran and the group having at least one pi-bond in conjugation with the pi-conjugated system of the indeno-fused naphthopyran. Conjugated bond systems may be represented by an arrangement of at least two double or triple bonds separated by one single bond, that is a system containing alternating double (or triple) bonds and single bonds, wherein the system contains at least two double (or triple) bonds. Non-limiting examples of groups that may extend the pi-conjugated system of the indeno-fused naphthopyran according to various non-limiting embodiments disclosed herein are set forth below in detail.

As previously discussed, the more actinic radiation that a photochromic material absorbs on a per molecule basis, the more likely the photochromic material will be to make the transformation from the closed-form to the open-form. Further, as previously discussed, photochromic materials that absorb more actinic radiation on a per molecule basis may generally be used in lower concentrations than those that absorb less actinic radiation on a per molecule basis, while still achieving the desired optical effects.

Although not meant to be limiting herein, it has been observed by the inventors that the indeno-fused naphthopyrans that comprise a group that extends the pi-conjugated system of the indeno-fused naphthopyran bonded at the 11-position thereof according to certain non-limiting embodiments disclosed herein may absorb more actinic radiation on a per molecule basis than a comparable indeno-fused naphthopyran without a group that extends the pi-conjugated system of the comparable indeno-fused naphthopyran bonded at the 11-position thereof. That is, the indeno-fused naphthopyrans according to certain non-limiting embodiments disclosed herein may display hyperchromic absorption of actinic radiation. As discussed above, as used herein the term "hyperchromic absorption" refers to an increase in the absorption of electromagnetic radiation by a photochromic material having an extended pi-conjugated system on a per molecule basis as compared to a comparable photochromic material that does not have an extended pi-conjugated system. Thus, while not meant to be limiting herein, it is contemplated that the indeno-fused naphthopyrans according to certain non-limiting embodiments disclosed herein may be advantageously employed in many applications, including applications wherein it may be necessary or desirable to limit the amount of the photochromic material employed.

The amount of radiation absorbed by a material (or the "absorbance" of the material) can be determined using a spectrophotometer by exposing the material to incident radiation having a particular wavelength and intensity and comparing the intensity of radiation transmitted by the material to that of the incident radiation. For each wavelength tested, the absorbance ("A") of the material is given by the following equation:

$$A = \log I_0/I$$

wherein "$I_0$" is the intensity of the incident radiation and "I" is the intensity of the transmitted radiation. An absorption spectrum for the material can be obtained by plotting the absorbance of a material vs. wavelength. By comparing the absorption spectrum of photochromic materials that were tested under the same conditions, that is using the same concentration and path length for electromagnetic radiation passing through the sample (e.g., the same cell length or sample thickness), an increase in the absorbance of one of the materials at a given wavelength can be seen as an increase in the intensity of the spectral peak for that material at that wavelength.

Referring now to Fig. 1, there are shown the absorption spectra for two different photochromic materials. Absorption spectra **1a** and **1b** were obtained from .22 cm x 15.24 cm x 15.24 cm acrylic chips that were made by adding 0.0015 molal (m) solutions of a photochromic material to be tested to a monomer blend, and subsequently casting the mixture to form the acrylic chips. Absorption spectrum **1c** was obtained from a 22 cm x 15.24 cm x 15.24 cm acrylic chip that was obtained by adding 0.00075 m solution of the same photochromic material used to obtain spectrum **1a** to the above-mentioned monomer blend and casting. The preparation of acrylic test chips is described in more detail in the Examples.

More particularly, absorption spectrum **1a** is the absorption spectrum at "full concentration" (i.e., 0.0015 m) for an indeno-fused naphthopyran according to one non-limiting embodiment disclosed herein comprising a group that extends the pi-conjugated system of the indeno-fused naphthopyran bonded at the 11-position thereof. Specifically, absorption spectrum **1a** is the absorption spectrum for a 3,3-di(4-methoxyphenyl)-6,7-dimethoxy-11-(4-(phenyl)phenyl)-13,13-dimethyl-3H,13H-indeno[2',3':3,4]naphtho[1,2-b]pyran. Since the absorbance of this photochromic material exceeded the maximum detection limit over the range of wavelengths tested, a plateau in absorbance is observed in absorption spectrum **1a.** Absorption spectrum **1b** is the absorption spectrum at "full concentration" (i.e., 0.0015 m) for a comparable indeno-fused naphthopyran without a group that extends the pi-conjugated system of the comparable indeno-fused naphthopyran bonded at the 11-position thereof. Specifically, absorption spectrum **1b** is the absorption spectrum for a 3,3-di(4-methoxyphenyl)-6,7-dimethoxy-13,13-dimethyl-3H,13H-indeno[2',3':3,4]naphtho[1,2-b]pyran.

As can be seen from absorption spectra **1a** and **1b** in Fig. 1, the indeno-fused naphthopyran comprising the group that extends the pi-conjugated system of the indeno-fused naphthopyran bonded at the 11-position thereof (spectrum **1a**) according to one non-limiting embodiment disclosed herein displays an increase in absorption of electromagnetic radiation having a wavelength ranging from 320 nm to 420 nm (i.e., displays hyperchromic absorption of electromagnetic radiation) as compared to a comparable indeno-fused naphthopyran without the group that extends the pi-conjugated system of the comparable indeno-fused naphthopyran bonded at the 11-position thereof (spectrum **1b**).

Referring again to Fig. 1, as previously discussed, absorption spectrum **1c** is the absorption spectrum for the same indeno-fused naphthopyran as spectrum **1a,** but was obtained from a sample having one-half of the full-concentration used to obtain absorption spectrum **1a.** As can be seen by comparing spectra **1c** and **1b** in Fig. 1, at one-half the concentration of the comparable photochromic material, the indeno-fused naphthopyran comprising the group that extends the pi-conjugated system of the indeno-fused naphthopyran bonded at the 11-position thereof according to one non-limiting embodiment disclosed herein displays hyperchromic absorption of electromagnetic radiation having a wavelength from 320 nm to 420 nm as compared to the comparable indeno-fused naphthopyran without the group that extends the pi-conjugated system of the comparable indeno-fused naphthopyran at the 11-position thereof at full concentration.

Another indication of the amount of radiation a material can absorb is the extinction coefficient of the material. The extinction coefficient ("ε") of a material is related to the absorbance of the material by the following equation:

$$\varepsilon = A / (c \times l)$$

wherein "A" is the absorbance of the material at a particular wavelength, "c" is the concentration of the material in moles per liter (mol/L) and "1" is the path length (or cell thickness) in centimeters. Further, by plotting the extinction coefficient vs. wavelength and integrating over a range of wavelengths (e.g., = $\int \varepsilon(\lambda)d\lambda$) it is possible to obtain an "integrated extinction coefficient" for the material. Generally speaking, the higher the integrated extinction coefficient of a material, the more radiation the material will absorb on a per molecule basis.

The photochromic materials according various non-limiting embodiments disclosed herein may have an integrated extinction coefficient greater than $1.0 \times 10^6$ nm/(mol x cm) or (nm x $mol^{-1}$ x $cm^{-1}$) as determined by integration of a plot of extinction coefficient of the photochromic material vs. wavelength over a range of wavelengths ranging from 320 nm to 420 nm, inclusive. Further, the photochromic materials according to various non-limiting embodiments disclosed herein may have an integrated extinction coefficient of at least $1.1 \times 10^6$nm x $mol^{-1}$ x $cm^{-1}$ or at least $1.3 \times 10^6$ nm x $mol^{-1}$ x $cm^{-1}$ as determined by integration of a plot of extinction coefficient of the photochromic material vs.

wavelength over a range of wavelengths ranging from 320 nm to 420 nm, inclusive. For example, according to various non-limiting embodiments, the photochromic material may have an integrated extinction coefficient ranging from 1.1 x $10^6$ to 4.0 x $10^6$ nm x $mol^{-1}$ x $cm^{-1}$ (or greater) as determined by integration of a plot of extinction coefficient of the photochromic material vs. wavelength over a range of wavelengths ranging from 320 nm to 420 nm, inclusive. However, as indicated above, generally speaking the higher the integrated extinction coefficient of a photochromic material, the more radiation the photochromic material will absorb on a per molecule basis. Accordingly, other non-limiting embodiments disclosed herein contemplate photochromic materials having an integrated extinction coefficient greater than 4.0 x $10^6$ nm x $mol^{-1}$ x $cm^{-1}$.

As previously discussed, for many conventional photochromic materials, the wavelengths of electromagnetic radiation required to cause the material to transformation from a closed-form (or unactivated state) to an open-form (or activated state) may range from 320 nm to 390 nm. Thus, conventional photochromic materials may not achieve their fully-colored state when used in applications that are shielded from a substantial amount of electromagnetic radiation in the range of 320 nm to 390 nm. Although not meant to be limiting herein, it has been observed by the inventors that indeno-fused naphthopyrans comprising a group that extends the pi-conjugated system of the indeno-fused naphthopyran at the 11-position thereof according to certain non-limiting embodiments disclosed herein may have a closed-form absorption spectrum for electromagnetic radiation that is bathochromically shifted as compared to a closed-form absorption spectrum for electromagnetic radiation of a comparable indeno-fused naphthopyran without the group that extends the pi-conjugated system of the comparable indeno-fused naphthopyran bonded at the 11-position thereof. As discussed above, as used herein the term "closed-form absorption spectrum" refers to the absorption spectrum of the photochromic material in the closed-form or unactivated state.

For example, referring again to Fig. 1, absorption spectrum **1a,** which is the absorption spectrum for an indeno-fused naphthopyran according to one non-limiting embodiment disclosed herein, is bathochromically shifted- that is, the absorption spectrum is displaced toward longer wavelengths- as compared to absorption spectrum **1b.** Since absorption spectrum **1a** has an increased absorption in the 390 nm to 420 nm range as compared to absorption spectrum **1b,** it is contemplated the photochromic material from which absorption spectrum **1a** was obtained may be advantageously employed in applications wherein a substantial amount of electromagnetic radiation in the range of 320 nm to 390 nm is shielded or blocked- for example, in applications involving use behind a windshield.

As discussed above, the photochromic materials according to various non-limiting embodiments disclosed herein comprise an indeno-fused naphthopyran and a group that extends the pi-conjugated system of the indeno-fused naphthopyran bonded at the 11-position thereof. Non-limiting examples of groups that may extend the pi-conjugated system of the indeno-fused naphthopyran according to various non-limiting embodiments disclosed herein, include a substituted or unsubstituted aryl group, such as, but not limited to, phenyl, naphthyl, fluorenyl, anthracenyl and phenanthracenyl; a substituted or unsubstituted heteroaryl group, such as, but not limited to, pyridyl, quinolinyl, isoquinolinyl, bipyridyl, pyridazinyl, cinnolinyl, phthalazinyl, pyrimidinyl, quinazolinyl, pyrazinyl, quinoxalinyl, phenanthrolinyl, triazinyl, pyrrolyl, indolyl, furfuryl, benzofurfuryl, thienyl, benzothienyl, pyrazolyl, indazolyl, imidazolyl, benzimidazolyl, triazolyl, benzotriazolyl, tetrazolyl, oxazolyl, benzoxazolyl, isoxazolyl, benzisoxazolyl, thiazolyl, benzothiazolyl, isothiazolyl, benzisothiazolyl, thiadiazolyl, benzothiadiazolyl, thiatriazolyl, purinyl, carbazolyl and azaindolyl; and a group represented by (III) or (IV) (below).

$$-X=Y \qquad (III)$$

$$-X'\equiv Y' \qquad (IV)$$

With reference to (III) above, non-limiting examples of groups that X may represent according to various non-limiting embodiments disclosed herein include $-CR^1$, -N, -NO, $-SR^1$, $-S(=O)R^1$ and $-P(=O)R^1$. Further, according to various non-limiting embodiments disclosed herein, if X represents -CR' or -N, Y may represent a group such as, but not limited to, $C(R^2)_2$, $NR^2$, O and S. Still further, according to various non-limiting embodiments disclosed herein, if X represents -NO, $-SR^1$, $-S(=O)R^1$ or $-P(=O)R^1$, Y may represent a group such as, but not limited to, O. Non-limiting examples of groups that $R^1$ may represent include amino, dialkyl amino, diaryl amino, acyloxy, acylamino, a substituted or unsubstituted $C_1$-$C_{20}$ alkyl, a substituted or unsubstituted $C_2$-$C_{20}$ alkenyl, a substituted or unsubstituted $C_2$-$C_{20}$ alkynyl, halogen, hydrogen, hydroxy, oxygen, a polyol residue (such as, but not limited to, those discussed herein below with respect to -G-), a substituted or unsubstituted phenoxy, a substituted or unsubstituted benzyloxy, a substituted or unsubstituted alkoxy, a substituted or unsubstituted oxyalkoxy, alkylamino, mercapto, alkylthio, a substituted or unsubstituted aryl, a substituted or unsubstituted heteroaryl, a substituted or unsubstituted heterocyclic group (e.g., piperazino, piperidino, morpholino, pyrrolidino, etc.), a reactive substituent, a compatibilizing substituent, and a photochromic material. Non-limiting examples of groups from which each $R^2$ group discussed above may be independently chosen include those groups discussed above with respect to $R^1$.

With reference to (IV) above, according to various non-limiting embodiments disclosed herein, X' may represent

a group including, but not limited to, -C or $-N^+$, and Y' may represent a group including, but not limited to, $CR^3$ or N. Non-limiting examples of groups that $R^3$ may represent include those groups discussed above with respect to $R^1$.

Alternatively, as discussed above, according to various non-limiting embodiments disclosed herein, the group that extends the pi-conjugated system of the indeno-fused naphthopyran bonded at the 11-position of the indeno-fused naphthopyran together with a group bonded at the 12-position of the indeno-fused naphthopyran or together with a group bonded at the 10-position of the indeno-fused naphthopyran may form a fused group, provided that the fused group is not a benzo-fused group. According to other non-limiting embodiments, the group bonded at the 11-position together with a group bonded at the 12-position or the 10-position may form a fused group, provided that the fused group extends the pi-conjugated system of the indeno-fused naphthopyran at the 11-position, but does not extend the pi-conjugated system of the indeno-fused naphthopyran at the 10-position or the 12-position. For example, according to various non-limiting embodiments disclosed herein, if the group bonded at the 11-position of the indeno-fused naphthopyran together with a group bonded at the 10-position or 12-position of the indeno-fused naphthopyran forms a fused group, the fused group may be indeno, dihydronaphthalene, indole, benzofuran, benzopyran or thianaphthene.

According to various non-limiting embodiments disclosed herein, the group that extends the pi-conjugated system of the indeno-fused naphthopyran bonded at the 11-position thereof may be a substituted or unsubstituted $C_2$-$C_{20}$ alkenyl; a substituted or unsubstituted $C_2$-$C_{20}$ alkynyl; a substituted or unsubstituted aryl; a substituted or unsubstituted heteroaryl; $-C(=O)R^1$, wherein $R^1$ may represent a group as set forth above; or -N(=Y) or $-N^+(\equiv Y')$, wherein Y may represent a group such as, but not limited to, $C(R^2)_2$, $NR^2$, O and S, and Y' may represent a group such as, but not limited to, $CR^3$ and N, wherein $R^2$ and $R^3$ may represent groups such as those discussed above. Substituents that may be bonded to the substituted $C_2$-$C_{20}$ alkenyl, substituted $C_2$-$C_{20}$ alkynyl, substituted aryl, and substituted heteroaryl groups according to these and other non-limiting embodiments disclosed herein include groups, which may be substituted or unsubstituted, such as, but not limited to, alkyl, alkoxy, oxyalkoxy, amide, amino, aryl, heteroaryl, azide, carbonyl, carboxy, ester, ether, halogen, hydroxy, oxygen, a polyol residue, phenoxy, benzyloxy, cyano, nitro, sulfonyl, thiol, a heterocyclic group, a reactive substituent, a compatibilizing substituent, and a photochromic material. Further, according to various non-limiting embodiments disclosed herein wherein the group that extends the pi-conjugated system of the indeno-fused naphthopyran comprises more than one substituent, each substituent may be independently chosen.

For example, according to one non-limiting embodiment, the group that extends the pi-conjugated system of the indeno-fused naphthopyran bonded at the 11-position thereof may be an aryl group or a heteroaryl group that is unsubstituted or substituted with at least one of a substituted or unsubstituted alkyl, a substituted or unsubstituted alkoxy, a substituted or unsubstituted oxyalkoxy, amide, a substituted or unsubstituted amino, a substituted or unsubstituted aryl, a substituted or unsubstituted heteroaryl, azide, carbonyl, carboxy, ester, ether, halogen, hydroxy, a polyol residue, a substituted or unsubstituted phenoxy, a substituted or unsubstituted benzyloxy, cyano, nitro, sulfonyl, thiol, a substituted or unsubstituted heterocyclic group, a reactive substituent, a compatiblizing substituent or a photochromic material. Further, if the aryl group or the heteroaryl group comprises more than one substituent, each substituent may be the same as or different from one or more of the remaining substituents.

According to another non-limiting embodiment, the group that extends the pi-conjugated system of the indeno-fused naphthopyran bonded at the 11-position thereof may be $-C(=O)R^1$, and $R^1$ may represent acylamino, acyloxy, a substituted or unsubstituted $C_1$-$C_{20}$ alkyl, a substituted or unsubstituted alkoxy, a substituted or unsubstituted oxyalkoxy, amino, dialkyl amino, diaryl amino, a substituted or unsubstituted aryl, a substituted or unsubstituted heteroaryl, a substituted or unsubstituted heterocyclic group, halogen, hydrogen, hydroxy, oxygen, a polyol residue, a substituted or unsubstituted phenoxy, a substituted or unsubstituted benzyloxy, a reactive substituent or a photochromic material.

Further, the photochromic materials comprising a group that extends the pi-conjugated system of the indeno-fused naphthopyran bonded at the 11-position according to various non-limiting embodiments disclosed herein may further comprise another photochromic material that is linked, directly or indirectly, to the group that extends the pi-conjugated system or another position on the photochromic material. For example, although not limiting herein, as shown in Fig. 2a, the group that extends the pi-conjugated system of the indeno-fused naphthopyran bonded at the 11-position thereof may be represented by-X=Y, wherein X represents $-CR^1$ and Y represents O (i.e., $-C(=O)R^1$), wherein $R^1$ represents a heterocyclic group (e.g., a piperazino group as shown in Fig. 2a) that is substituted with a photochromic material (e.g., a 3,3-diphenyl-6,11-dimethoxy-13,13-dimethyl-3H,13H-indeno[2',3':3,4]naphtho[1,2-b]pyran as shown in Fig. 2a). According to another non-limiting embodiment shown in Fig. 2b, the group that extends the pi-conjugated system of the indeno-fused naphthopyran bonded at the 11-position thereof may be represented by -X=Y, wherein X represents $-CR^1$ and Y represents O (i.e., $-C(=O)R^1$), wherein $R^1$ represents an oxyalkoxy (e.g., an oxyethoxy as shown in Fig. 2b) that is substituted with a photochromic material (e.g., a 3,3-diphenyl-6,11-dimethoxy-13,13-dimethyl-3H,13H-indeno[2',3':3,4]naphtho[1,2-b]pyran as shown in Fig. 2b).

Although not limiting herein, according to various non-limiting embodiments wherein the photochromic material comprising the group that extends the pi-conjugated system bonded at the 11-position thereof comprises an additional photochromic material that is linked thereto, the additional photochromic material may be linked to the photochromic material comprising the group that extends the pi-conjugated system bonded at the 11-position thereof by an insulating

group. As used herein, the term "insulating group" means a group having at least two consecutive sigma (σ) bonds that separate the pi-conjugated systems of the photochromic materials. For example, and without limitation herein, as shown in Figs. 2a and 2b, the additional photochromic material may be linked to the photochromic material comprising the group that extends the pi-conjugated system bonded at the 11-position thereof by one or more insulating group(s). Specifically, although not limiting herein, as shown in Fig. 2a, the insulating group may be the alkyl portion of a piperazino group, and, as shown in Fig. 2b, the insulating group may be the alkyl portion of an oxyalkoxy group.

Still further, and as discussed in more detail below, according to various non-limiting embodiments, the group that extends the pi-conjugated system of the indeno-fused naphthopyran bonded at the 11-position may comprise a reactive substituent or a compatibilizing substituent. As used herein the term "reactive substituent" means an arrangement of atoms, wherein a portion of the arrangement comprises a reactive moiety or a residue thereof. As used herein, the term "moiety" means a part or portion of an organic molecule that has a characteristic chemical property. As used herein, the term "reactive moiety" means a part or portion of an organic molecule that may react to form one or more bond(s) with an intermediate in a polymerization reaction, or with a polymer into which it has been incorporated. As used herein the term "intermediate in a polymerization reaction" means any combination of two or more monomer units that are capable of reacting to form one or more bond(s) to additional monomer unit(s) to continue a polymerization reaction or, alternatively, reacting with a reactive moiety of the reactive substituent on the photochromic material. For example, although not limiting herein, the reactive moiety may react with an intermediate in a polymerization reaction of a monomer or oligomer as a co-monomer in the polymerization reaction or may react as, for example and without limitation, a nucleophile or electrophile, that adds into the intermediate. Alternatively, the reactive moiety may react with a group (such as, but not limited to a hydroxyl group) on a polymer.

As used herein the term "residue of a reactive moiety" means that which remains after a reactive moiety has been reacted with a protecting group or an intermediate in a polymerization reaction. As used herein the term "protecting group" means a group that is removably bonded to a reactive moiety that prevents the reactive moiety from participating in a reaction until the group is removed. Optionally, the reactive substituents according to various non-limiting embodiments disclosed herein may further comprise a linking group. As used herein the term "linking group" means one or more group(s) or chain(s) of atoms that connect the reactive moiety to the photochromic material.

As used herein the term "compatibilizing substituent" means an arrangement of atoms that can facilitate integration of the photochromic material into another material or solvent. For example, according to various non-limiting embodiments disclosed herein, the compatibilizing substituent may facilitate integration of the photochromic material into a hydrophilic material by increasing the miscibility of the photochromic material in water or a hydrophilic polymeric, oligomeric, or monomeric material. According to other non-limiting embodiments, the compatibilizing substituent may facilitate integration of the photochromic material into a lipophilic material. Although not limiting herein, photochromic materials according to various non-limiting embodiments disclosed herein that comprise a compatibilizing substituent that facilitates integration into a hydrophilic material may be miscible in hydrophilic material at least to the extent of one gram per liter. Non-limiting examples of compatibilizing substitutents include those substitutents comprising the group -J, where -J represents the group -K or hydrogen, which are discussed herein below.

Further, it should be appreciated that some substituents may be both compatibilizing and reactive. For example, a substituent that comprises hydrophilic linking group(s) that connects a reactive moiety to the photochromic material may be both a reactive substituent and a compatibilizing substituent. As used herein, such substituents may be termed as either a reactive substituent or a compatibilizing substituent.

As discussed above, various non-limiting embodiments disclosed herein relate to photochromic materials comprising an indeno-fused naphthopyran and a group that extends the pi-conjugated system of the indeno-fused naphthopyran bonded at the 11-position thereof, provided that if the group bonded at the 11-position of the indeno-fused naphthopyran together with a group bonded at the 10-position or 12-position of the indeno-fused naphthopyran forms a fused group, said fused group is not a benzo-fused group; and wherein the 13-position of the indeno-fused naphthopyran is unsubstituted, mono-substituted or di-substituted, provided that if the 13-position of the indeno-fused naphthopyran is disubstituted, the substituent groups do not together form norbornyl. Further, according to other non-limiting embodiments, the indeno-fused naphthopyran may be free of spiro-cyclic groups at the 13-position of the indeno-fused naphthopyran. As used herein the phrase "free of spiro-cyclic groups at the 13-position" means that if the 13-position of the indeno-fused naphthopyran is di-substituted, the substituent groups do not together form a spiro-cyclic group. Non-limiting examples of suitable groups that may be bonded at the 13-position are set forth with respect to $R^7$ and $R^8$ in (XIV) and (XV) herein below.

Further, various non-limiting embodiments disclosed herein relate to photochromic materials comprising an indeno-fused naphthopyran and a group that extends the pi-conjugated system of the indeno-fused naphthopyran bonded at the 11-position thereof (as discussed above), wherein the indeno-fused naphthopyran is an indeno[2',3':3,4]naphtho [1,2-b]pyran, and wherein the 6-position and/or the 7-position of the indeno-fused naphthopyran may each independently be substituted with a nitrogen containing group or an oxygen containing group; and the 13-position of the indeno-fused naphthopyran may be di-substituted. Non-limiting examples of substituents that may be bonded at the 13-position

according to this non-limiting embodiment include hydrogen, $C_1$-$C_6$ alkyl, $C_3$-$C_7$ cycloalkyl, allyl, a substituted or unsubstituted phenyl, a substituted or unsubstituted benzyl, a substituted or unsubstituted amino and -C(O)$R^{30}$. Non-limiting examples of groups that $R^{30}$ may represent include hydrogen, hydroxy, $C_1$-$C_6$ alkyl, $C_1$-$C_6$ alkoxy, the unsubstituted, mono-or di-substituted aryl groups phenyl or naphthyl, phenoxy, mono- or di-($C_1$-$C_6$)alkyl substituted phenoxy or mono- and di-($C_1$-$C_6$)alkoxy substituted phenoxy. Suitable non-limiting examples of nitrogen containing groups and oxygen containing groups that may be present at the 6-position and/or the 7-position of the indeno-fused naphthopyran according to these and other non-limiting embodiments disclosed herein include those that are set forth with respect to $R^6$ in (XIV) and (XV) herein below.

Other non-limiting embodiments disclosed herein relate to photochromic materials comprising an indeno-fused naphthopyran, wherein the 13-position of the indeno-fused naphthopyran is unsubstituted, mono-substituted or di-substituted, provided that if the 13-position of the indeno-fused naphthopyran is di-substituted, the substituent groups do not together form norbornyl, and wherein the photochromic material has an integrated extinction coefficient greater than 1.0 x $10^6$ nm x $mol^{-1}$ x $cm^{-1}$ as determined by integration of a plot of extinction coefficient of the photochromic material vs. wavelength over a range of wavelengths ranging from 320 nm to 420 nm, inclusive. Further, according to these non-limiting embodiments the integrated extinction coefficient may range from 1.1 x $10^6$ to 4.0 x $10^6$ nm x $mol^{-1}$ x $cm^{-1}$ as determined by integration of a plot of extinction coefficient of the photochromic material vs. wavelength over a range of wavelengths ranging from 320 nm to 420 nm, inclusive. Still further, the photochromic materials according these non-limiting embodiments may comprise a group that extends the pi-conjugated system of the indeno-fused naphthopyran bonded at the 11-position thereof. Non-limiting examples of groups bonded at the 11-position of the indeno-fused naphthopyran that extend the pi-conjugated system of the indeno-fused naphthopyran include those discussed above.

One specific non-limiting embodiment disclosed herein provides a photochromic material comprising: (i) an indeno-fused naphthopyran chosen from an indeno[2',3':3,4]naphtho[1,2-b]pyran, an indeno[1',2':4,3]naphtho[2,1-b]pyran, and mixtures thereof, wherein the 13-position of the indeno-fused naphthopyran is unsubstituted, mono-substituted or di-substituted, provided that if the 13-position of the indeno-fused naphthopyran is di-substituted, the substituent groups do not together form norbornyl; and (ii) a group that extends the pi-conjugated system of the indeno-fused naphthopyran bonded at the 11-position thereof, wherein said group may be a substituted or unsubstituted aryl, a substituted or unsubstituted heteroaryl, or a group represented by -X=Y or -X'≡Y'. Non-limiting examples of groups that X, X', Y and Y' may represent are set forth above.

Alternatively, the group that extends the pi-conjugated system of the indeno-fused naphthopyran bonded at the 11-position of the indeno-fused naphthopyran together with a group bonded at the 12-position of the indeno-fused naphthopyran or together with a group bonded at the 10-position of the indeno-fused naphthopyran form a fused group, said fused group being indeno, dihydronaphthalene, indole, benzofuran, benzopyran or thianaphthene. Further, according to this non-limiting embodiment, the indeno-fused naphthopyran may be free of spiro-cyclic groups at the 13-position thereof.

As previously discussed, the photochromic materials according to various non-limiting embodiments disclosed herein may comprise at least one of a reactive substituent and/or a compatibilizing substituent. Further, according to various non-limiting embodiments disclosed herein wherein the photochromic material comprises multiple reactive substituents and/or multiple compatibilizing substituents, each reactive substituent and each compatibilizing substituent may be independently chosen. Non-limiting examples of reactive and/or compatibilizing substituents that may be used in conjunction with the various non-limiting embodiments disclosed herein may be represented by one of:

-A'-D-E-G-J (V);

-G-E-G-J (VI);

-D-E-G-J (VII);

-A'-D-J (VIII);

-D-G-J (IX);

-D-J (X);

-A'-G-J (XI);

-G-J (XII);

and

-A'-J (XIII).

With reference to (V)-(XIII) above, non-limiting examples of groups that -A'- may represent according to various non-limiting embodiments disclosed herein include -O-, -C(=O)-, $-CH_2-$, -OC(=O)- and -NHC(=O)-, provided that if -A'- represents -O-, -A'- forms at least one bond with -J.

Non-limiting examples of groups that -D- may represent according to various non-limiting embodiments include a diamine residue or a derivative thereof, wherein a first amino nitrogen of said diamine residue may form a bond with -A'-, the group that extends the pi-conjugated system of the indeno-fused naphthopyran bonded at the 11-position thereof, or a substituent or an available position on the indeno-fused naphthopyran, and a second amino nitrogen of said diamine residue may form a bond with -E-, -G- or -J; and an amino alcohol residue or a derivative thereof, wherein an amino nitrogen of said amino alcohol residue may form a bond with -A'-, the group that extends the pi-conjugated system of the indeno-fused naphthopyran bonded at the 11-position thereof, or a substituent or an available position on the indeno-fused naphthopyran, and an alcohol oxygen of said amino alcohol residue may form a bond with -E-, -G- or -J. Alternatively, according to various non-limiting embodiments disclosed herein the amino nitrogen of said amino alcohol residue may form a bond with -E-, -G- or -J, and said alcohol oxygen of said amino alcohol residue may form a bond with -A'-, the group that extends the pi-conjugated system of the indeno-fused naphthopyran bonded at the 11-position thereof, or a substituent or an available position on the indeno-fused naphthopyran.

Non-limiting examples of suitable diamine residues that -D- may represent include an aliphatic diamine residue, a cyclo aliphatic diamine residue, a diazacycloalkane residue, an azacyclo aliphatic amine residue, a diazacrown ether residue, and an aromatic diamine residue. Specific non-limiting examples diamine residues that may be used in conjunction with various non-limiting embodiments disclosed herein include the following:

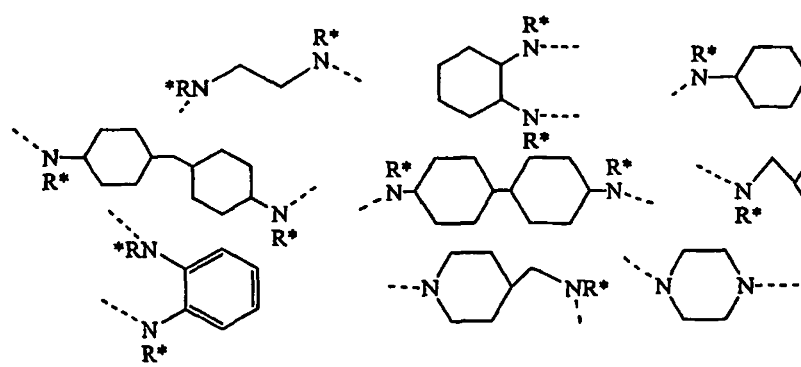

R*= H or alkyl

Non-limiting examples of suitable amino alcohol residues that -D- may represent include an aliphatic amino alcohol residue, a cyclo aliphatic amino alcohol residue, an azacyclo aliphatic alcohol residue, a diazacyclo aliphatic alcohol residue and an aromatic amino alcohol residue. Specific non-limiting examples amino alcohol residues that may be used in conjunction with various non-limiting embodiments disclosed herein include the following:

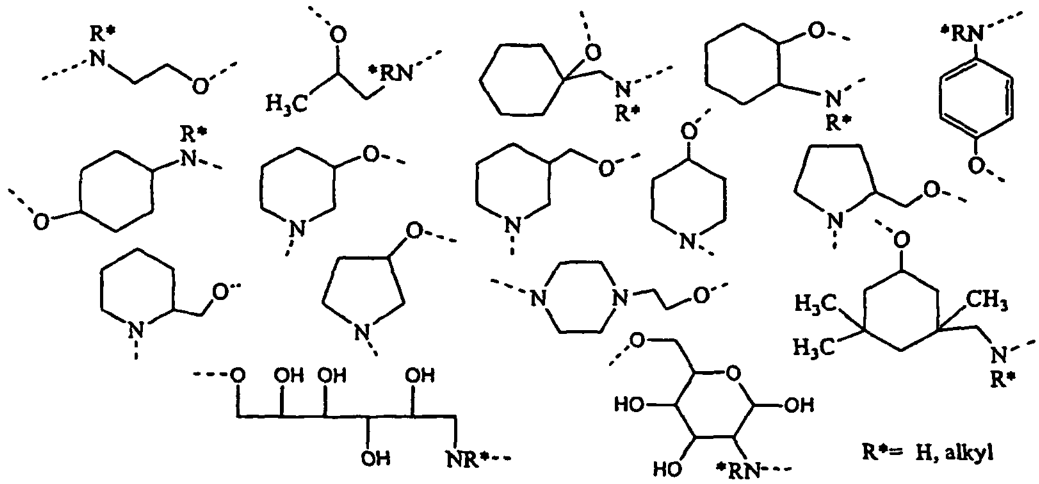

R*= H, alkyl

With continued reference to (V)-(XIII) above, according to various non-limiting embodiments disclosed herein, -E- may represent a dicarboxylic acid residue or a derivative thereof, wherein a first carbonyl group of said dicarboxylic acid residue may form a bond with -G- or -D-, and a second carbonyl group of said dicarboxylic acid residue may form a bond with -G-. Non-limiting examples of suitable dicarboxylic acid residues that -E- may represent include an aliphatic dicarboxylic acid residue, a cycloaliphatic dicarboxylic acid residue and an aromatic dicarboxylic acid residue. Specific non-limiting examples of dicarboxylic acid residues that may be used in conjunction with various non-limiting embodiments disclosed herein include the following:

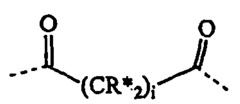

i=1 to 4
R* = H or alkyl

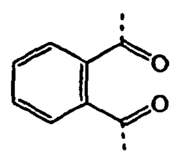 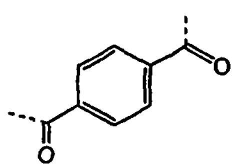 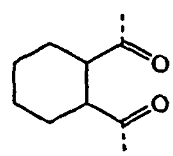 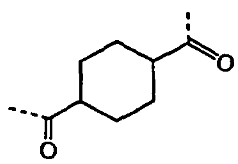

According to various non-limiting embodiments disclosed herein, -G- may represent a group $-[(OC_2H_4)_x(OC_3H_6)_y(OC_4H_8)_z]-O-$, wherein x, y and z are each independently chosen and range from 0 to 50, and a sum of x, y, and z ranges from 1 to 50; a polyol residue or a derivative thereof, wherein a first polyol oxygen of said polyol residue may form a bond with -A'-, -D-, -E-, the group that extends the pi-conjugated system of the indeno-fused naphthopyran bonded at the 11-position thereof, or a substituent or an available position on the indeno-fused naphthopyran, and a second polyol oxygen of said polyol residue may form a bond with -E- or -J; or a combination thereof, wherein the first polyol oxygen of the polyol residue forms a bond with a group $-[(OC_2H_4)_x(OC_3H_6)_y(OC_4H_8)_z]-$ (i.e., to form the group $-[(OC_2H_4)_x(OC_3H_6)_y(OC_4H_8)_z]-O-$), and the second polyol oxygen forms a bond with -E- or -J. Non-limiting examples of suitable polyol residues that -G- may represent include an aliphatic polyol residue, a cyclo aliphatic polyol residue and an aromatic polyol residue.

Specific non-limiting examples of polyols from which the polyol residues that -G-may represent may be formed according to various non-limiting embodiments disclosed herein include (a) low molecular weight polyols having an average molecular weight less than 500, such as, but not limited to, those set forth in U.S. Patent No. 6,555,028 at col. 4, lines 48-50, and col. 4, line 55 to col. 6, line 5; (b) polyester polyols, such as, but not limited to, those set forth in U.S. Patent No. 6,555,028 at col. 5, lines 7-33; (c) polyether polyols, such as but not limited to those set forth in U.S. Patent No. 6,555,028 at col. 5, lines 34-50; (d) amide-containing polyols, such as, but not limited to, those set forth in U.S. Patent No. 6,555,028 at col. 5, lines 51-62; (e) epoxy polyols, such as, but not limited to, those set forth in U.S. Patent No. 6,555,028 at col. 5 line 63 to col. 6, line 3; (f) polyhydric polyvinyl alcohols, such as, but not limited to, those set forth in U.S. Patent No. 6,555,028 at col. 6, lines 4-12 ; (g) urethane polyols, such as, but not limited to those set forth in U.S. Patent No. 6,555,028 at col. 6, lines 13-43 ; (h) polyacrylic polyols, such as, but not limited to those set forth in U.S. Patent No. 6,555,028 at col. 6, lines 43 to col. 7, line 40; (i) polycarbonate polyols, such as, but not limited to, those set forth in U.S. Patent No. 6,555,028 at col. 7; lines 41-55; and (j) mixtures of such polyols.

Referring again to (V)-(XIII) above, according to various non-limiting embodiments disclosed herein, -J may represent a group -K, wherein -K represents a group such as, but not limited to, $-CH_2COOH$, $-CH(CH_3)COOH$, -C(O)

$(CH_2)_wCOOH$, $-C_6H_4SO_3H$, $-C_5H_{10}SO_3H$, $-C_4H_8SO_3H$, $-C_3H_6SO_3H$, $-C_2H_4SO_3H$ and $-SO_3H$, wherein "w" ranges from I to 18. According to other non-limiting embodiments -J may represent hydrogen that forms a bond with an oxygen or a nitrogen of linking group to form a reactive moiety such as -OH or -NH. For example, according to various non-limiting embodiments disclosed herein, -J may represent hydrogen, provided that if -J represents hydrogen, -J is bonded to an oxygen of -D- or -G-, or a nitrogen of -D-.

According to still other non-limiting embodiments, -J may represent a group -L or residue thereof, wherein -L may represent a reactive moiety. For example, according to various non-limiting embodiments disclosed herein -L may represent a group such as, but not limited to, acryl, methacryl, crotyl, 2-(methacryloxy)ethylcarbamyl, 2-(methacryloxy) ethoxycarbonyl, 4-vinylphenyl, vinyl, 1-chlorovinyl or epoxy. As used herein, the terms acryl, methacryl, crotyl, 2-(methacryloxy)ethylcarbamyl, 2-(methacryloxy)ethoxycarbonyl, 4-vinylphenyl, vinyl, 1-chlorovinyl, and epoxy refer to the following structures:

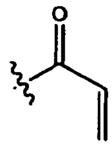
acryl

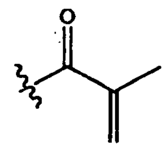
methacryl

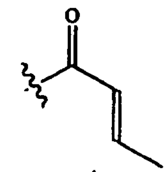
crotyl

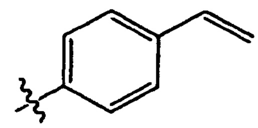
4-vinylphenyl

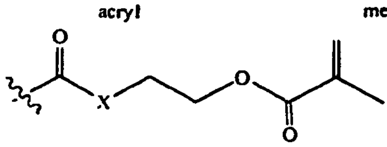
X = NH: 2-(methacryloxy)ethylcarbamyl
X = O: 2-(methacryloxy)ethoxycarbonyl

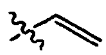
vinyl

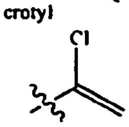
1-chlorovinyl

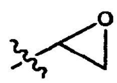
epoxy

As previously discussed, -G- may represent a residue of a polyol, which is defined herein to include hydroxy-containing carbohydrates, such as those set forth in U.S. Patent No. 6,555,028 at col. 7, line 56 to col. 8, line 17. The polyol residue may be formed, for example and without limitation herein, by the reaction of one or more of the polyol hydroxyl groups with a precursor of -A'-, such as a carboxylic acid or a methylene halide, a precursor of polyalkoxylated group, such as polyalkylene glycol, or a hydroxyl substituent of the indeno-fused naphthopyran. The polyol may be represented by $q\text{-}(OH)_a$ and the residue of the polyol may be represented by the formula $-O\text{-}q\text{-}(OH)_{a-1}$, wherein $q$ is the backbone or main chain of the polyhydroxy compound and "a" is at least 2.

Further, as discussed above, one or more of the polyol oxygens of -G- may form a bond with -J (i.e., forming the group -G-J). For example, although not limiting herein, wherein the reactive and/or compatiblizing substituent comprises the group -G-J, if -G-represents a polyol residue and -J represents a group -K that contains a carboxyl terminating group, -G-J may be produced by reacting one or more polyol hydroxyl groups to form the group -K (for example as discussed with respect to Reactions B and C at col. 13, line 22 to col. 16, line 15 of U.S. Patent No. 6,555,028 ) to produce a carboxylated polyol residue. Alternatively, if -J represents a group -K that contains a sulfo or sulfono terminating group, although not limiting herein, -G-J may be produced by acidic condensation of one or more of the polyol hydroxyl groups with $HOC_6H_4SO_3H$; $HOC_5H_{10}SO_3H$; $HOC_4H_8SO_3$ H; $HOC_3H_6SO_3H$; $HOC_2H_4SO_3H$; or $H_2SO_4$, respectively. Further, although not limiting herein, if-G-represents a polyol residue and -J represents a group -L chosen from acryl, methacryl, 2-(methacryloxy)ethylcarbamyl and epoxy, -L may be added by condensation of the polyol residue with acryloyl chloride, methacryloyl chloride, 2-isocyanatoethyl methacrylate or epichlorohydrin, respectively.

As discussed above, according to various non-limiting embodiments disclosed herein, a reactive substituent and/or a compatibilizing substituent may be bonded to group that extends the pi-conjugated system of the indeno-fused naphthopyran bonded at the 11-position of the indeno-fused naphthopyran. For example, as discussed above, the group that extends the pi-conjugated system of the indeno-fused naphthopyran bonded at the 11-position thereof may be an aryl or heteroaryl that is substituted with the reactive and/or compatibilizing substituent, or may be a group represented by -X=Y or -X'≡Y', wherein the groups X, X', Y and Y' may comprise the reactive and/or compatibilizing substituent as discussed above. For example, according to one non-limiting embodiment as shown in Fig. 3a, the group that extends the pi-conjugated system may be an aryl group (e.g., a phenyl group as shown in Fig. 3a) that is substituted with a reactive substituent (e.g., a (2-methacryloxyethoxy)carbonyl as shown in Fig. 3a), which may be represented by -A'-G-J (as discussed above), wherein -A'-represents -C(=O)-, -G- represents $-[OC_2H_4]O-$, and -J represents methacryl.

Additionally or alternatively, a reactive and/or compatibilizing substituent may be bonded at a substituent or an available position on the indeno-fused naphthopyran ring other than at the 11-position. For example, although not limiting herein, in addition to or instead of having a reactive and/or compatibilizing substituent bonded to the group that extends the pi-conjugated system of the indeno-fused naphthopyran bonded at the 11-position of the indeno-fused

naphthopyran, the 13-position of the indeno-fused naphthopyran may be mono- or di-substituted with a reactive and/or compatibilizing substituent. Further, if the 13-position is di-substituted, each substituent may be the same or different. In another non-limiting example, in addition to or instead of having a reactive and/or compatibilizing substituent bonded to the group that extends the pi-conjugated system of the indeno-fused naphthopyran bonded at the 11-position of the indeno-fused naphthopyran, a reactive and/or compatibilizing substituent may be substituted at the 3-position of an indeno[2',3':3,4]naphtho[1,2-b]pyran, the 2-position of an indeno[1',2':4,3]naphtho[2,1-b]pyran, and/or the 6- or 7-positions of these indeno-fused naphthopyrans. Further, if the photochromic material comprises more than one reactive and/or compatibilizing substituent, each reactive and/or compatiblizing substituent may be the same as or different from one or more of the remaining reactive and/or compatibilizing substituents.

For example, referring now to Fig. 3b, according to one non-limiting embodiment, the group that extends the pi-conjugated system of the indeno-fused naphthopyran bonded at the 11-position thereof is a substituted aryl group (e.g., a 4-(phenyl)phenyl group as shown in Fig. 3b), and the photochromic material further comprises a reactive substituent (e.g., a 3-(2-methacryloxyethyl)carbamyloxymethylenepiperidino-1-yl) group as shown in Fig. 3b), which may be represented by -D-J (as discussed above), wherein -D- represents an azacyclo aliphatic alcohol residue, wherein the nitrogen of the azacyclo aliphatic alcohol residue forms a bond with the indeno-fused naphthopyran at the 7-position, and the alcohol oxygen of the azacyclo aliphatic alcohol residue forms a bond with -J, wherein -J represents 2-(methacryloxy)ethylcarbamyl. Another non-limiting example of a photochromic material according to various non-limiting embodiments disclosed herein that has a reactive substituent at the 7-position thereof is a 3-(4-morpholinophenyl)-3-phenyl-6-methoxy-7-(3-(2-methacryloxyethyl)carbamyloxymethylenepiperidino-1-yl)-11-phenyl-13,13-dimethyl-3H, 13H-indeno[2',3':3,4]naphtho[1,2-b]pyran.

One non-limiting example of a photochromic material according to various non-limiting embodiments disclosed herein that has a reactive substituent at the 3-position thereof is a 3-(4-(2-(2-methacryloxyethyl)carbamylethoxy)phenyl)-3-phenyl-6,7-dimethoxy-11-phenyl-13,13-dimethyl-3H,13H-indeno[2',3':3,4]naphtho[1,2-b]pyran.

Still other non-limiting examples of reactive and/or compatibilizing substituents are set forth in U.S. Patent No. 6,555,028, at col. 3, line 45 to col. 4, line 26, and U.S. Patent No. 6,113,814 at col. 3, lines 30-64.

Other non-limiting embodiments disclosed herein provide a photochromic material represented by (XIV), (XV) (shown below) or a mixture thereof.

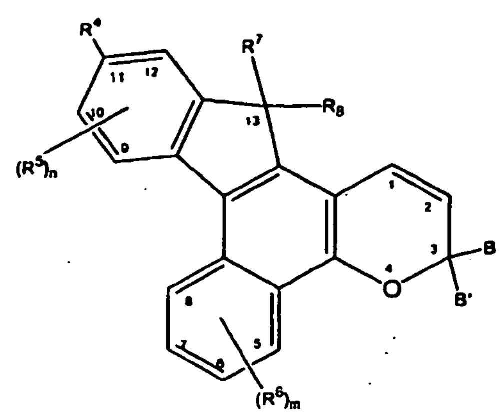

**(XIV)**

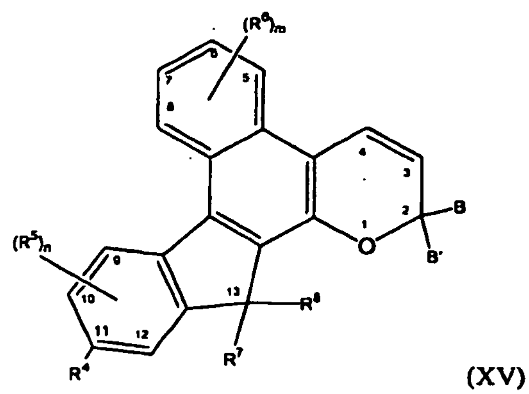

**(XV)**

With reference to (XIV) and (XV) above, according to various non-limiting embodiments disclosed herein $R^4$ may represent a substituted or unsubstituted aryl; a substituted or unsubstituted heteroaryl; or a group represented by -X=Y or -X'≡ Y'. Non-limiting examples of groups that X, X', Y and Y' may represent are set forth above. Suitable non-limiting examples of aryl and heteroaryl substituents are set forth above in detail

Alternatively, according to various non-limiting embodiments disclosed herein, the group represented by $R^4$ together with a group represented by an $R^5$ bonded at the 12-position of the indeno-fused naphthopyran or together with a group represented by an $R^5$ group bonded at the 10-position of the indeno-fused naphthopyran may form a fused group. Examples of suitable fused groups include, without limitation, indeno, dihydronaphthalene, indole, benzofuran, benzopyran and thianaphthene.

With continued reference to (XIV) and (XV), according to various non-limiting embodiments disclosed herein, "n" may range from 0 to 3 and "m" may range from 0 to 4. According to various non-limiting embodiments disclosed herein, where n is at least one and/or m is at least one, the groups represented by each $R^5$ and/or each $R^6$ may be independently chosen. Non-limiting examples of groups that $R^5$ and/or $R^6$ may represent include a reactive substituent; a compatibilizing substituent; hydrogen; $C_1$-$C_6$ alkyl; chloro; fluoro; $C_3$-$C_7$ cycloalkyl; a substituted or unsubstituted

phenyl, said phenyl substituents being $C_1$-$C_6$ alkyl or $C_1$-$C_6$ alkoxy; $-OR^{10}$ or $-OC(=O)R^{10}$, wherein $R^{10}$ may represent a group such as, but not limited to, S, hydrogen, amine, $C_1$-$C_6$ alkyl, phenyl($C_1$-$C_3$)alkyl, mono($C_1$-$C_6$)alkyl substituted phenyl($C_1$-$C_3$)alkyl, mono($C_1$-$C_6$)alkoxy substituted phenyl($C_1$-$C_3$) alkyl, ($C_1$-$C_6$)alkoxy($C_2$-$C_4$)alkyl, $C_3$-$C_7$ cycloalkyl and mono($C_1$-$C_4$)alkyl substituted $C_3$-$C_7$ cycloalkyl; a mono-substituted phenyl, said phenyl having a substituent located at the para position, the substituent being a dicarboxylic acid residue or derivative thereof, a diamine residue or derivative thereof, an amino alcohol residue or derivative thereof, a polyol residue or derivative thereof, $-(CH_2)-$, $-(CH_2)_t-$ or $-[O-(CH_2)_t]_k-$, wherein "t" may range from 2 to 6, and "k" may range from 1 to 50, and wherein the substituent may be connected to an aryl group on another photochromic material; and a nitrogen-containing group.

Non-limiting examples of nitrogen-containing groups that $R^5$ and/or $R^6$ may represent include $-N(R^{11})R^{12}$, wherein the groups represented by $R^{11}$ and $R^{12}$ may be the same or different. Examples of groups that $R^{11}$ and $R^{12}$ may represent according to various non-limiting embodiments disclosed herein include, without limitation, hydrogen, $C_1$-$C_8$ alkyl, phenyl, naphthyl, furanyl, benzofuran-2-yl, benzofuran-3-yl, thienyl, benzothien-2-yl, benzothien-3-yl, dibenzofuranyl, dibenzothienyl, benzopyridyl, fluorenyl, $C_1$-$C_8$ alkylaryl, $C_3$-$C_{20}$ cycloalkyl, $C_4$-$C_{20}$ bicycloalkyl, $C_5$-$C_{20}$ tricycloalkyl and $C_1$-$C_{20}$ alkoxyalkyl. Alternatively, according to various non-limiting embodiments, $R^{11}$ and $R^{12}$ may represent groups that come together with the nitrogen atom to form a $C_3$-$C_{20}$ hetero-bicycloalkyl ring or a $C_4$-$C_{20}$ hetero-tricycloalkyl ring.

Other non-limiting examples of a nitrogen containing groups that $R^5$ and/or $R^6$ may represent include nitrogen containing rings represented by (XVI) below.

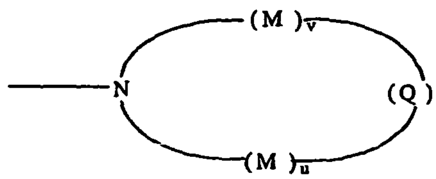

(XVI)

With reference to (XVI), non-limiting examples of groups that -M- may represent according to various non-limiting embodiments disclosed herein include $-CH_2-$, $-CH(R^{13})-$, $-C(R^{13})_2-$, -CH(aryl)-, $-C(aryl)_2-$ and $-C(R^{13})(aryl)-$. Non-limiting examples of groups that -Q- may represent according to various non-limiting embodiments disclosed herein include those discussed above for -M-, -O-, -S-, -S(O)-, $-SO_2-$, NH-, $-N(R^{13})-$ and -N(aryl)-. According to various non-limiting embodiments disclosed herein, each $R^{13}$ may independently represent $C_1$-$C_6$ alkyl, and each group designated "(aryl)" may independently represent phenyl or naphthyl. Further, according to various non-limiting embodiments disclosed herein, "u" may range from 1 to 3 and "v" may range from 0 to 3, provided that if v is 0, -Q- represents a group discussed above with respect to -M-.

Still other non-limiting examples of a suitable nitrogen containing groups that $R^5$ and/or $R^6$ may represent include groups represented by (XVIIA) or (XVIIB) below.

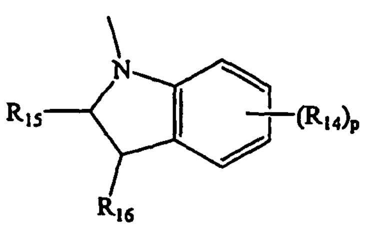

(XVIIA)

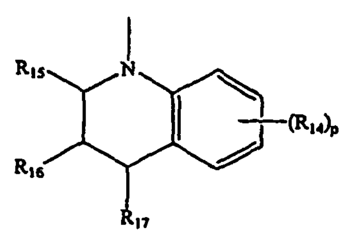

(XVIIB)

According to various non-limiting embodiments disclosed herein, the groups represented by $R^{15}$, $R^{16}$ and $R^{17}$ respectively in (XVIIA) and (XVIIB) above may be the same as or different from one another. Non-limiting examples of groups that $R^{15}$, $R^{16}$ and $R^{17}$ may independently represent according to various non-limiting embodiments disclosed herein include hydrogen, $C_1$-$C_6$ alkyl, phenyl, and naphthyl. Alternatively, according to various non-limiting embodiments, $R^{15}$ and $R^{16}$ may represent groups that together form a ring of 5 to 8 carbon atoms. Further, according to various non-liming embodiments disclosed herein, "p" may range from 0 to 3, and ifp is greater than one, each group represented by $R^{14}$ may be the same as or different from one or more other $R^{14}$ groups. Non-limiting examples of groups that $R^{14}$ may represent according to various non-limiting embodiments disclosed herein include $C_1$-$C_6$ alkyl, $C_1$-$C_6$ alkoxy, fluoro, and chloro.

Yet other non-limiting examples of nitrogen containing groups that $R^5$ and/or $R^6$ may represent include sub-

stituted or unsubstituted $C_4$-$C_{18}$ spirobicyclic amines and substituted or unsubstituted $C_4$-$C_{18}$ spirotricyclic amines. Non-limiting examples of spirobicyclic and spirotricyclic amine substituents include aryl, $C_1$-$C_6$ alkyl, $C_1$-$C_6$ alkoxy and phenyl ($C_1$-$C_6$)alkyl.

Alternatively, according to various non-limiting embodiments disclosed herein, a group represented by an $R^6$ in the 6-position and a group represented by an $R^6$ in the 7-position may together form a group represented by (XVIIIA) or (XVIIIB) below.

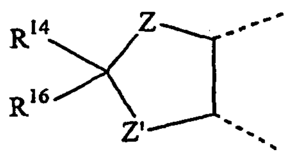
(XVIIIA)

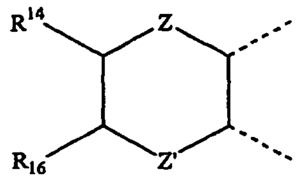
(XVIIIB)

In (XVIIIA) or (XVIIIB), the groups Z and Z' may be the same as or different from each other. Non-limiting examples of groups that Z and Z' may represent according to various non-limiting embodiments disclosed herein include oxygen and -$NR^{11}$-. Non-limiting examples of groups that $R^{11}$, $R^{14}$ and $R^{16}$ may represent according to various non-limiting embodiments disclosed herein include those discussed above.

Referring again to (XIV) and (XV), according to various non-limiting embodiments disclosed herein the groups represented by $R^7$ and $R^8$, respectively, may be the same or different. Non-limiting examples of groups that $R^7$ and $R^8$ may represent according to various non-limiting embodiments disclosed herein include a reactive substituent; a compatiblizing substituent; hydrogen; hydroxy, $C_1$-$C_6$ alkyl; $C_3$-$C_7$ cycloalkyl; allyl; a substituted or unsubstituted phenyl or benzyl, wherein each of said phenyl and benzyl group substituents is independently $C_1$-$C_6$ alkyl or $C_1$-$C_6$ alkoxy; chloro; fluoro; a substituted or unsubstituted amino; -C(O)$R^9$, wherein $R^9$ may represent groups such as, but not limited to, hydrogen, hydroxy, $C_1$-$C_6$ alkyl, $C_1$-$C_6$ alkoxy, the unsubstituted, mono- or di-substituted phenyl or naphthyl wherein each of said substituents is independently $C_1$-$C_6$ alkyl or $C_1$-$C_6$ alkoxy, phenoxy, mono- or di-($C_1$-$C_6$)akyl substituted phenoxy, mono- or di-($C_1$-$C_6$)alkoxy substituted phenoxy, amino, mono- or di-($C_1$-$C_6$)alkylamino, phenylamino, mono- or di-($C_1$-$C_6$)alkyl substituted phenylamino and mono- or di-($C_1$-$C_6$)alkoxy substituted phenylamino;-O$R^{18}$, wherein $R^{18}$ may represent groups such as, but not limited to, $C_1$-$C_6$ alkyl, phenyl($C_1$-$C_3$)alkyl, mono($C_1$-$C_6$)alkyl substituted phenyl ($C_1$-$C_3$)alkyl, mono($C_1$-$C_6$)alkoxy substituted phenyl($C_1$-$C_3$)alkyl, $C_1$-$C_6$ alkoxy($C_2$-$C_4$)alkyl, $C_3$-$C_7$ cycloalkyl, mono ($C_1$-$C_4$)alkyl substituted $C_3$-$C_7$ cycloalkyl, $C_1$-$C_6$ chloroakyl, $C_1$-$C_6$ fluoroalkyl, allyl and -CH($R^{19}$)T, wherein $R^{19}$ may represent hydrogen or $C_1$-$C_3$ alkyl, T may represent CN, $CF_3$ or COO$R^{20}$, wherein $R^{20}$ may represent hydrogen or $C_1$-$C_3$ alkyl, or wherein $R^{18}$ may be represented by - C(=O)U, wherein U may represents groups such as, but not limited to, hydrogen, $C_1$-$C_6$ alkyl, $C_1$-$C_6$ alkoxy, an unsubstituted, mono- or di-substituted phenyl or naphthyl wherein each of said substituents is independently $C_1$-$C_6$ alkyl or $C_1$-$C_6$ alkoxy, phenoxy, mono- or di-($C_1$-$C_6$)alkyl substituted phenoxy, mono- or di- ($C_1$-$C_6$)alkoxy substituted phenoxy, amino, mono-or di-($C_1$-$C_6$)alkylamino, phenylamino, mono- or di-($C_1$-$C_6$)alkyl substituted phenylamino or mono- and di-($C_1$-$C_6$)alkoxy substituted phenylamino; and a mono-substituted phenyl, said phenyl having a substituent located at the para position, the substituent being a dicarboxylic acid residue or derivative thereof, a diamine residue or derivative thereof, an amino alcohol residue or derivative thereof, a polyol residue or derivative thereof, $-(CH_2)-$, $-(CH_2)_t-$ or $-[O-(CH_2)_t]_k-$, wherein "t" may range from 2 to 6 and "k" may range from 1 to 50, and wherein the substituent may be connected to an aryl group on another photochromic material.

Alternatively, $R^7$ and $R^8$ may represent groups that may together form an oxo group; a spiro-carbocyclic group containing 3 to 6 carbon atoms (provided that the spiro-carbocyclic group is not norbornyl); or a spiro-heterocyclic group containing 1 to 2 oxygen atoms and 3 to 6 carbon atoms including the spirocarbon atom. Further, the spiro-carboxyclic and spiro-heterocyclic groups may be annellated with 0, 1, or 2 benzene rings.

Further according to various non-limiting embodiments, the groups represented by B and B' in (XIV) and (XV) may be the same or different. One non-limiting example of a group that B and/or B' may represent according to various non-limiting embodiments disclosed herein include an aryl group (for example, although not limiting herein, a phenyl group or a naphthyl group) that is mono-substituted with a reactive substituent and/or a compatiblizing substituent.

Other non-limiting examples of groups that B and B' may represent according to various non-limiting embodiments disclosed herein include an unsubstituted, mono-, di- or tri-substituted aryl group (such as, but not limited to, phenyl or naphthyl); 9-julolidinyl; an unsubstituted, mono- or di-substituted heteroaromatic group chosen from pyridyl, furanyl, benzofuran-2-yl, benzofuran-3-yl, thienyl, benzothien-2-yl, benzothien-3-yl, dibenzofuranyl, dibenzothienyl, carbazoyl, benzopyridyl, indolinyl and fluorenyl. Examples of suitable aryl and heteroaromatic substituent include, without limitation, hydroxy, aryl, mono- or di-($C_1$-$C_{12}$)alkoxyaryl, mono- or di-($C_1$-$C_{12}$)alkylaryl, haloaryl, $C_3$-$C_7$ cycloalkylaryl, $C_3$-$C_7$ cycloalkyl, $C_3$-$C_7$ cycloalkyloxy, $C_3$-$C_7$ cycloalkyloxy($C_1$-$C_{12}$)alkyl, $C_3$-$C_7$ cycloalkyloxy($C_1$-$C_{12}$)alkoxy, aryl ($C_1$-$C_{12}$)alkyl, aryl($C_1$-$C_{12}$)alkoxy, aryloxy, aryloxy($C_1$-$C_{12}$)alkyl, aryloxy($C_1$-$C_{12}$)akoxy, mono- or di($C_1$-$C_{12}$)alkylaryl

$(C_1\text{-}C_{12})$alkyl, mono- or di-$(C_1\text{-}C_{12})$alkoxyaryl$(C_1\text{-}C_{12})$alkyl, mono- or di-$(C_1\text{-}C_{12})$alkylaryl$(C_1\text{-}C_{12})$alkoxy, mono- or di-$(C_1\text{-}C_{12})$alkoxyaryl$(C_1\text{-}C_{12})$alkoxy, amino, mono- or di-$(C_1\text{-}C_{12})$alkylamino, diarylamino, piperazino, N-$(C_1\text{-}C_{12})$alkylpiperazino, N-arylpiperazino, aziridino, indolino, piperidino, morpholino, thiomorpholino, tetrahydroquinolino, tetrahydroisoquinolino, pyrrolidyl, $C_1\text{-}C_{12}$ alkyl, $C_1\text{-}C_{12}$ haloalkyl, $C_1\text{-}C_{12}$ Alkoxy, mono$(C_1\text{-}C_{12})$akoxy$(C_1\text{-}C_{12})$alkyl, acryloxy, methacryloxy, and halogen. Non-limiting examples of suitable halogen substituents include bromo, chloro and fluoro. Non-limiting examples of suitable aryl groups include phenyl and naphthyl.

Other non-limiting examples of suitable aryl and heteroaromatic substituents include those represented by $-C(=O)R^{21}$, wherein $R^{21}$ may represent groups such as, but not limited to, piperidino or morpholino, or $R^{21}$ may be represented by $-OR^{22}$ or $-N(R^{23})R^{24}$, wherein $R^{22}$ may represent groups, such as but not limited to allyl, $C_1\text{-}C_6$ alkyl, phenyl, mono$(C_1\text{-}C_6)$alkyl substituted phenyl, mono$(C_1\text{-}C_6)$alkoxy substituted phenyl, phenyl$(C_1\text{-}C_3)$alkyl, mono$(C_1\text{-}C_6)$alkyl substituted phenyl$(C_1\text{-}C_3)$alkyl, mono$(C_1\text{-}C_6)$alkoxy substituted phenyl$(C_1\text{-}C_3)$alkyl, $C_1\text{-}C_6$ alkoxy$(C_2\text{-}C_4)$alkyl and $C_1\text{-}C_6$ haloalkyl. Further, the groups represented by $R^{23}$ and $R^{24}$ may be the same or different and may include, without limitation $C_1\text{-}C_6$ alkyl, $C_5\text{-}C_7$ cycloalkyl and a substituted or unsubstituted phenyl, wherein said phenyl substituents may include $C_1\text{-}C_6$ alkyl and $C_1\text{-}C_6$ alkoxy. Non-limiting examples of suitable halogen substituents include bromo, chloro and fluoro.

Still other non-limiting examples of groups that B and B' may represent according to various non-limiting embodiments disclosed herein include an unsubstituted or mono-substituted group chosen from pyrazolyl, imidazolyl, pyrazolinyl, imidazolinyl, pyrrolinyl, phenothiazinyl, phenoxazinyl, phenazinyl and acridinyl, wherein said substituents may be $C_1\text{-}C_{12}$ alkyl, $C_1\text{-}C_{12}$ alkoxy, phenyl or halogen; and a mono-substituted phenyl, said phenyl having a substituent located at the para position, the substituent being a dicarboxylic acid residue or derivative thereof, a diamine residue or derivative thereof, an amino alcohol residue or derivative thereof, a polyol residue or derivative thereof, $-(CH_2)-$, $-(CH_2)_t-$ or $-[O-(CH_2)_t]_k-$, wherein "t" may range form 2 to 6 and "k" may range from 1 to 50, wherein the substituent may be connected to an aryl group on another photochromic material.

Yet other non-limiting examples of groups that B and B' may represent according to various non-limiting embodiments disclosed herein include groups represented by (XXIXA), (XXIXB) or (XXX) below.

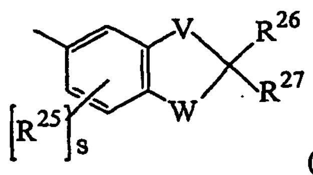
(XXIXA)

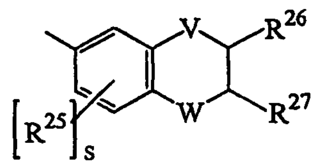
(XXIXB)

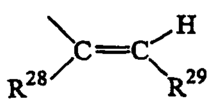
(XXX)

With reference to (XXIXA) and (XXIXB) above, non-limiting examples of groups that V may represent according to various non-limiting embodiments disclosed herein include represent $-CH_2-$ and -O-. Non-limiting examples of groups that W may represent according to various non-limiting embodiments disclosed herein include oxygen and substituted nitrogen, provided that if W is substituted nitrogen, V is $-CH_2-$. Suitable non-limiting examples of nitrogen substituents include hydrogen, $C_1\text{-}C_{12}$ alkyl and $C_1\text{-}C_{12}$ acyl. Further, according to various non-limiting embodiments disclosed herein, "s" may range from 0 to 2, and, if s is greater than one, each group represented by $R^{25}$ may be the same as or different from one or more other $R^{25}$ groups. Non-liming examples of groups that $R^{25}$ may represent include $C_1\text{-}C_{12}$ alkyl, $C_1\text{-}C_{12}$ alkoxy, hydroxy and halogen. Non-limiting examples of groups that $R^{26}$ and $R^{27}$ may represent according to various non-limiting embodiments disclosed herein include hydrogen and $C_1\text{-}C_{12}$ alkyl.

With reference to (XXX) above, non-limiting examples of groups that $R^{28}$ may represent according to various non-limiting embodiments disclosed herein include hydrogen and $C_1\text{-}C_{12}$ alkyl. Non-limiting examples of groups that $R^{29}$ may represent according to various non-limiting embodiments disclosed herein include an unsubstituted, mono- or di-substituted naphthyl, phenyl, furanyl or thienyl, said substituents being $C_1\text{-}C_{12}$ alkyl, $C_1\text{-}C_{12}$ alkoxy or halogen.

Alternatively, B and B' may represent groups that, taken together, may form a fluoren-9-ylidene or mono- or di-substituted fluoren-9-ylidene, each of said fluoren-9-ylidene substituents independently being $C_1\text{-}C_{12}$ alkyl, $C_1\text{-}C_{12}$ alkoxy or halogen.

As previously discussed, the photochromic materials comprising a group that extends the pi-conjugated system of the indeno-fused naphthopyran bonded at the 11-position thereof may be further linked to another photochromic material and may further comprise a reactive and/or compatibilizing substituent, such as, but not limited to those set forth above. For example, referring again to Fig. 2a, there is shown a photochromic material according to various non-limiting embodiments disclosed herein, wherein the indeno-fused naphthopyran is an indeno[2',3':3,4]naphtho[1,2-b] pyran (for example, as represented by (XIV) above), wherein the group that extends the pi-conjugated system of the indeno-fused naphthopyran bonded at the 11-position thereof (e.g., a group represented by $R^4$) may be represented by -X=Y, wherein X represents $-CR^1$ and Y is O (i.e., $-C(=O)R^1$), wherein $R^1$ represents a heterocyclic group (e.g., a piperazino as shown in Fig. 2a) that is substituted with a photochromic material (e.g., a 3,3-diphenyl-6,11-dimethoxy-13,13 dimethyl-3H,13H-indeno[2',3':3,4]naphtho[1,2-b]pyran as shown in Fig. 2a). Further, although not limiting herein, as shown in Fig. 2a, the group represented by B (on the indeno-fused naphthopyran comprising the group that extends the pi-conjugated system of the indeno-fused naphthopyran bonded at the 11-position thereof) may comprise a reactive substituent that may be represented by -A'-D-J. That is, according to this non-limiting embodiment, the group represented by B may be an aryl group (e.g., a phenyl group as shown in Fig. 2a) that is mono-substituted with a reactive substituent (e.g., (2-methacryloxyethyl)carbamyloxy as shown in Fig. 2a) that may be represented by -A'-D-J, wherein A' is (-OC (=O)-), -D- is the residue of an amino alcohol wherein an amino nitrogen is bonded to -A'- and an alcohol oxygen is bonded to -J, and -J is methacryl.

According to another non-limiting embodiment wherein the photochromic material is represented by (XIV) or (XV) above, or a mixture thereof, at least one of a group represented by an $R^6$ at the 6-position, an $R^6$ group at the 7-position, B, B', $R^7$, $R^8$ or $R^4$ may comprise a reactive and/or compatibilizing substituent.

According to still another non-limiting embodiment, wherein the photochromic material is an [2',3':3,4]naphtho [1,2-b]pyran represented by (XIV) above, each of a group represented by an $R^6$ group at the 7-position and an $R^6$ group at the 6-position of the indeno[2',3':3,4]naphtho[1,2-b]pyran may be independently an oxygen containing group represented by $-OR^{10}$, wherein $R^{10}$ may represent groups including $C_1$-$C_6$ alkyl, a substituted or unsubstituted phenyl wherein said phenyl substituents may be $C_1$-$C_6$ alkyl or $C_1$-$C_6$ alkoxy, phenyl($C_1$-$C_3$)alkyl, mono($C_1$-$C_6$)alkyl substituted phenyl ($C_1$-$C_3$)alkyl, mono($C_1$-$C_6$)alkoxy substituted phenyl($C_1$-$C_3$)alkyl, ($C_1$-$C_6$)alkoxy($C_2$-$C_4$)alkyl, $C_3$-$C_7$ cycloalkyl and mono ($C_1$-$C_4$)alkyl substituted $C_3$-$C_7$ cycloalkyl; a nitrogen-containing group represented by $-N(R^{11})R^{12}$, wherein $R^{11}$ and $R^{12}$ may represent the same or different groups, which may include, without limitation hydrogen, $C_1$-$C_8$ alkyl, $C_1$-$C_8$ alkylaryl, $C_3$-$C_{20}$ cycloalkyl, $C_4$-$C_{20}$ bicycloalkyl, $C_5$-$C_{20}$ tricycloalkyl and $C_1$-$C_{20}$ alkoxyalkyl, wherein said aryl group may be phenyl or naphthyl; the nitrogen containing ring represented by (XVI) above, wherein each -M- may represent a group such as $-CH_2-$, $-CH(R^{13})-$, $-C(R^{13})_2-$, -CH(aryl)-, $-C(aryl)_2-$ or $-C(R^{13})(aryl)-$, and -Q-may represent a group such as those set forth above for -M-, -O-, -S-, -NH-, $-N(R^{13})-$ or-N(aryl)-, wherein each $R^{13}$ may independently represent $C_1$-$C_6$ alkyl and each group designated (aryl) independently may represent phenyl or naphthyl, u ranges from 1 to 3, and v ranges from 0 to 3, provided that when v is 0, -Q- represents a group set forth above for -M-; or a reactive substituent, provided that the reactive substituent comprises a linking group comprising an aliphatic amino alcohol residue, a cyclo aliphatic amino alcohol residue, an azacyclo aliphatic alcohol residue, a diazacyclo aliphatic alcohol residue, a diamine residue, an aliphatic diamine residue, a cyclo aliphatic diamine residue, a diazacycloalkane residue, an azacyclo aliphatic amine residue, an oxyalkoxy group, an aliphatic polyol residue or a cyclo aliphatic polyol residue that forms a bond with the indeno[2',3':3,4]naphtho[1,2-b]pyran at the 6-position or the 7-position. Alternatively, according to this non-limiting embodiment, a group represented by an $R^6$ group in the 6-position and a group represented by an $R^6$ group in the 7-position of the indeno[2',3':3,4]naphtho[1,2-b]pyran may together form a group represented (XVIIIA) or (XVIIIB) above, wherein the groups represented by Z and Z' may be the same or different, and may include oxygen and the group $-NR^{11}-$, where $R^{11}$ represents a group as set forth above.

Further, according various non-limiting embodiments disclosed herein, the groups represented by $R^7$ and $R^8$ may each independently be hydrogen, $C_1$-$C_6$ alkyl, $C_3$-$C_7$ cycloalkyl, allyl, a substituted or unsubstituted phenyl or benzyl, a substituted or unsubstituted amino, and a group $-C(O)R^9$, wherein $R^9$ may represent groups including, without limitation, hydrogen, hydroxy, $C_1$-$C_6$ alkyl, $C_1$-$C_6$ alkoxy, the unsubstituted, mono-or di-substituted aryl groups phenyl or naphthyl, phenoxy, mono- or di-($C_1$-$C_6$)alkoxy substituted phenoxy, and mono- or di-($C_1$-$C_6$)alkoxy substituted phenoxy.

Still other non-limiting embodiments disclosed herein relate to photochromic materials comprising: (i) a naphthopyran, said a naphthopyran being at least one of a benzofurano-fused naphthopyran, an indolo-fused naphthopyran or a benzothieno-fused naphthopyran; and (ii) a group that extends the pi-conjugated system of the naphthopyran bonded at the 11-position thereof. Although not limiting herein, the naphthopyrans according to these non-limiting embodiments may be generally represented by structures (XXXI) and (XXXII) below, wherein X* is O, N, or S.

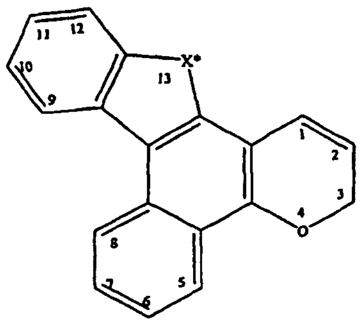

(XXXI)

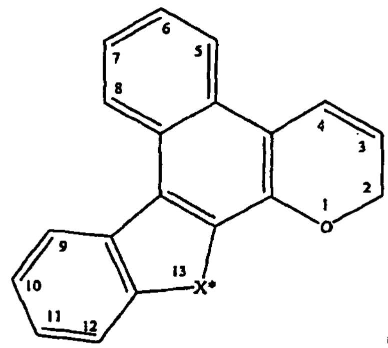

(XXXII)

Non-limiting examples of 11-position groups that may extend the pi-conjugated system of the benzofurano-fused naphthopyrans, the indolo-fused naphthopyrans and the benzothieno-fused naphthopyrans according to various non-limiting embodiments disclosed herein include those 11-position groups that may extend the pi-conjugated system of the indeno-fused naphthopyrans discussed above. For example, according to various non-limiting embodiments disclosed herein, the group that extends the pi-conjugated system of the naphthopyran bonded at the 11-position thereof may be a substituted or unsubstituted aryl group (non-limiting examples of which are set forth above), a substituted or unsubstituted heteroaryl group (non-limiting examples of which are set forth above), or a group represented by -X=Y or -X' ≡Y', wherein X, Y, X' and Y' may represent groups as set forth above in detail.

Alternatively, according to various non-limiting embodiments disclosed herein, the group that extends the pi-conjugated system of the benzofurano-fused naphthopyran, the indolo-fused naphthopyran or the benzothieno-fused naphthopyran bonded at the 11-position thereof together with a group bonded at the 12-position of said naphthopyran or together with a group bonded at the 10-position of said naphthopyran may form a fused group. Although not required, according one non-limiting embodiment wherein the group bonded at the 11-position together with a group bonded at the 12-position or the 10-position forms a fused group, the fused group may extends the pi-conjugated system of the benzofurano-fused naphthopyran, the indolo-fused naphthopyran or the benzothieno-fused naphthopyran at the 11-position, but not the 10-position or the 12-position thereof. Suitable non-limiting examples of such fused groups include indeno, dihydronaphthalene, indole, benzofuran, benzopyran and thianaphthene.

Further, according to various non-limiting embodiments, the 13-position of the indolo-fused naphthopyran may be unsubstituted or mono-substituted. Non-limiting examples of suitable 13-position substituents include those discussed with respect to $R^7$ and $R^8$ in structures (XIV) and (XV) above.

Suitable non-limiting examples of groups that may be bonded at the 4-, 5-, 6-, 7-, 8-, 9-,10-, and 12-positions of the benzofurano-fused naphthopyran, the indolo-fused naphthopyran or the benzothieno-fused naphthopyran according to various non-limiting embodiments include those groups discussed with respect to $R^5$ and $R^6$ in structures (XIV) and (XV) above. Suitable non-limiting examples of groups that may be bonded at the 3-position of the benzofurano-fused naphthopyran, the indolo-fused naphthopyran or the benzothieno-fused naphthopyran represented by (XXXI) or the 2-position of the benzofurano-fused naphthopyran, the indolo-fused naphthopyran or the benzothieno-fused naphthopyran represented by (XXXII) according to various non-limiting embodiments include those groups discussed with respect to B and B' in structures (XIV) and (XV) above

Methods of making photochromic materials comprising indeno-fused naphthopyrans according to various non-limiting embodiments disclosed herein will now be discussed with reference to the general reaction schemes presented in Figs. 4-8. Fig. 4 depicts a reaction scheme for making substituted 7H-benzo[C]fluoren-5-ol compounds that may be further reacted as shown in Figs. 5-8 to form photochromic materials comprising an indeno-fused naphthopyran and a group that extends the pi-conjugated system of the indeno-fused naphthopyran bonded at the 11-position thereof according to various non-limiting embodiments disclosed herein. It should be appreciated that these reaction schemes are presented for illustration only and are not intended to be limiting herein. Additional examples of methods of making photochromic materials according to various non-limiting embodiments disclosed herein are set forth in the Examples.

Referring now to Fig. 4, a solution of a $\gamma$-substituted benzoyl chloride, represented by structure (a) in Fig. 4, and benzene, represented by structure (b) in Fig. 4, which may have one or more substituents $\gamma^1$, in methylene chloride are added to a reaction flask. Suitable $\gamma$-substituents include, for example and without limitation, halogen. Suitable $\gamma^1$ substituents include, for example and without limitation, those groups set forth above for $R^6$. Anhydrous aluminum chloride catalyzes the Friedel-Crafts acylation to give a substituted benzophenone represented by structure **(c)** in Fig. 4. This material is then reacted in a Stobbe reaction with dimethyl succinate to produce a mixture of half-esters, one of which is represented by structure **(d)** in Fig. 4. Thereafter the half-esters are reacted in acetic anhydride and toluene at an elevated temperature to produce, after recrystallization, a mixture of substituted naphthalene compounds, one of which is represented by structure **(e)** in Fig. 4. The mixture of substituted naphthalene compounds is then reacted with

methyl magnesium chloride to produce a mixture of substituted naphthalene compounds, one of which is represented by structure **(f)** in Fig. 4. The mixture of substituted naphthalene compounds is then cyclized with dodecylbenzene sulfonic acid to afford a mixture of 7H-benzo[C]fluoren-5-ol compounds, one of which is represented by structure **(g)** in Fig. 4.

Referring now to Fig. 5, the 7H-benzo[C]fluoren-5-ol compound represented by structure **(g)** is refluxed with copper cyanide in anhydrous 1-methyl-2-pyrrolidinone to give, upon workup, a 9-cyano-7H-benzo[C]fluoren-5-ol compound represented by structure **(h)**. As further indicated in PATH A of Fig. 5, the compound represented by structure **(h)** may be further reacted with a propargyl alcohol represented by structure **(i)** to produce the indeno-fused naphthopyran (represented by structure **(j)** in Fig. 5) according to one non-limiting embodiment disclosed herein, wherein a cyano group that extends the pi-conjugated system of the indeno-fused naphthopyran is bonded at the 11-position thereof. Suitable non-limiting examples of groups that B and B' may represent are discussed above.

Alternatively, as shown in PATH B of Fig. 5, the compound represented by structure **(h)** may be hydrolyzed with aqueous sodium hydroxide under reflux conditions to produce the 9-carboxy-7H-benzo[C]fluoren-5-ol compound represented by structure **(k)** in Fig. 5. As further indicated in Fig. 5, the compound represented by structure **(k)** may be further reacted with a propargyl alcohol represented by structure **(i)** to produce the indeno-fused naphthopyran (represented by structure **(l)** in Fig. 5) according to one non-limiting embodiment disclosed herein, wherein a carboxy group that extends the pi-conjugated system of the indeno-fused naphthopyran is bonded at the 11-position thereof.

Alternatively, as shown in PATH C of Fig. 5, the compound represented by structure **(k)** may be esterified with an alcohol (represented by the formula $\gamma^2$ OH in Fig. 5) in aqueous hydrochloric acid to produce the 9-$\gamma^2$carboxyl-7H-benzo[C]fluoren-5-ol compound represented by structure **(m)** in Fig. 5. Examples of suitable alcohols include, without limitation, methanol, diethylene glycol, alkyl alcohol, substituted and unsubstituted phenols, substituted and unsubstituted benzyl alcohols, polyols and polyol residues, such as, but not limited to, those discussed above with respect to -G-. The compound represented by structure **(m)** may be further reacted with a propargyl alcohol represented by structure **(i)** to produce the indeno-fused naphthopyran (represented by structure **(n)** in Fig. 5) according to one non-limiting embodiment disclosed herein, wherein a carbonyl group that extends the pi-conjugated system of the indeno-fused naphthopyran is bonded at the 11-position thereof. Non-limiting examples of carbonyl groups that may be bonded at the 11-position according to various non-limiting embodiments disclosed herein include methoxycarbonyl, 2-(2-hydroxyethoxy)ethoxycarbonyl, alkoxycarbonyl, substituted and unsubstituted phenoxycarbonyl, substituted and unsubstituted benzyloxycarbonyl and esters of polyols.

Referring now to Fig.6, the 7H-benzo[C]fluoren-5-ol compound represented by structure (g) may be reacted with a phenyl boronic acid represented by structure **(o)**, which may be substituted with a group represented by $\gamma^3$ as shown in Fig. 6, to form the 9-(4-$\gamma^3$-phenyl)-7H-benzo[C]fluoren-5-ol compound represented by structure **(p)** in Fig. 6. Examples of suitable boronic acids include, without limitation, substituted and unsubstituted phenylboronic acids, 4-fluorophenylboronic acid, (4-hydroxymethyl)phenylboronic acid, biphenylboronic acid, and substituted and unsubstituted arylboronic acids. The compound represented by structure **(p)** may be further reacted with a propargyl alcohol represented by structure **(i)** to produce the indeno-fused naphthopyran (represented by structure **(q)** in Fig. 6), wherein a phenyl group that extends the pi-conjugated system of the indeno-fused naphthopyran is bonded at the 11-position thereof. Although not required, according to various non-limiting embodiments disclosed herein and as shown in Fig. 6, the phenyl group bonded at the 11-position may be substituted. Non-limiting examples of substituted phenyl groups that may be bonded at the 11-position according to various non-limiting embodiments disclosed herein include 4-fluorophenyl, 4-(hydroxymethyl)phenyl, 4-(phenyl)phenyl group, alkylphenyl, alkoxyphenyl, halophenyl, and alkoxycarbonylphenyl. Further, the substituted phenyl at the 11-position may have up to five substituents, and those substituents may be a variety of different substituents at any of the positions ortho, meta or para to the indeno-fused naphthopyran.

Referring now to Fig. 7, the 7H-benzo[C]fluoren-5-ol compound represented by structure **(g)** may be coupled in the presence of a palladium catalysis with a terminal alkyne group represented by structure **(r)**, which may be substituted with a group represented by $\gamma^4$ as shown in Fig. 7, to form the 9-alkynyl-7H-benzo[C]fluoren-5-ol compound represented by structure '**(s)**' in Fig. 7. Examples of suitable terminal alkynes include, without limitation, acetylene, 2-methyl-3-butyn-2-ol, phenylacetylene, and alkylacetylene. The compound represented by structure '**(s)**' may be further reacted with a propargyl alcohol represented by structure **(i)** to produce the indeno-fused naphthopyran (represented by structure **(t)** in Fig. 7) having an alkynyl group that extends the pi-conjugated system of the indeno-fused naphthopyran bonded at the 11-position thereof. Although not required, as shown in Fig. 7, the alkynyl group bonded at the 11-position may be substituted with a group represented by $\gamma^4$. Non-limiting examples of alkynyl groups that may be bonded at the 11-position according to various non-limiting embodiments disclosed herein include ethynyl, 3-hydroxy-3-methylbutynl, 2-phenylethynyl and alkyl acetylenes.

Referring now to Fig. 8, the 7H-benzo[C]fluoren-5-ol compound represented by structure **(g)** may be reacted with an alkene represented by structure **(u)**, which may be substituted with a group represented by $\gamma^5$ as shown in Fig. 8, to form the 9-alkenyl-7H-benzo[C]fluoren-5-ol compound represented by structure **(v)** in Fig. 8. Examples of suitable alkenes include, without limitation 1-hexene, styrenes, and vinyl chlorides. The compound represented by structure **(v)**

may be further reacted with a propargyl alcohol represented by structure **(i)** to produce the indeno-fused naphthopyran (represented by structure **(w)** in Fig. 8) having an alkenyl group that extends the pi-conjugated system of the indeno-fused naphthopyran bonded at the 11-position thereof. Although not required, as shown in Fig. 8, the alkenyl group bonded at the 11-position may be substituted with up to three $\gamma^5$ groups. Non-limiting examples of alkenyl groups that may be bonded at the 11-position according to various non-limiting embodiments disclosed herein include substituted and unsubstituted ethylenes, 2-phenyl ethylenes, and 2-chloroethylenes.

Further, non-limiting examples of methods of forming benzofurano-fused naphthopyrans, indolo-fused naphthopyrans, and/or benzothieno-fused naphthopyrans that may be useful (with appropriate modifications that will be recognized by those skilled) in forming the benzofurano-fused naphthopyrans, indolo-fused naphthopyrans and/or benzothieno-fused naphthopyrans according to various non-limiting embodiments disclosed herein are set forth in U.S. Patent No. 5,651,923 at col. 6, line 43 to col. 13, line 48; International Patent Application Publication No. WO98/28289A1 at page 7, line 12 to page 9, line 10; and International Patent Application Publication No. WO99/23071 A1 at page 9, lines 1 to page 14, line 3.

As discussed above, the photochromic materials according to various non-limiting embodiments disclosed herein may be incorporated into at least a portion of an organic material, such as a polymeric, oligomeric or monomeric material to form a photochromic composition, which may be used, for example and without limitation, to form photochromic articles, such as optical elements, and coating compositions that may be applied to various substrates. As used herein the terms "polymer" and "polymeric material" refer to homopolymers and copolymers (e.g., random copolymers, block copolymers, and alternating copolymers), as well as blends and other combinations thereof. As used herein the terms "oligomer" and "oligomeric material," refer to a combination of two or more monomer units that is capable of reacting with additional monomer unit(s). As used herein the term "incorporated into" means physically and/or chemically combined with. For example, the photochromic materials according to various non-limiting embodiments disclosed herein may be physically combined with at least a portion of an organic material, for example and without limitation, by mixing or imbibing the photochromic material into the organic material; and/or chemically combined with at least a portion of an organic material, for example and without limitation, by copolymerization or otherwise bonding the photochromic material to the organic material.

Further, it is contemplated that the photochromic materials according to various non-limiting embodiments disclosed herein may each be used alone, in combination with other photochromic materials according to various non-limiting embodiments disclosed herein, or in combination with an appropriate complementary conventional photochromic material. For example, the photochromic materials according to various non-limiting embodiments disclosed herein may be used in conjunction with conventional photochromic materials having activated absorption maxima within the range of 300 to 1000 nanometers. Further, the photochromic materials according to various non-limiting embodiments disclosed herein may be used in conjunction with a complementary conventional polymerizable or a compatiblized photochromic material, such as for example, those disclosed in U.S. Patent Nos. 6,113,814 (at col. 2, line 39 to col. 8, line 41), and 6,555,028 (at col. 2, line 65 to col. 12, line 56).

As discussed above, according to various non-limiting embodiments disclosed herein, the photochromic compositions may contain a mixture of photochromic materials. For example, although not limiting herein, mixtures of photochromic materials may be used to attain certain activated colors such as a near neutral gray or near neutral brown. See, for example, U.S. Patent No. 5,645,767, col. 12, line 66 to col. 13, line 19, which describes the parameters that define neutral gray and brown colors.

Various non-limiting embodiments disclosed herein provide a photochromic composition comprising an organic material, said organic material being at least one of polymeric material, an oligomeric material and a monomeric material, and a photochromic material according to any of the non-limiting embodiments of set forth above incorporated into at least a portion of the organic material. According to various non-limiting embodiments disclosed herein, the photochromic material may be incorporated into a portion of the organic material by at least one of blending and bonding the photochromic material with the organic material or a precursor thereof. As used herein with reference to the incorporation of photochromic materials into an organic material, the terms "blending" and "blended" mean that the photochromic material is intermixed or intermingled with at least a portion of the organic material, but not bonded to the organic material. Further, as used herein with reference to the incorporation of photochromic materials into an organic material, the terms "bonding" or "bonded" mean that the photochromic material is linked to a portion of the organic material or a precursor thereof. For example, although not limiting herein, the photochromic material may be linked to the organic material through a reactive substituent.

According to one non-limiting embodiment wherein the organic material is a polymeric material, the photochromic material may be incorporated into at least a portion of the polymeric material or at least a portion of the monomeric material or oligomeric material from which the polymeric material is formed. For example, photochromic materials according to various non-limiting embodiments disclosed herein that have a reactive substituent may be bonded to an organic material such as a monomer, oligomer, or polymer having a group with which a reactive moiety may be reacted, or the reactive moiety may be reacted as a co-monomer in the polymerization reaction from which the organic material

is formed, for example, in a co-polymerization process.

As discussed above, the photochromic compositions according to various non-limiting embodiments disclosed herein may comprise an organic material chosen from a polymeric material, an oligomeric material and/or a monomeric material. Examples of polymeric materials that may be used in conjunction with various non-limiting embodiments disclosed herein include, without limitation: polymers ofbis(allyl carbonate) monomers; diethylene glycol dimethacrylate monomers; diisopropenyl benzene monomers; ethoxylated bisphenol A dimethacrylate monomers; ethylene glycol bismethacrylate monomers; poly(ethylene glycol) bismethacrylate monomers; ethoxylated phenol bismethacrylate monomers; alkoxylated polyhydric alcohol acrylate monomers, such as ethoxylated trimethylol propane triacrylate monomers; urethane acrylate monomers; vinylbenzene monomers; and styrene. Other non-limiting examples of suitable polymeric materials include polymers of polyfunctional, e.g., mono-, di- or multi-functional, acrylate and/or methacrylate monomers; poly($C_1$-$C_{12}$ alkyl methacrylates), such as poly(methyl methacrylate); poly(oxyalkylene)dimethacrylate; poly(alkoxylated phenol methacrylates); cellulose acetate; cellulose triacetate; cellulose acetate propionate; cellulose acetate butyrate; poly(vinyl acetate); poly(vinyl alcohol); poly(vinyl chloride); poly(vinylidene chloride); polyurethanes; polythiourethanes; thermoplastic polycarbonates; polyesters; polyethylene terephthalate); polystyrene; poly($\alpha$-methylstyrene); copolymers of styrene and methyl methacrylate; copolymers of styrene and acrylonitrile; polyvinylbutyral; and polymers of diallylidene pentaerythritol, particularly copolymers with polyol (allyl carbonate) monomers, e.g., diethylene glycol bis(allyl carbonate), and acrylate monomers, e.g., ethyl acrylate, butyl acrylate. Also contemplated are copolymers of the aforementioned monomers, combinations, and blends of the aforementioned polymers and copolymers with other polymers, e.g., to form interpenetrating network products.

Further, according to various non-limiting embodiments wherein transparency of the photochromic composition is desired, the organic material may be a transparent polymeric material. For example, according to various non-limiting embodiments, the polymeric material may be an optically clear polymeric material prepared from a thermoplastic polycarbonate resin, such as the resin derived from bisphenol A and phosgene, which is sold under the trademark, LEXAN®; a polyester, such as the material sold under the trademark, MYLAR®; a poly(methyl methacrylate), such as the material sold under the trademark, PLEXIGLAS®; and polymerizates of a polyol(allyl carbonate) monomer, especially diethylene glycol bis(allyl carbonate), which monomer is sold under the trademark CR-39®; and polyurea-polyurethane (polyurea urethane) polymers, which are prepared, for example, by the reaction of a polyurethane oligomer and a diamine curing agent, a composition for one such polymer being sold under the trademark TRIVEX® by PPG Industries, Inc. Other non-limiting examples of suitable polymeric materials include polymerizates of copolymers of a polyol (allyl carbonate), e.g., diethylene glycol bis(allyl carbonate), with other copolymerizable monomeric materials, such as, but not limited to: copolymers with vinyl acetate, copolymers with a polyurethane having terminal diacrylate functionality, and copolymers with aliphatic urethanes, the terminal portion of which contain allyl or acrylyl functional groups. Still other suitable polymeric materials include, without limitation, poly(vinyl acetate), polyvinylbutyral, polyurethane, polythiourethanes, polymers chosen from diethylene glycol dimethacrylate monomers, diisopropenyl benzene monomers, ethoxylated bisphenol A dimethacrylate monomers, ethylene glycol bismethacrylate monomers, poly(ethylene glycol) bismethacrylate monomers, ethoxylated phenol bismethacrylate monomers and ethoxylated trimethylol propane triacrylate monomers, cellulose acetate, cellulose propionate, cellulose butyrate, cellulose acetate butyrate, polystyrene and copolymers of styrene with methyl methacrylate, vinyl acetate and acrylonitrile. According to one non-limiting embodiment, the polymeric material may be an optical resins sold by PPG Industries, Inc. under the CR-designation, e.g., CR-307, CR-407, and CR-607.

According to one specific non-limiting embodiment, the organic material may be a polymeric material is chosen from poly(carbonate), copolymers of ethylene and vinyl acetate; copolymers of ethylene and vinyl alcohol; copolymer of ethylene, vinyl acetate, and vinyl alcohol (such as those that result from the partial saponification of copolymers of ethylene and vinyl acetate); cellulose acetate butyrate; poly(urethane); poly(acrylate); poly(methacrylate); epoxies; aminoplast functional polymers; poly(anhydride); poly(urea urethane); N-alkoxymethyl(meth)acrylamide functional polymers; poly(siloxane); poly(silane); and combinations and mixtures thereof.

As previously discussed, it has been observed by the inventors that the photochromic materials according to certain non-limiting embodiments disclosed herein may display hyperchromic absorption of electromagnetic radiation having a wavelength from 320 nm to 420 nm as compared to a photochromic materials comprising a comparable indeno-fused naphthopyran without the group that extends the pi-conjugated system of the comparable indeno-fused naphthopyran bonded at the 11-position thereof. Accordingly, photochromic compositions comprising the photochromic materials according to various non-limiting embodiments disclosed herein may also displays increased absorption of electromagnetic radiation having a wavelength from 320 nm to 420 nm as compared to a photochromic composition comprising a comparable indeno-fused naphthopyran without the group that extends the pi-conjugated system of the comparable indeno-fused naphthopyran bonded at the 11-position thereof.

Additionally, as previously discussed, since the photochromic materials according to certain non-limiting embodiments disclosed herein may display hyperchromic properties as discussed above, it is contemplated that the amount or concentration of the photochromic material present in photochromic compositions according to various non-limiting embodiments disclosed herein may be reduced as compared to the amount or concentration of a conventional photochromic materials that is typically required to achieve a desired optical effect. Since it may be possible to use less of the photochromic materials according to certain non-limiting embodiments disclosed herein than conventional photochromic materials while still achieving the desired optical effects, it is contemplated that the photochromic materials according to various non-limiting embodiments disclosed herein may be advantageously employed in applications wherein it is necessary or desirable to limit the amount of photochromic material used.

Further, as previously discussed, it has been observed by the inventors that the photochromic materials according to certain non-limiting embodiments disclosed herein may have a closed-form absorption spectrum for electromagnetic radiation having a wavelength ranging from 320 nm to 420 nm that is bathochromically shifted as compared to a closed-form absorption spectrum for electromagnetic radiation having a wavelength ranging from 320 nm to 420 nm of a photochromic material comprising a comparable indeno-fused naphthopyran without the group that extends the pi-conjugated system of comparable the indeno-fused naphthopyran bonded at the 11-position thereof. Accordingly, Photochromic compositions comprise the photochromic materials according to various non-limiting embodiments disclosed herein may also have an absorption spectrum for electromagnetic radiation having a wavelength ranging from 320 nm to 420 nm that is bathochromically shifted as compared to an absorption spectrum for electromagnetic radiation having a wavelength ranging from 320 nm to 420 nm of a photochromic composition comprising a comparable indeno-fused naphthopyran without the group that extends the pi-conjugated system of the comparable indeno-fused naphthopyran bonded at the 11-position thereof.

As previously discussed, the present invention further contemplates photochromic articles, such as optical elements, made using the photochromic materials and compositions according to various non-limiting embodiments disclosed herein. As used herein the term "optical" means pertaining to or associated with light and/or vision. The optical elements according to various non-limiting embodiments disclosed herein may include, without limitation, ophthalmic elements, display elements, windows, mirrors, and liquid crystal cell elements. As used herein the term "ophthalmic" means pertaining to or associated with the eye and vision. Non-limiting examples of ophthalmic elements include corrective and non-corrective lenses, including single vision or multi-vision lenses, which may be either segmented or non-segmented multi-vision lenses (such as, but not limited to, bifocal lenses, trifocal lenses and progressive lenses), as well as other elements used to correct, protect, or enhance (cosmetically or otherwise) vision, including without limitation, magnifying lenses, protective lenses, visors, goggles, as well as, lenses for optical instruments (for example, cameras and telescopes). As used herein the term "display" means the visible or machine-readable representation of information in words, numbers, symbols, designs or drawings. Non-limiting examples of display elements include screens, monitors, and security elements, such as security marks. As used herein the term "window" means an aperture adapted to permit the transmission of radiation therethrough. Non-limiting examples of windows include automotive and aircraft transparencies, windshields, filters, shutters, and optical switches. As used herein the term "mirror" means a surface that specularly reflects a large fraction of incident light. As used herein the term "liquid crystal cell" refers to a structure containing a liquid crystal material that is capable of being ordered. One non-limiting example of a liquid crystal cell element is a liquid crystal display.

Various non-limiting embodiments disclosed herein provide photochromic articles, such as optical elements, comprising a substrate and a photochromic material according to any of the non-limiting embodiments discussed above connected to a portion of the substrate. As used herein, the term "connected to" means associated with, either directly or indirectly through another material or structure.

According to various non-limiting embodiments disclosed herein wherein the substrate of the photochromic article comprises a polymeric material, the photochromic material may be connected to at least a portion of the substrate by incorporating the photochromic material into at least a portion of the polymeric material of the substrate, or by incorporating the photochromic material into at least a portion of the oligomeric or monomeric material from which the substrate is formed. For example, according to one non-limiting embodiment, the photochromic material may be incorporated into the polymeric material of the substrate by the cast-in-place method or by imbibition. Imbibition and the cast-in-place method are discussed below.

According to still other non-limiting embodiments, the photochromic material may be connected to at least a portion of the substrate of the photochromic article as part of at least partial coating that is connected to at least a portion of a substrate. According to this non-limiting embodiment, the substrate may be a polymeric substrate or an inorganic substrate (such as, but not limited to, a glass substrate). Further, the photochromic material may be incorporated into at least a portion of a coating composition prior to application of the coating composition to the substrate, or alternatively, a coating composition may be applied to the substrate, at least partially set, and thereafter the photochromic material may be imbibed into at least a portion of the coating. As used herein, the terms "set" and "setting" include, without limitation, curing, polymerizing, cross-linking, cooling, and drying.

The at least partial coating comprising the photochromic material may be connected to at least a portion of the substrate, for example, by applying a coating composition comprising the photochromic material to at least a portion of a surface of the substrate, and at least partially setting the coating composition. Additionally or alternatively, the at least partial coating comprising the photochromic material may be connected to the substrate, for example, through one or

more additional at least partial coatings. For example, while not limiting herein, according to various non-limiting embodiments, an additional coating composition may be applied to a portion of the surface of the substrate, at least partially set, and thereafter the coating composition comprising the photochromic material may be applied over the additional coating and at least partially set. Non-limiting methods of applying coating compositions to substrates are discussed herein below.

Non-limiting examples of additional coatings and films that may be used in conjunction with the photochromic articles disclosed herein include primer coatings and films; protective coatings and films, including transitional coatings and films and abrasion resistant coatings and films; anti-reflective coatings and films; conventional photochromic coating and films; and polarizing coatings and films; and combinations thereof. As used herein the term "protective coating or film" refers to coatings or films that can prevent wear or abrasion, provide a transition in properties from one coating or film to another, protect against the effects of polymerization reaction chemicals and/or protect against deterioration due to environmental conditions such as moisture, heat, ultraviolet light, oxygen, etc.

Non-limiting examples of primer coatings and films that may be used in conjunction with various non-limiting embodiments disclosed herein include coatings and films comprising coupling agents, at least partial hydrolysates of coupling agents, and mixtures thereof. As used herein "coupling agent" means a material having a group capable of reacting, binding and/or associating with a group on a surface. Coupling agents according to various non-limiting embodiments disclosed herein may include organometallics such as silanes, titanates, zirconates, aluminates, zirconium aluminates, hydrolysates thereof and mixtures thereof. As used herein the phrase "at least partial hydrolysates of coupling agents" means that some to all of the hydrolyzable groups on the coupling agent are hydrolyzed. Other non-limiting examples of primer coatings that are suitable for use in conjunction with the various non-limiting embodiments disclosed herein include those primer coatings described U.S. Patent 6,025,026 at col. 3, line 3 to col. 11, line 40 and U.S. Patent 6,150,430 at col. 2, line 39 to col. 7, line 58.

As used herein, the term "transitional coating and film" means a coating or film that aids in creating a gradient in properties between two coatings or films, or a coating and a film. For example, although not limiting herein, a transitional coating may aid in creating a gradient in hardness between a relatively hard coating and a relatively soft coating. Non-limiting examples of transitional coatings include radiation-cured, acrylate-based thin films as described in U.S. Patent Application Publication 2003/0165686 at paragraphs 79-173.

As used herein the term "abrasion resistant coating and film" refers to a protective polymeric material that demonstrates a resistance to abrasion that is greater than a standard reference material, e.g., a polymer made of CR-39® monomer available from PPG Industries, Inc, as tested in a method comparable to ASTM F-735 Standard Test Method for Abrasion Resistance of Transparent Plastics and Coatings Using the Oscillating Sand Method. Non-limiting examples of abrasion resistant coatings include abrasion-resistant coatings comprising organosilanes, organosiloxanes, abrasion-resistant coatings based on inorganic materials such as silica, titania and/or zirconia, organic abrasion-resistant coatings of the type that are ultraviolet light curable, oxygen barrier-coatings, UV-shielding coatings, and combinations thereof.

Non-limiting examples of antireflective coatings and films include a monolayer, multilayer or film of metal oxides, metal fluorides, or other such materials, which may be deposited onto the articles disclosed herein (or onto films that are applied to the articles), for example, through vacuum deposition, sputtering, etc. Non-limiting examples of conventional photochromic coatings and films include, but are not limited to, coatings and films comprising conventional photochromic materials. Non-limiting examples of polarizing coatings and films include, but are not limited to, coatings and films comprising dichroic compounds that are known in the art.

As discussed above, according to various non-limiting embodiments, an additional at least partial coating or film may be formed on the substrate prior to forming the coating comprising the photochromic material according to various non-limiting embodiments disclosed herein on the substrate. For example, according to certain non-limiting embodiments a primer coating may be formed on the substrate prior to applying the coating composition comprising the photochromic material. Additionally or alternatively, the additional at least partial coating or film may be formed on the substrate after forming coating comprising the photochromic material according to various non-limiting embodiments disclosed herein on the substrate, for example, as an overcoating. For example, according to certain non-limiting embodiments, a transitional coating may be formed over the coating comprising the photochromic material, and an abrasion resistant coating may be formed over the transitional coating.

Another non-limiting embodiments provides an optical element adapted for use behind a substrate that blocks a substantial portion of electromagnetic radiation in the range of 320 nm to 390 nm, the optical element comprising a photochromic material comprising an indeno-fused naphthopyran and a group that extends the pi-conjugated system of the indeno-fused naphthopyran bonded at the 11-position thereof connected to at least a portion of the optical element, wherein the at least a portion of the optical element absorbs a sufficient amount of electromagnetic radiation having a wavelength greater than 390 nm passing through the substrate that blocks a substantial portion of electromagnetic radiation in the range of 320 nm to 390 nm such that the at least a portion of the optical element transforms from a first state to a second state. For example, according to this non-limiting embodiment, the first state may be a bleached state

and the second state may be a colored state that corresponds to the colored state of the photochromic material(s) incorporated therein.

As previously discussed, many conventional photochromic materials require electromagnetic radiation having a wavelength ranging from 320 nm to 390 nm to cause the photochromic material to transformation from a closed-form to an open-form (e.g., from a bleached state to a colored state). Therefore, conventional photochromic materials may not achieve their fully-colored state when used in applications that are shielded from a substantial amount of electromagnetic radiation in the range of 320 nm to 390 nm. Further, as previous discussed, it has been observed by the inventors that photochromic material according to certain non-limiting embodiments disclosed herein may display both hyperchromic and bathochromic properties. That is, the indeno-fused naphthopyrans comprising a group that extends the pi-conjugated system of the indeno-fused naphthopyran at the 11-position thereof according to certain non-limiting embodiments disclosed herein may not only display hyperchromic absorption of electromagnetic radiation as discussed above, but may also have a closed-form absorption spectrum for electromagnetic radiation having a wavelength ranging from 320 nm to 420 nm that is bathochromically shifted as compared to a closed-form absorption spectrum for electromagnetic radiation having a wavelength ranging from 320 nm to 420 run of a comparable indeno-fused naphthopyran without the group that extends the pi-conjugated system of the comparable indeno-fused naphthopyran bonded at the 11-position thereof. Accordingly, the photochromic materials according to certain non-limiting embodiments disclosed herein may absorb a sufficient amount of electromagnetic radiation passing through a substrate that blocks a substantial portion of electromagnetic radiation having a wavelength ranging from 320 to 390 nm such that the photochromic material may transform from a closed-form to an open-form. That is, the amount of electromagnetic radiation having a wavelength of greater than 390 nm that is absorbed by the photochromic materials according to various non-limiting embodiments disclosed herein may be sufficient to permit the photochromic materials to transform from a closed-form to an open-form, thereby enabling their use behind a substrate that blocks a substantial portion of electromagnetic radiation having a wavelength ranging from 320 nm to 390 nm.

Non-limiting methods of making photochromic compositions and photochromic articles, such as optical elements, according to various non-limiting embodiments disclosed herein will now be discussed. One non-limiting embodiment provides a method of making a photochromic composition, the method comprising incorporating a photochromic material into at least a portion of an organic material. Non-limiting methods of incorporating photochromic materials into an organic material include, for example, mixing the photochromic material into a solution or melt of a polymeric, oligomeric, or monomeric material, and subsequently at least partially setting the polymeric, oligomeric, or monomeric material (with or without bonding the photochromic material to the organic material); and imbibing the photochromic material into the organic material (with or without bonding the photochromic material to the organic material).

Another non-limiting embodiment provides a method of making a photochromic article comprising connecting a photochromic material according to various non-limiting embodiments discussed above, to at least a portion a substrate. For example, if the substrate comprises a polymeric material, the photochromic material may be connected to at least a portion of the substrate by at least one of the cast-in-place method and by imbibition. For example, in the cast-in-place method, the photochromic material may be mixed with a polymeric solution or melt, or other oligomeric and/or monomeric solution or mixture, which is subsequently cast into a mold having a desired shape and at least partially set to form the substrate. Optionally, according to this non-limiting embodiment, the photochromic material may be bonded to a portion of the polymeric material of the substrate, for example, by copolymerization with a monomeric precursor thereof. In the imbibition method, the photochromic material may be diffuse into the polymeric material of the substrate after it is formed, for example, by immersing a substrate in a solution containing the photochromic material, with or without heating. Thereafter, although not required, the photochromic material may be bonded with the polymeric material.

Other non-limiting embodiments disclosed herein provide a method of making an optical element comprising connecting a photochromic material to at least a portion of a substrate by at least one of in-mold casting, coating and lamination. For example, according to one non-limiting embodiment, wherein the substrate comprises a polymeric material, the photochromic material may be connected to at least a portion of a substrate by in-mold casting. According to this non-limiting embodiment, a coating composition comprising the photochromic material, which may be a liquid coating composition or a powder coating composition, is applied to the surface of a mold and at least partially set. Thereafter, a polymer solution or melt, or oligomeric or monomeric solution or mixture is cast over the coating and at least partially set. After setting, the coated substrate is removed from the mold. Non-limiting examples of powder coatings in which the photochromic materials according to various non-limiting embodiments disclosed herein may be employed are set forth in U.S. Patent No. 6,068,797 at col. 7, line 50 to col. 19, line 42.

According to still another non-limiting embodiment, wherein the substrate comprises a polymeric material or an inorganic material such as glass, the photochromic material may be connected to at least a portion of a substrate by coating. Non-limiting examples of suitable coating methods include spin coating, spray coating (e.g., using a liquid or powder coating), curtain coating, roll coating, spin and spray coating, over-molding, and combinations thereof. For example, according to one non-limiting embodiment, the photochromic material may be connected to the substrate by over-molding. According to this non-limiting embodiment, a coating composition comprising the photochromic material

(which may be a liquid coating composition or a powder coating composition as previously discussed) may be applied to a mold and then the substrate may be placed into the mold such that the substrate contacts the coating causing it to spread over at least a portion of the surface of the substrate. Thereafter, the coating composition may be at least partially set and the coated substrate may be removed from the mold. Alternatively, over-molding may be done by placing the substrate into a mold such that an open region is defined between the substrate and the mold, and thereafter injecting a coating composition comprising the photochromic material into the open region. Thereafter, the coating composition may be at least partially set and the coated substrate may be removed from the mold.

Additionally or alternatively, a coating composition (with or without a photochromic material) may be applied to a substrate (for example, by any of the foregoing methods), the coating composition may be at least partially set, and thereafter, a photochromic material may be imbibed (as previously discussed) into the coating composition.

According to yet another non-limiting embodiment, wherein the substrate comprises a polymeric material or an inorganic material such as glass, the photochromic material may be connected to at least a portion of a substrate by lamination. According to this non-limiting embodiment, a film comprising the photochromic material may be adhered or otherwise connected to a portion of the substrate, with or without an adhesive and/or the application of heat and pressure. Thereafter, if desired, a second substrate may be applied over the first substrate and the two substrates may be laminated together (i.e., by the application of heat and pressure) to form an element wherein the film comprising the photochromic material is interposed between the two substrates. Methods of forming films comprising a photochromic material may include for example and without limitation, combining a photochromic material with a polymeric solution or oligomeric solution or mixture, casting or extruding a film therefrom, and, if required, at least partially setting the film. Additionally or alternatively, a film may be formed (with or without a photochromic material) and imbibed with the photochromic material (as discussed above).

Further, various non-limiting embodiments disclosed herein contemplate the use of various combinations of the forgoing methods to form photochromic articles according to various non-limiting embodiments disclosed herein. For example, and without limitation herein, according to one non-limiting embodiment, a photochromic material may be connected to substrate by incorporation into an organic material from which the substrate is formed (for example, using the cast-in-place method and/or imbibition), and thereafter a photochromic material (which may be the same or different from the aforementioned photochromic material) may be connected to a portion of the substrate using the in-mold casting, coating and/or lamination methods discussed above.

Further, it will be appreciated by those skilled in the art that the photochromic compositions and articles according to various non-limiting embodiments disclosed herein may further comprise other additives that aid in the processing and/or performance of the composition or article. Non-limiting examples of such additives include from photoinitiators, thermal initiators, polymerization inhibitors, solvents, light stabilizers (such as, but not limited to, ultraviolet light absorbers and light stabilizers, such as hindered amine light stabilizers (HALS)), heat stabilizers, mold release agents, rheology control agents, leveling agents (such as, but not limited to, surfactants), free radical scavengers, adhesion promoters (such as hexanediol diacrylate and coupling agents), and combinations and mixtures thereof.

According to various non-limiting embodiments, the photochromic materials described herein may be used in amounts (or ratios) such that the organic material or substrate into which the photochromic materials are incorporated or otherwise connected exhibits desired optical properties. For example, the amount and types of photochromic materials may be selected such that the organic material or substrate may be clear or colorless when the photochromic material is in the closed-form (i.e., in the bleached or unactivated state) and may exhibit a desired resultant color when the photochromic material is in the open-form (that is, when activated by actinic radiation). The precise amount of the photochromic material to be utilized in the various photochromic compositions and articles described herein is not critical provided that a sufficient amount is used to produce the desired effect. It should be appreciated that the particular amount of the photochromic material used may depend on a variety of factors, such as but not limited to, the absorption characteristics of the photochromic material, the color and intensity of the color desired upon activation, and the method used to incorporate or connect the photochromic material to the substrate. Although not limiting herein, according to various non-limiting embodiments disclosed herein, the amount of the photochromic material that is incorporated into an organic material may range from 0.01 to 40 weight percent based on the weight of the organic material.

Various non-limiting embodiments disclosed herein will now be illustrated in the following non-limiting examples.

## EXAMPLES

In Part 1 of the Examples, the synthesis procedures used to make photochromic materials according to various non-limiting embodiments disclosed herein are set forth in Examples 1-15, and the procedures used to make four comparative photochromic materials are described in Comparative Examples (CE) 1-4. In Part 2, the test procedures and results are described. In Part 3, the absorption properties of modeled photochromic materials are described.

PART 1: SYNTHESIS PROCEDURES

Example 1

Step 1

1,2-Dimethoxybenzene (31.4 g) and a solution of 4-bromobenzoyl chloride (50.0 g) in 500 mL of methylene chloride were added to a reaction flask fitted with a solid addition funnel under a nitrogen atmosphere. Solid anhydrous aluminum chloride (60.0 g) was added to the reaction mixture with occasionally cooling of the reaction mixture in an ice/ water bath. The reaction mixture was stirred at room temperature for 3 hours. The resulting mixture was poured into 300 mL of a 1:1 mixture of ice and 1N HCl and stirred vigorously for 15 minutes. The mixture was extracted twice with 100 mL methylene chloride. The organic extracts were combined and washed with 50 mL of 10 wt % NaOH followed by 50 mL of water. The methylene chloride solvent was removed by rotary evaporation to give 75.0 g of a yellow solid. Nuclear magnetic resonance ("NMR") spectra showed the product to have a structure consistent with 3,4-dimethoxy 4'-bromobenzophenone.

Step 2

Potassium t-butoxide (30.1 g) and 70.0 g of 3,4-dimethoxy 4'-bromobenzophenone from Step 1 were added to a reaction flask containing 500 mL of toluene under a nitrogen atmosphere. The mixture was heated to reflux and dimethyl succinate (63.7 g) was added dropwise over 1 hour. The mixture was refluxed for 5 hours and cooled to room temperature. The resulting mixture was poured into 300 mL of water and vigorously stirred for 20 minutes. The aqueous and organic phases were separated and the organic phase was extracted with 100 mL portions of water three times. The combined aqueous layers were washed with 150 mL portions of chloroform three times. The aqueous layer was acidified to pH 2 with 6N HCl and a precipitate formed. The aqueous layer was extracted with three 100 mL portions of chloroform. The organic extracts were combined and concentrated by rotary evaporation. NMR spectra of the resulting oil showed the product to have structures consistent with a mixture of (E and Z) 4-(3,4-dimthoxyphenyl)-4-(4-bromophenyl)-3-methoxycarbonyl-3-butenoic acids.

Step 3

The crude half-esters from Step 2 (100.0 g), 60 mL of acetic anhydride, and 300 mL of toluene were added to a reaction flask under a nitrogen atmosphere. The reaction mixture was heated to 110° C for 6 hours, cooled to room temperature, and the solvents (toluene and acetic anhydride) removed by rotary evaporation. The residue was dissolved in 300 mL of methylene chloride and 200 mL of water. Solid $Na_2CO_3$ was added to the biphasic mixture until bubbling ceased. The layers separated and the aqueous layer was extracted with 50 mL portions of methylene chloride. The organic extracts were combined and the solvent was removed by rotary evaporation to yield a thick red oil. The oil was dissolved in warm methanol and chilled at 0°C for 2 hours. The resulting crystals were collected by vacuum filtration, washed with cold methanol to produce the mixtures of 1-(4-bromophenyl)-2-methoxycarbonyl-4-acetoxy-6,7-dimethoxynaphthalene and 1-(3,4-dimethoxyphenyl-2-methoxycarbonyl-4-acetoxy-6-bromonaphthalene. The product mixture was used without further purification in subsequent reaction.

Step 4

The mixture (50.0 g) from Step 3 was weighed into a reaction flask under a nitrogen atmosphere and 300 mL of anhydrous THF was added. Methyl magnesium chloride (200 mL of 3.0M in THF) was added to the reaction mixture over 1 hour. The reaction mixture was stirred overnight and then poured into 300 mL of a 1:1 mixture of ice and 1N HCl. The mixture was extracted with chloroform (three times with 300 mL). The organic extracts were combined, washed with saturated aqueous NaCl solution (400 mL) and dried over anhydrous $Na_2SO_4$. Removal of the solvent by rotary evaporation yielded 40.0 g of 1-(4-bromophenyl)-2-(dimethylhydroxymethyl)-4-hydroxy-6,7-dimethoxynaphthalene and 1-(3,4-dimethoxyphenyl-2-(dimethylhydroxymethyl)-4-hydroxy-6-bromonaphthalene.

Step 5

The products from Step 4 (30.0 g) were placed in a reaction flask equipped with a Dean-Stark trap and 150 mL of toluene was added. The reaction mixture was stirred under a nitrogen atmosphere and dodecylbenzene sulfonic acid (about 0.5 mL) was added. The reaction mixture was heated at reflux for 2 hours and cooled to room temperature. Upon cooling the mixture to room temperature for 24 hours, the white solid was precipitated. NMR spectra showed the

product to have a structure consistent with 2,3-dimethoxy-7,7-dimethyl-9-bromo-7H-benzo[C]fluoren-5-ol. This material was not purified further but was used directly in the next step.

Step 6

The product from Step 5 (10.0 g) was placed in a reaction flask under a nitrogen atmosphere and 100 mL of anhydrous 1-methyl-2-pyrrolidinone was added. CuCN (4.5 g) was added to the reaction mixture. The reaction mixture was heated at reflux for 4 hours and cooled to room temperature. To the resulting mixture was added 100 mL of 6N HCl and the mixture was stirred for 10 minutes. The mixture was washed with 150 mL portions of ethyl acetate three times. The organic extracts were combined and the solvent was removed by rotary evaporation to give 7.2 g of a gray solid. NMR spectra showed the product to have a structure consistent with 2,3-dimethoxy-7,7-dimethyl-9-cyano-7H-benzo[C]fluoren-5-ol.

Step 7

2,3-Dimethoxy-7,7-dimethyl-9-cyano-7H-benzo[C]fluoren-5-ol from Step 6 (10 g), 1,1-bis(4-methoxyphenyl)-2-propyn-1-ol (8.0 g, the product of Example 1, Step 1 of U.S. Patent No. 5,458,814 ), dodecylbenzene sulfonic acid (0.5 g) and chloroform (preserved with pentene, 250 mL) were combined in a reaction flask and stirred at room temperature for 5 hours. The reaction mixture was washed with 50 % saturated aqueous $NaHCO_3$ (200 mL) and the organic layer was dried over anhydrous $Na_2SO_4$. The solvent was removed by rotary evaporation. Hot methanol was added to the resulting residue and the solution cooled to room temperature. The resulting precipitate was collected by vacuum filtration and washed with cold methanol yielding 14.0 g of 3,3-di(4-methoxyphenyl)-6,7-dimethoxy-11-cyano-13,13-dimethyl-3H,13H-indeno[2',3':3,4]naphtho[1,2-b]pyran, (i.e., an indeno-fused naphtho[1,2-b]pyran with a cyano group that extends the pi-conjugated system of the indeno-fused naphthopyran bonded at the 11-position thereof). The product was used without further purification in the subsequent reaction.

Example 2:

Step 1

2,3-Dimethoxy-7,7-dimethyl-9-cyano-7H-benzo[C]fluoren-5-ol from Step 6 of Example 1 (10.0 g) was placed in a flask under a nitrogen atmosphere and NaOH (20 g) was added. To the mixture, ethanol (100mL) and water (100 mL) were added. The reaction mixture was heated at reflux for 24 hours and cooled to room temperature. The resulting mixture was poured into 200 mL of a 1:1 mixture of ice and 6N HCl and stirred vigorously for 15 minutes. The mixture was washed with 150 mL portions of ethyl acetate three times. The organic extracts were combined and the solvent was removed by rotary evaporation to give 9.0 g of a white solid. NMR spectra showed the product to have a structure consistent with 2,3-dimethoxy-7,7-dimethyl-9-carboxy-7H-benzo[C]fluoren-5-ol.

Step 2

The procedure of Step 7 of Example 1 was followed except that 2,3-dimethoxy-7,7-dimethyl-9-carboxy-7H-benzo[C]fluoren-5-ol of Step I was used in place of 2,3-dimethoxy-7,7-dimethyl-9-cyano-7H-benzo[C]fluoren-5-ol to produce 3,3-di(4-methoxyphenyl)-6,7-dimethoxy-11-carboxy-13,13-dimethyl-3H,13H-indeno[2',3':3,4]naphtho[1,2-b]pyran.

Example 3:

Step 1

2,3-Dimethoxy-7,7-dimethyl-9-carboxy-7H-benzo[C]fluoren-5-ol from Step 1 of Example 2 (5.0 g), 1.0 mL of aqueous HCl, and 100 mL of methanol were combined in a flask and heated at reflux for 24 hours. The reaction mixture was cooled and the resulting precipitate was collected by vacuum filtration and washed with cold methanol yielding 4.9 g of a white solid. NMR spectra showed the product to have a structure consistent with 2,3-dimethoxy-7,7-dimethyl-9-methoxycarbonyl-7H-benzo[C]fluoren-5-ol.

Step 2

The procedure of Step 7 of Example 1 was followed except that 2,3-dimethoxy-7,7-dimethyl-9-methoxycarbonyl-7H-benzo[C]fluoren-5-ol of Step 1 was used in place of 2,3-dimethoxy-7,7-dimethyl-9-cyano-7H-benzo[C]fluoren-5-ol to produce 3,3-di(4-methoxyphenyl)-6,7-dimethoxy-11-methoxycarbonyl-13,13-dimethyl-3H,13H-indeno[2',3':3,4] naphtho[1,2-b]pyran.

Example 4:

3,3-Di(4-methoxyphenyl)-6,7-dimethoxy-11-carboxy-13,13-dimethyl-3H,13H-indeno[2',3':3,4]naphtho[1,2-b] pyran from Step 2 of Example 2 (1.8 g), diethylene glycol (0.2 g), dicyclohexyl carbodiimide (1.2 g), 4-(dimethylamino)-pyridine (0.01 g) and dichloromethane (10 mL) were added to a flask and heated under reflux for 24 hours. The solid produced was removed by filtration and the remaining solvent was removed by rotary evaporation. Ether was added to the resulting residue and the solution cooled to room temperature. The precipitate obtained was collected by vacuum filtration and washed with diethyl ether yielding 2.1 g of 3,3-di(4-methoxyphenyl)-6,7-dimethoxy-11-(2-(2-hydroxyethoxy) ethoxycarbonyl)-13,13-dimethyl-3H,13H-indeno[2',3' :3,4]naphtho[1,2-b]pyran.

Example 5:

Step 1

2,3-Dimethoxy-7,7-dimetllyl-9-bromo-benzo[C]fluoren-5-ol from Step 5 of Example 1 (1.4 g), tetrakis(triphenylphosphine)palladium (0.12 g), 4-fluorophenylboronic acid (0.6 g), sodium carbonate (1.06 g), ethylene glycol dimethyl ether (50 mL), and water (50 mL) were combined in a reaction flask under a nitrogen atmosphere and stirred for 1 hour at room temperature. The mixture was then heated at reflux for 24 hours. After this time, the mixture was filtered and extracted with ethyl acetate (three times with 300 mL). The organic extracts were combined and the solvent was removed by rotary evaporation to give 1.2 g of a white solid. NMR spectra showed the product to have a structure consistent with 2,3-dimethoxy-7,7-dimethyl-9-(4-fluorophenyl)-7H-benzo[C]fluoren-5-ol.

Step 2

The procedure of Step 7 of Example 1 was followed except that 2,3-dimethoxy-7,7-dimethyl-9-(4-fluorophenyl)-7H-benzo[C]fluoren-5-ol of Step 1 was used in place of 2,3-dimethoxy-5-hydroxy-7,7-dimethyl-9-cyano-7H-benzo[C] fluoren-5-ol to produce 3,3-di(4-methoxyphenyl)-6,7-dimethoxy-11-(4-fluorophenyl)-13,13-dimethyl-3H,13H-indeno[2', 3':3,4]naphtho[1,2-b]pyran.

Example 6:

Step 1

The procedure of Step 1 of Example 5 was followed except that 4-phenylphenylboronic acid was used in place of 4-fluorophenylboronic acid to produce 2,3-dimethoxy-7,7-dimethyl-9-(4-(phenyl)phenyl)-7H-benzo[C]fluoren-5-ol.

Step2

The procedure of Step 7 of Example 1 was followed except that 2,3-dimethoxy-7,7-dimethyl-9-(4-(phenyl) phenyl)-7H-benzo[C]fluoren-5-ol of Step 1 was used in place of 2,3-dimethoxy-7,7-dimethyl-9-cyano-7H-benzo[C]fluoren-5-ol to produce 3,3-di(4-methoxyphenyl)-6,7-dimethoxy-11-(4-(phenyl)phenyl)-13,13-dimethyl-3H,13H-indeno[2', 3':3,4]naphtho[1,2-b]pyran.

Example 7:

Step 1

The procedure of Step 1 of Example 5 was followed except that 4-(hydroxymethyl) phenylboronic acid was used in place of 4-fluorophenylboronic acid to produce 2,3-dimethoxy-7,7-dimethyl-9-(4-(hydroxymethyl)phenyl)-7H-benzo[C]fluoren-5-ol.

Step 2

The procedure of Step 7 of Example 1 was followed except that 2,3-dimethoxy 7,7-dimethyl-9-(4-(hydroxymethyl)phenyl)-7H-benzo[C]fluoren-5-ol of Step 1 was used in place of 2,3-dimethoxy-7,7-d.imethyl-9-cyano-7H-benzo[C]fluoren-5-ol to produce 3,3-di(4-methoxyphenyl)-6,7-dimethoxy-11-(4-(hydroxymethyl)phenyl)-13,13-dimethyl-3H,13H-indeno[2',3':3,4]naphtho[1,2-b]pyran.

Example 8:

Step 1

2,3-Dimethoxy-7,7-dimethyl-9-bromo-7H-benzo[C]fluoren-5-ol from Step 5 of Example 1 (5.0 g), triphenylphosphine (0.16 g), dichlorobis(triphenylphosphine) palladium (0.12 g), copper iodide (0.06 g), 2-methyl-3-butyn-2-ol (1.56 g) and diisopropylamine (30 mL) were combined in a reaction flask under a nitrogen atmosphere and stirred for 1 hour at room temperature. The mixture was then heated at 80˚C for 24 hours. After this time, the solid was filtered off over a short pad of silica gel and the solution was concentrated under vacuum. NMR spectra confirmed the resulting white solid to have the structure 2,3-dimethoxy-7,7-dimethyl-9-(3-hydroxy-3-methylbutyn)-7H-benzo[C]fluoren-5-ol.

Step 2

The procedure of Step 7 of Example 1 was followed except that 2,3-dimethoxy-7,7-dimethyl-9-(3-hydroxy-3-methylbutyn)-7H-benzo[C]fluoren-5-ol of Step 1 was used in place of 2,3-dimethoxy-7,7-dimethyl-9-cyano-7H-benzo[C]fluoren-5-ol to produce 3,3-di(4-methoxyphenyl)-6,7-dimethoxy-11-(3-hydroxy-3-methylbutyn)-13-dimethyl-3H,13H-indeno[2',3':3,4]naphtho[1,2-b]pyran.

Example 9:

Step 1

The procedure of Step 1 of Example 8 was followed except that phenylacetylene was used in place of 2-methyl-3-butyn-2-ol to produce 2,3-dimethoxy-7,7-dimethyl-9-(2-phenylethynyl)-7H-benzo[C]fluoren-5-ol.

Step 2

The procedure of Step 7 of Example 1 was followed except that 2,3-dimethoxy-7,7-dimethyl-9-(2-phenylethynyl)-7H-benzo[C]fluoren-5-ol of Step 1 was used in place of 2,3-dimethoxy-7,7-dimethyl-9-cyano-7H-benzo[C]fluoren-5-ol to produce 3,3-di(4-methoxyphenyl)-6,7-dimethoxy-11-(2-phenylethynyl)-13,13-dimethyl-3H, 13H-indeno[2',3':3,4]naphtho[1,2-b]pyran.

Example 10:

Step 1

4-Biphenylcarbonyl chloride (150 g), 1,2-dimethoxybenzene (88 mL), and dichloromethane (1.4 L) were combined in a reaction flask under a nitrogen atmosphere. The reaction flask was cooled in an ice bath and aluminum chloride anhydrous (92.3 g) was added slowly over 30 minutes using a solid addition funnel. The ice bath was removed and the reaction mixture allowed to warm to room temperature. Additional 1,2-dimethoxybenzene (40 mL) and aluminum chloride (30 grams) were added to the reaction flask. After 1.5 hours the reaction mixture was slowly poured into a mixture of saturated aqueous $NH_4Cl$ and ice (1.5 L). The layers were separated and the aqueous layer was extracted with two 750 mL portions of dichloromethane. The organic portions were combined and washed with 50 % saturated aqueous solution of $NaHCO_3$ (1 L). The organic layer was dried over anhydrous magnesium sulfate and concentrated by rotary evaporation. The resulting residue was dissolved in hot t-butyl methyl ether and allowed to cool to room temperature slowly. A white solid precipitated and was collected by vacuum filtration, washing with cold t-butyl methyl ether yielding 208 g of 3,4-dimethoxy-4'-phenylbenzophenone.

Step 2

3,4-Dimethoxy-4'-phenylbenzophenone from Step 1 (200 g), potassium *tert*-butoxide (141 g), and toluene (3 L) were combined in a flask under a nitrogen atmosphere and heating begun. To this was added dimethyl succinate (144 mL) dropwise over 45 minutes. Reaction mixture was heated to 70 ˚C for 1.5 hours and then cooled to room temperature. The reaction mixture was poured into a mixture of saturated aqueous NaCl and ice (3 L). The layers were

separated and the aqueous layer was extracted with two 1L portions of diethyl ether. The organic layers were discarded and the aqueous layer was acidified to pH 1 with conc. HCl. Dichloromethane (2 L) was added, the mixture extracted and the layers separated. The aqueous layer was extracted with two I L portions of dichloromethane. The organic layers were combined and washed with water (2 L). The organic layer was dried over anhydrous magnesium sulfate and concentrated by rotary evaporation to an orange colored oil yielding 287 g of a mixture of (E and Z) 3-methoxycarbonyl-4-(4-phenyl)phenyl-4-(3,4-dimethoxyphenyl)-3-butenoic acid. The product was used without further purification in the subsequent reaction.

Step 3

A mixture of (E and Z) 3-methoxycarbonyl-4-(4-phenyl)phenyl-4-(3,4-dimethoxyphenyl)-3-butenoic acid from Step 2 (272 g) and acetic anhydride (815 mL) were combined in a reaction flask under a nitrogen atmosphere and heated to reflux for 13 hours. The reaction mixture was cooled to room temperature and then slowly poured into ice water (1 L). The mixture was stirred for 3 hours and then saturated aqueous $NaHCO_3$ (2 L) was slowly added. Additional sodium bicarbonate (750 grams) was slowly added portionwise. Dichloromethane (2.5 L) was added to the mixture, which was then filtered, and the filtrate phase separated. The aqueous layer was extracted with dichloromethane (1 L). The organic layers were combined, dried over anhydrous magnesium sulfate, and concentrated by rotary evaporation to a dark red solid. The red solid was slurried in hot ethanol, cooled to room temperature, collected by vacuum filtration, and washed with cold ethanol yielding 187.5 g of a mixture of 1-(4-phenyl)phenyl-2-methoxycarbonyl-4-acetoxy-6,7-dimethoxynaphthalene and 1-(3,4-dimethoxyphenyl)-2-methoxycarbonyl-4-acetoxy-6-phenylnaphthalene. The product was used without further purification in the subsequent reaction.

Step 4

The mixture of 1-(4-phenyl)phenyl-2-methoxycarbonyl-4-acetoxy-6,7-dimethoxynaphthalene and 1-(3,4-dimethoxyphenyl)-2-methoxycarbonyl-4-acetoxy-6-phenylnaphthalene from Step 3 (172 g), water (1035 mL), methanol (225 mL), and sodium hydroxide (258 g) were combined in a reaction flask and heated to reflux for 5 hours. The reaction mixture was cooled to room temperature and was then slowly poured into a mixture of water (1.5 L), conc. HCl (500 mL) and ice. A white solid precipitated and was filtered and washed with water. The solid was dissolved in a small amount of anhydrous tetrahydrofuran and then diluted with t-butyl methyl ether. This solution was washed with saturated aqueous NaCl and the organic layer was dried over anhydrous magnesium sulfate and concentrated by rotary evaporation to a light orange solid. The solid was slurried in hot toluene, cooled to room temperature, filtered, and washed with cold toluene yielding 127 g of a white solid (1-(4-phenyl)phenyl-2-carboxy-4-hydroxy-6,7-dimethoxynaphthalene). The product was used in the subsequent reaction without purification.

Step 5

1-(4-Phenyl)phenyl-2-carboxy 4-hydroxy-6,7-dimethoxynaphthalene from Step 4 (25 g), acetic anhydride (29 mL), 4-(dimethylamino)pyridine (115 mg), and 1,2,4-trimethylbenzene (500 mL) were combined in a reaction flask under a nitrogen atmosphere and heated to 50 ˚C for one hour. Dodecylbenzene sulfonic acid (10.3 g) was added to the reaction mixture and the temperature increased to 144 ˚C. After 28 hours the reaction mixture was slowly cooled to room temperature and a solid precipitated. The reaction mixture was filtered and washed with toluene yielding 23.0 g of a red solid (2,3-dimethoxy-5-acetoxy-11-phenyl-7H-benzo[C]fluoren-7-one). The product was used in the subsequent reaction without further purification.

Step 6

2,3-Dimethoxy-5-acetoxy-11-phenyl-7H-benzo[C]fluoren-7-one from Step 5 (4.22 g) and anhydrous tetrahydrofuran (85 mL) were combined in a reaction flask under a nitrogen atmosphere and cooled in an ice bath. To this was added 13.5 mL of an ethylmagnesium bromide solution (3.0 M in diethyl ether) dropwise over 20 minutes. The reaction mixture was allowed to warm to room temperature and was then poured into a mixture of saturated aqueous $NH_4Cl$ and ice (100 mL). The mixture was diluted with ethyl acetate (40 mL) and then the layers were separated. The aqueous layer was extracted with two 70 mL portions of ethyl acetate. The organic layers were combined and washed with saturated aqueous $NaHCO_3$ (100 mL), dried over $NaSO_4$, and concentrated by rotary evaporation to afford an orange solid. The solid was slurried in hot *t*-butyl methyl ether, cooled to room temperature, filtered, and washed with cold *t*-butyl methyl ether yielding 2.6 g of a light orange solid (2,3-dimethoxy-7-hydroxy-7-ethyl-11-phenyl-7H-benzo[C]fluoren-5-ol). The product was used in the subsequent reaction without further purification.

Step 7

2,3-Dimethoxy-7-hydroxy-7-ethyl-11-phenyl-7H-benzo[C]fluoren-5-ol from Step 6 (2.59 g), 1,1-bis(4-methoxyphenyl)-2-propyn-1-ol (2.19 g, the product of Example 1, Step 1 of U.S. Patent No. 5,458,814), and dichloromethane (52 mL) were combined in a reaction flask under a nitrogen atmosphere. To this was added trifluoroacetic acid (41 mg). After 2 hours *p*-toluenesulfonic acid monohydrate (29 mg) was added to the reaction flask. After an additional 45 minutes the reaction mixture was diluted with dichloromethane (25 mL) and then washed with 50% saturated aqueous $NaHCO_3$ (50 mL). The organic layer was dried over anhydrous magnesium sulfate and concentrated by rotary evaporation. Hot acetonitrile was added to the resulting residue and a solid precipitated. The mixture was cooled to room temperature, vacuum filtered, and washed with cold acetonitrile yielding 3.43 g of a light green solid (3,3-di(4-methoxyphenyl)-6,7-dimethoxy-11-phenyl-13-ethyl-13-hydroxy-3H,13H-indeno[2',3':3,4]naphtho[1,2-b]pyran). The product was used in the subsequent reaction without further purification.

Step 8

3,3-Di(4-methoxyphenyl)-6,7-dimethoxy-11-phenyl-13-ethyl-13-hydroxy-3H,13H-indeno[2',3':3,4]naphtho[1,2-b]pyran from Step 7 (3.4 g), anhydrous methanol (35 mL), toluene (34 mL), and p-toluenesulfonic acid monohydrate (75 mg) were combined in a reaction flask under a nitrogen atmosphere and heated to reflux. After 4 hours the reaction mixture was cooled to room temperature and diluted with toluene (35 mL). The reaction mixture was washed with two 35 mL portions of 50% saturated aqueous $NaHCO_3$. The organic layer was dried over anhydrous magnesium sulfate and concentrated by rotary evaporation. Hot methanol was added to the resulting residue and a solid precipitated. The mixture was cooled to room temperature, vacuum filtered, and the solid washed with cold methanol yielding 3.06 g of a light yellow solid. Mass spectrometry ("MS") analysis and NMR spectra show the product to have a structure consistent with 3,3-di(4-methoxyphenyl)-6,7-dimethoxy-11-phenyl-13-ethyl-13-methoxy-3H,13H-indeno[2',3':3,4]naphtho[1,2-b] Pyran.

Example 11:

Step 1

2,3-Dimethoxy-7,7-dimethyl-9-bromo-7H-benzo[C]fluoren-5-ol from Step 5 of Example 1 (5 g), tetrakis(triphenylphosphine)palladium (0.43 g), 4-methoxycarbonyl phenylboronic acid (2.5 g), sodium carbonate (3 g), ethylene glycol dimethyl ether (90 mL), and water (30 mL) were combined in a reaction flask under nitrogen atmosphere and stirred for 1 hour at room temperature. The mixture was then heated at reflux for 24 hours. Water (60 mL) and sodium hydroxide (1 g) were added, and the reaction mixture was heated at reflux for 20 hours. After this time, the mixture was cooled to room temperature, and aqueous HCl (10%) was added to the mixture under stirring, the mixture was filtered and extracted with ethyl acetate (three times with 100 mL) and dichloromethane (three times with 100 mL). The organic extracts were combined and the solvent was removed by rotary evaporation to give 5 g of a yellow solid (2,3-dimethoxy-7,7-dimethyl-9-(4-hydroxycarbonylphenyl)-7H-benzo[C]fluoren-5-ol). The product was used without further purification in the subsequent reaction.

Step 2

2,3-Dimethoxy-7,7-dimethyl-9-(4-hydroxycarbonylphenyl)-7H-benzo[C]fluoren-5-ol from Step 1 (7.5 g), 1-phenyl-1-(4-methoxyphenyl)-2-propyn-1-ol (4.0 g, made as described in Example 1, Step 1 of U.S. Patent No. 5,458,814), dodecylbenzene sulfonic acid (0.2 g) and chloroform (preserved with pentene, 70 mL) were combined in a reaction flask and stirred at room temperature for 2 hours. The reaction mixture was concentrated, and acetone (100 mL) was added to the residue, and the slurry was filtered, yielding 6.5 g of a green solid. The product was used without further purification in the subsequent reaction.

Step 3

3-Phenyl-3-(4-methoxyphenyl)-6,7-dimethoxy-11-(4-hydroxycarbonylphenyl)-13,13-dimethyl-3H,13H-indeno[2',3':3,4]naphtho[1,2-b]pyran from Step 2 (0.2 g), 2-hydroxyethyl methacrylate (0.5 mL), dicyclohexyl carbodiimide (0.2 g), 4-(dimethylamino)-pyridine (0.04 g) and dimethylformamide (20 mL) were added to a flask and heated to 55-58°C for 3 hours. Water was added to the reaction mixture, the precipitation was filtered out, yielding 0.27 g of an off-green solid. MS analysis supports the molecular weight of 3-phenyl-3-(4-methoxyphenyl)-6,7-dimethoxy-11-(4-(2-methacryloxyethoxy)carbonylphenyl)-13,13-dimethyl-3H,13H-indeno[2',3':3,4]naphtho[1,2-b]pyran.

Example 12:

Step 1

2,3-Dimethoxy-7,7-dimethyl-9-bromo-7H-benzo[C]fluoren-5-ol from Step 5 of Example 1 (4.7 g), 1,1-bis(4-methoxyphenyl)-2-propyn-1-ol (3.5g, the product of Example 1, Step 1 of U.S. Patent No. 5,458,814), pyridinium p-toluenesulfonate (0.15 g), trimethyl orthoformate (3.5 mL) and chloroform (preserved with pentene, 100 mL) were combined in a reaction flask and stirred at reflux for half hour. The reaction mixture was concentrated. Acetone was added to the residue, the slurry was filtered, yielding 7.7 g of an off white solid, MS analysis supports the molecular weight of 3,3-di(4-methoxyphenyl)-6,7-dimethoxy-11-bromo-13,13-dimethyl-3H,13H-indeno[2',3':3,4]naphtho[1,2-b]pyran. The product was used without further purification in the subsequent reaction.

Step2

The procedure of Step 1 of Example 5 was followed except that 4-phenylphenylboronic acid was used in place of 4-fluorophenylboronic acid to produce 3,3-di(4-methoxyphenyl)-6,7-dimethoxy-11-(4-phenylphenyl)-13,13-dimethyl-3H,13H-indeno[2',3':3,4]naphtho[1,2-b]pyran. The product was used without further purification in the subsequent reaction.

Step 3

3,3-Di(4-methoxyphenyl)-6,7-dimethoxy-11-(4-(phenyl)phenyl)-13,13-dimethyl-3H,13H-indeno[2',3':3,4]naphtho[1,2-b]pyran from Step 2 (above) (6 g), 3-piperidinemethanol (1.3 g) and tetrahydrofuran (60 mL) were combined in a dry reaction flask under nitrogen atmosphere, butyl lithium (10 mL, 2.5 M in hexane) was cannulated into the reaction flask under stirring. The mixture was stirred for 30 minutes at room temperature and then carefully poured into ice water. The mixture was extracted with ethyl acetate (three times with 100 mL). The extracts were combined and washed with saturated aqueous sodium chloride solution. The solution was dried over $Na_2SO_4$ and filtered. The solution was concentrated and the residue was purified by silica gel chromatography (ethyl acetate/hexanes (v/v): 1/1). The major fraction was collected from column and concentrated, yielding 5 g of purple foam. MS analysis supports the molecular weight of 3,3-di(4-methoxyphenyl)-6-methoxy-7-((3-hydroxymethylenepiperidino)-1-yl)-11-(4-phenyl)phenyl))-13,13-dimethyl-3H,13H-indeno[2',3':3,4]naphtho[1,2-b]pyran. The product was used without further purification in the subsequent reaction.

Step 4

3,3-Di(4-methoxyphenyl)-6-methoxy-7-((3-hydroxymethylenepiperidino)-1-yl)-11-(4-phenyl)phenyl)-13,13-dimethyl-3H,13H-indeno[2',3':3,4]naphtho[1,2-b]pyran from Step 3 (5 g), 2-isocyanatoethylmethacrylate (1 mL), dibutyltin dilaurate (1 drop) and ethyl acetate (50 mL) were combined in a reaction flask with a condenser open to air. The mixture was heated at reflux for 20 minutes. Methanol (5 mL) was added to the mixture to quench excess 2-isocyanatoethylmethacrylate. The reaction mixture was concentrated and the residue was purified by silica gel chromatography (ethyl acetate/hexanes (v/v): 1/1). The major fraction was collected from the column and concentrated, yielding 6 g of a purple foam. MS analysis supports the molecular weight of 3,3-di(4-methoxyphenyl)-6-methoxy-7-((3-(2-methacryloxyethyl)carbamyloxymethylene piperidino)-1-yl)-11-(4-(phenyl)phenyl)-13,13-dimethyl-3H,13H-indeno [2',3':3,4] naphtho[1,2-b]pyran.

Example 13:

Step 1

The procedures of Example 1 were followed except that 4-bromo-4'-methoxybenzophenone was used in place of 3,4-dimethoxy-4'-bromobenzophenone to produce 3-methoxy-9-bromo-7,7-dimethyl-7H-benzo[C]fluoren-5-ol.

Step2

4-Hydroxybenzophenone (100 g), 2-chloroethanol (50 g), sodium hydroxide (20 g) and water (500 mL) were combined in a reaction flask. The mixture was heated at reflux for 6 hours. The oily layer was separated and crystallized upon cooling, the crystalline material was washed with aqueous sodium hydroxide followed by fresh water and dried, yielding an off-white solid 85 g. The product was used without further purification in the subsequent reaction.

Step 3

The product from Step 2 (30 g) was dissolved in anhydrous dimethylformamide (250 mL) in a reaction flask with overhead stirring. Sodium acetylide paste in toluene (15 g, ~9wt%) was added to the reaction flask under vigorous stirring. After the reaction is complete, the mixture was added to water (500 mL), and the solution was extracted with ethyl ether (twice with 500 mL). The extracts were combined and washed with saturated aqueous sodium chloride solution and dried over sodium sulfate. The solution was then filtered and concentrated, and the dark residue was purified by silica gel chromatography (ethyl acetate/hexanes (v/v): 1/1). The major fraction was collected from column and concentrated, yielding 33 g of a white solid (1-phenyl-1-(4-(2-hydroxyethoxy)phenyl)-2-propyn-1-ol).

Step 4

3-Methoxy- 9-bromo-7,7-dimethyl-7H-benzo[C]fluoren-5-ol from Step 1 (5 g), 1-phenyl-1-(4-(2-hydroxyethoxy) phenyl)-2-propyn-1-ol from Step 3 (4 g), dodecylbenzene sulfonic acid (2 drops) and chloroform (40 mL) were combined in a reaction flask. The mixture was heated at reflux for an hour and then concentrated. The residue was purified by silica gel chromatography (ethyl acetate/hexanes (v/v): 1/1). The major fraction was collected from the column and concentrated to 7 g of an expanded green foam. MS analysis supports the molecular weight of 3-phenyl-3-(2-hydroxyethoxy)phenyl-6-methoxy-11-bromo-13,13-dimethyl-3H,13H-indeno[2',3':3,4]naphtho[1,2-b]pyran.

Step 5

3-Phenyl-3-(4-(2-hydroxyethoxy)phenyl)-6-methoxy-11-bromo-13,13-dimethyl-3H,13H-indeno[2',3':3,4]naphtho[1,2-b]pyran from Step 4 (3.5 g), tetrakis(triphenylphosphine)palladium (0.12 g), phenylboronic acid (1.05 g), sodium carbonate (1.33 g), ethylene glycol dimethyl ether (50 mL), and water (10 mL) were combined in a reaction flask under nitrogen atmosphere and stirred for 1 hour at room temperature. The mixture was then heated at reflux for 28 hours. After this time, water (30 mL) was added to the mixture. The mixture was extracted with ethyl acetate (200 mL), the extract was washed with water and saturated aqueous sodium chloride solution and dried over sodium sulfate. The solution was filtered and concentrated. The residue was purified by silica gel chromatography (ethyl acetate/hexanes (v/v): 1/1.5). The major fraction was recrystallized in ethyl acetate/hexanes (v/v: 1/2), yielding 1.6 g of a yellow-green solid. NMR spectra supports the structure of 3-phenyl-3-(4-(2-hydroxyethoxy)phenyl)-6-methoxy-11-phenyl-13,13-dimethyl-3H,13H-indeno [2',3':3,4]naphtho[1,2-b]pyran.

Step 6

3- Phenyl- 3-(4-(2- hydroxyethoxy) phenyl)- 6- methoxy- 11- phenyl- 13,13- dimethyl- 3H, 13H- indeno [2', 3': 3,4] naphtho[1,2-b]pyran from Step 5 (1 g), 2-isocyanatoethylmethacrylate (0.8 mL), dibutyltin dilaurate (1 drop) and ethyl acetate (20 mL) were combined in a reaction flask with a condenser open to air. The mixture was heated at reflux for 1 hour. Methanol (4 mL) was added to the mixture to quench excess 2-isocyanatoethylmethacrylate. The reaction mixture was concentrated and the residue was purified by silica gel chromatography (dichloromethane/hexanes/acetone (v/v/v): 10/5/1).
The major fraction was collected from column and concentrated to an expanded blue-green foam. MS analysis supports the molecular weight of 3-phenyl-3-(4-(2-(2-methacryloxyethyl)carbamyloxyethoxy)phenyl)-6-methoxy-11-phenyl-13,13-dimethyl-3H,13H-indeno[2',3':3,4]naphtho[1,2-b]pyran.

Example 14:

Step 1

The procedures of Example 1 were followed except that 4,4'-dimethoxybenzophenone was used in place of 3,4-dimethoxy-4'-bromobenzophenone to produce 3,9-dimethoxy-7,7-dimethyl-7H-benzo[C]-fluoren-5-ol.

Step 2

3,9-Dimethoxy-7,7-dimethyl-7H-benzo[C]fluoren-5-ol from Step 1 (3 g), the product of Example 13 Step 3 (1-phenyl-1-(4-(2-hydroxyethoxy)phenyl)-2-propyn-1-ol (5 g), p-toluenesulfonic acid (0.2 g) and chloroform (preserved with pentene, 10 mL) were combined in a reaction flask and stirred at room temperature for half hour. The reaction mixture was concentrated. The residue was purified by silica gel chromatography (ethyl acetate/hexanes (v/v): 1/1). The major fraction was collected from column and concentrated, methanol was added to the residue and the precipitation was

filtered, yielding 3 g of a yellow-green solid. MS analysis supports the molecular weight of 3-phenyl-3-(4-(2-hydroxyethoxy) phenyl)-6,11-dimethoxy-13,13-dimethyl-3H,13H-indeno[2',3':3,4]naphtho[1,2-b]pyran.

Step 3

The product of Example 2 Step 1 2,3-dimethoxy-7,7-dimethyl-9-carboxy-7H-benzo[C]fluoren-5-ol (0.77 g), 1-phenyl-1-(4-methoxyphenyl)-2-propyn-1-ol (1 g, made as described in Example 1, Step 1 of U.S. Patent No. 5,458,814), pyridinium p-toluenesulfonate (0.04 g), trimethyl orthoformate (0.5 mL) and chloroform (preserved with pentene, 50 mL) were combined in a reaction flask and stirred at reflux for 22 hours. The reaction mixture was concentrated, and the residue was added to acetone and t-butyl methyl ether (v/v: 1:1), the slurry was filtered, yielding 1 g of a yellow-green solid. MS analysis supports the molecular weight of 3-phenyl-3-(4-methoxyphenyl)-6,7-dimethoxy-11-carboxy-13,13-dimethyl-3H,13H-indeno[2',3':3,4]naphtho[1,2-b]pyran. The product was used without further purification in the subsequent reaction.

Step 4

3-Phenyl -3-(4-(2-hydroxyethoxy)phenyl)-6,11-dimethoxy-13,13-dimethyl-3H,13H-indeno[2',3':3,4]naphtho [1,2-b]pyran from Step 2 (0.7g), 3-phenyl-3-(4-methoxyphenyl)-6,7-dimethoxy-11-carboxy-13,13-dimethyl-3H,13H-indeno[2',3':3,4]naphtho[1,2-b]pyran from Step 3 (0.5 g), dicyclohexyl carbodiimide (1 g), 4-(dimethylamino)-pyridine (0.17 g) and dichloromethane (50 mL) were added to a flask and heated at reflux for 27 hours. The reaction mixture was concentrated, and the residue was purified by silica gel chromatography (dichloromethane/hexanes/methanol (v/v/v): 10/10/1). The major fraction was collected from column and concentrated to 0.7 g of blue-green foam. MS analysis supports the molecular weight of 3-phenyl-3-(4-methoxyphenyl)-6,7-dimethoxy-13,13-dimethyl-11-(2-(4-(3-phenyl-6,11-dimethoxy-13,13 dimethyl-3H,13H-indeno[2',3':3,4]naphtho[1,2-b]pyran-3-yl)phenoxy)ethoxycarbonyl)-3H,13H-indeno [2',3':3,4]naphtho[1,2-b]pyran.

Example 15:

Step 1

*p*-Hydroxybenzophenone (45 g), 3,4-dihydro-2H-pyran (30 mL), dodecylbenzenesulfonic acid (10 drops) and dichloromethane (450 mL) were combined to a reaction flask under nitrogen atmosphere. The mixture was stirred at room temperature for 2 hours and poured into saturated aqueous sodium bicarbonate solution. The dichloromethane phase was separated and dried over sodium sulfate. The solution was filtered and concentrated. The residue was used in subsequent reaction without further purification.

Step 2

The product from Step 1 (80 g) was dissolved in anhydrous dimethylformamide (130 mL) in a reaction flask with overhead stirring, sodium acetylide in toluene (35 g, ~9wt%) was added to the reaction flask under vigorous stirring. After the reaction was complete, the mixture was poured into water (200 mL), and the solution was extracted with ethyl ether (three times with 200 mL). The extracts were combined and washed with saturated aqueous sodium chloride solution and dried over sodium sulfate. The solution was filtered and concentrated. The product was used in subsequent reaction without further purification.

Step 3

The product from Step 2 (80 g), p-toluenesulfonic acid (0.14g) and anhydrous methanol (50 mL) were combined in a reaction flask. The mixture was stirred at room temperature for 30 minutes and poured into saturated aqueous sodium bicarbonate solution (15 mL)/water (150 mL), the mixture was extracted with ethyl acetate (three times with 200 mL), and the extracts were combined and dried over sodium sulfate. The solution was filtered and concentrated. The product was used in subsequent reaction without further purification.

Step 4

The product of Example 2, Step 1 (2,3-dimethoxy-7,7-dimethyl-9-carboxy-7H-benzo[C]-fluoren-5-ol, g), the product from Step 3 (3 g), dodecylbenzenesulfonic acid (5 drops), tetrahydrofuran (5 mL), and chloroform (40 mL) were combined in a reaction flask, the mixture was heat at reflux for 2 hours, and then concentrated. Methanol was added to

the residue, and the slurry was filtered yielding 0.7 g of an off-white solid. MS analysis supports the molecular weight of 3-phenyl-3-(4-hydroxyphenyl)-6,7-dimethoxy-11-carboxy-13,13-dimethyl-3H,13H-indeno [2',3':3,4]naphtho[1,2-b] pyran.

Step 5

4-Fluorobenzophenone (30 g), piperazine (23 g), triethyl amine (23 mL), potassium carbonate (22 g) and dimethyl sulfoxide (50 mL) were combined in a reaction flask, and the mixture was heated at reflux for 20 hours. After this time, the mixture was cooled and poured into water, the slurry was extracted with chloroform and the chloroform phase was washed with water twice and dried over sodium sulfate. The solution was concentrated to 45 g of orange oil. The product was used in subsequent reaction without further purification.

Step 6

The procedure of Step 2 was followed except that the product from Step 5 was used in place of the product from Step 1. After the work-up, the residue was purified by silica gel chromatography (ethyl acetate/methanol (v/v): 1/1). The major fraction was collected from column and concentrated to 17 g of a yellowish solid.

Step 7

3,9-Dimethoxy-7,7-dimethyl-7H-benzo[C]fluoren-5-ol from Step 1 of Example 14 (1 g), the product from Step 6 (above) (3g), p-toluenesulfonic acid (0.2g) and chloroform (70 mL) were combined in a reaction flask, the mixture was stirred at room temperature for 20 minutes and then poured into saturated aqueous potassium carbonate solution (20 mL), the chloroform phase was separated and dried over sodium sulfate. The solution was filtered and concentrated. The residue was purified by silica gel chromatography (ethyl acetate/methanol (v/v): 1/1). The blue fraction was collected and concentrated, the residue was added to methanol and the slurry was filtered, yielding 0.6 g of a green solid. MS analysis supports the molecular weight of 3-phenyl-3-(4-piperazinophenyl)-6,11-dimethoxy-13,13-dimethyl-3H,13H-indeno[2',3':3,4]naphtho[1,2-b]pyran. The product was used without further purification in the subsequent reaction.

Step 8

3-Phenyl-3-(4-hydroxyphenyl)-6,7-dimethoxy-11-carboxy-13,13-dimethyl-3H,13H-indeno[2',3':3,4]naphtho [1,2-b]pyran from Step 4 (0.45 g), 2-isocyanatoethylmethacrylate (1.5 mL), dibutyltin dilaurate (1 drop) and dimethylformamide (3 mL) were combined in a reaction flask, the mixture was heated to 80°C for 2 hours. The mixture was poured into water and extracted with ethyl acetate. The extract was washed with water twice and dried over sodium sulfate. The solution was filtered and concentrated. The residue was added to acetone and methanol (v/v: 1/1), the slurry was filtered, yielding 0.6 g of a yellow solid.

Step 9

3-Phenyl-3-(4-piperazinophenyl)-6,11-dimethoxy-13,13-dimethyl-3H,13H-indeno[2',3':3,4]naphtho[1,2-b] pyran from Step 7 (0.5 g), 3-phenyl-3-(4-(2-methacryloxyethyl)carbamyloxyphenyl)-6,7-dimethoxy-11-carboxy-13,13-dimethyl-3H,13H-indeno[2',3':3,4]naphtho[1,2-b]pyran from Step 8 (0.7 g), dicyclohexyl carbodiimide (0.5 g), 4-(dimethylamino)-pyridine (0.08 g) and dimethylformamide (10 mL) were added to a flask and heated at 80°C for 18 hours. The mixture was poured into water, the slurry was filtered, and the solid (0.5 g) was further purified by silica gel chromatography (ethyl acetate/methanol (v/v): 1/1). The pure fraction was concentrated to yield 130 mg of an expanded blue-green foam. MS analysis supports the molecular weight of 3-phenyl-3-(4-(2-methacryloxyethyl)carbamyloxyphenyl)-6,7-dimethoxy-13,13-dimethyl-11-((1-(4-(3-phenyl-6,11-dimethoxy-13,13-dimethyl-3H,13H-indeno[2',3':3,4]naphtho[1,2-b]pyran-3-yl) phenyl)piperazino-4-yl)carbonyl)-3H,13H-indeno[2',3':3,4]naphtho[1,2-b]pyran.

Comparative Example CE1:

Step 1

Potassium t-butoxide (50.0 g) and benzophenone (100.0 g) were added to a reaction flask containing 500 mL of toluene under a nitrogen atmosphere. To the mixture was added dimethyl succinate (150.0 g) dropwise over 1 hour. The mixture was stirred for 5 hours at room temperature. The resulting mixture was poured into 300 mL of water and vigorously stirred for 20 minutes. The aqueous and organic phases were separated and the organic phases were

extracted with 100 mL portions of water three times. The combined aqueous layers were washed with 150 ml portions of chloroform three times. The aqueous layer was acidified to pH 2 with 6N HCl and a precipitate formed. The aqueous layer was extracted with three 100 mL portions of chloroform. The organic extracts were combined and concentrated by rotary evaporation. NMR spectra showed the product to have a structure of 4,4-diphenyl-3-methoxycarbonyl-3-butenoic acid.

Step 2

The crude half-ester from Step 1 (100.0 g), 60 mL of acetic anhydride, and 300 mL of toluene were added to a reaction flask under a nitrogen atmosphere. The reaction mixture was heated at 110° C for 6 hours, cooled to room temperature, and the solvents (toluene and acetic acid) removed by rotary evaporation. The residue was dissolved in 300 mL of methylene chloride and 200 mL of water. Solid $Na_2CO_3$ was added to the biphasic mixture until bubbling ceased. The layers separated and the aqueous layer was extracted with 50 mL portions of methylene chloride. The organic extracts were combined and the solvent removed by rotary evaporation to yield thick red oil. The oil was dissolved in warm methanol and chilled at 0° C for 2 hours. The resulting crystals were collected by vacuum filtration, washed with cold methanol to produce the 1-phenyl-2-methoxycarbonyl-4-acetoxynaphthalene. The product mixture was used without further purification in subsequent reaction.

Step 3

1-Phenyl-2-methoxycarbonyl-4-acetoxy-naphthalene from Step 2 (100 g), water (100 mL), methanol (200 mL), and sodium hydroxide (100 g) were combined in a reaction flask and heated to reflux for 5 hours. The reaction mixture was cooled to room temperature and was then slowly poured into mixture of water (1.5 L), conc. HCl (500 mL) and ice. A white solid precipitated and was filtered and washed with water. The solid was dissolved in a small amount of anhydrous tetrahydrofuran and then diluted with t-butyl methyl ether. This solution was washed with saturated aqueous NaCl and the organic layer was dried over anhydrous magnesium sulfate and concentrated by rotary evaporation to a light orange solid. NMR spectra showed the product to have a structure of 1-phenyl-2-carboxy-4-hydroxynaphthalene.

Step 4

1-Phenyl-2-carboxy-4-hydroxy-naphthalene from Step 3 (50 g), acetic anhydride (60 mL), 4-(dimethylamino) pyridine (200 mg), and 1,2,4-trimethylbenzene (500 mL) were combined in a reaction flask under a nitrogen atmosphere and heated to 50°C for one hour. Dodecylbenzene sulfonic acid (5.0 g) was added to the reaction mixture and the temperature increased to 144°C. After 28 hours the reaction mixture was slowly cooled to room temperature and a solid precipitated. The reaction mixture was filtered and washed with toluene yielding 40.0 g of a red solid 5-acetoxy-7H-benzo[C]fluoren-7-one. The product was used in the subsequent reaction without further purification.

Step 5

5-Acetoxy-7H-benzo[C]fluoren-7-one from Step 4 (10 g) and anhydrous tetrahydrofuran (150 mL) were combined in a reaction flask under a nitrogen atmosphere and cooled in an ice bath. To this was added 2 grams of NaH. The reaction mixture was allowed to warm to room temperature and was then poured into a mixture of saturated aqueous $NH_4Cl$ and ice (100 mL). The mixture was diluted with ethyl acetate (100 mL) and then the layers were separated. The aqueous layer was extracted with two 50 mL portions of ethyl acetate. The organic layers were combined and washed with saturated aqueous $NaHCO_3$ (100 mL), dried over $NaSO_4$, and concentrated by rotary evaporation to afford 5-hydroxy-7H-benzo[C]fluoren-7-ol.

Step 6

5-Hydroxy-7H-benzo[C]fluoren-5-ol from Step 5 (2.40 g), 1,1-bis(4-methoxyphenyl)-2-propyn-1-ol, (2.19 g, the product of Example 1, Step 1 of U.S. Patent No. 5,458,814), dodecylbenzene sulfonic acid (0.12 g) and chloroform (52 mL) were combined in a reaction flask and stirred at room temperature for 5 hours. The reaction mixture was washed with 50% saturated aqueous $NaHCO_3$ (200 mL) and the organic layer was dried over anhydrous sodium sulfate. The solvent was removed by rotary evaporation and the product was isolated by column chromatography (hexane/ethyl acetate: 2/1). NMR spectra showed the product to have a structure of 3,3-di(4-methoxyphenyl)-13-hydroxy-3H,13H-indeno[2',3' :3,4]naphtho[1,2-b]pyran.

Comparative Example CE2:

The procedures of comparative Example CE1 were followed except that 4,4'-dimethylbenzophenone was used in place of benzophenone to produce 3,3-di(4-methoxyphenyl)-6,11-dimethyl-13-hydroxy-3H,13H-indeno[2',3':3,4] naphtho[1,2-b]pyran.

Comparative Example CE3:

Step 1

The procedures of Steps 2-5 of Example 1 were followed except that naphthobenzophenone was used in place of 3,4-dimethoxy-4'-bromobenzophenone to produce 13,13-dimethyl-dibenzo[a,g]fluoren-11-ol.

Step2

13,13-Dimethyl-dibenzo[a,g]fluoren-11-ol from step 1 (2.50 g), 1,1-bis(4-methoxyphenyl)-2-propyn-1-ol (2.19g, the product of Example 1, Step 1 of U.S. Patent No. 5,458,814), dodecylbenzene sulfonic acid (0.12 g), and chloroform (52 mL) were combined in a reaction flask and stirred at room temperature for 5 hours. The reaction mixture was washed with 50 % saturated aqueous $NaHCO_3$ (200 mL) and the organic layer was dried over anhydrous sodium sulfate. The solvent was removed by rotary evaporation and the product was isolated by column chromatography (hexane/ethyl acetate: 85/15, $R_f$=0.3). NMR spectra showed the product to have a structure of 3,3-di(4-methoxyphenyl)-13,13-dimethyl-3H,13H-benz[p]-indeno[2',3':3,4]naphtho[1,2-b]pyran.

Comparative Example CE4:

Step 1

The procedures of Steps 1-5 of Example 1 were followed except that benzoyl chloride was used in place of bromobenzoyl chloride to produce 2,3-dimethoxy-7,7-dimethyl-7H-benzo[C]fluoren-5-ol.

Step 2

The procedure of Step 7 of Example 1 was followed except that 2,3-dimethoxy-7,7-dimethyl-7H-benzo[C] fluoren-5-ol of Step 1 was used in place of 2,3-dimethoxy-7,7-dimethyl-9-cyano-7H-benzo[C]fluoren-5-ol to produce 3,3-di(4-methoxyphenyl)-6,7-dimethoxy-13,13-dimethyl-3H,13H-indeno [2',3':3,4]naphtho[1,2-b]pyran.

PART 2: TESTING

Absorption Testing

The photochromic performance of the photochromic materials of Examples 1-15, Comparative Examples CE1-CE4, as well as eleven additional photochromic materials (Examples 16-26, listed below in Table 1) comprising a group that extends the pi-conjugated system of the indeno-fused naphthopyran bonded at the 11-position thereof were tested using the following optical bench set-up. It will be appreciated by those skilled in the art that the photochromic materials of Examples 16-26 may be made in accordance with the teachings and examples disclosed herein with appropriate modifications, which will be readily apparent to those skilled in the art. Further, those skilled in the art will recognize that various modifications to the disclosed methods, as well as other methods, may be used in making the photochromic materials of Examples 1-26.

Prior to testing the molar absorbance, a solution of each photochromic material in chloroform was made at a concentration as indicated in Table 1. Each solution was then placed in an individual test cell having a thickness of 1 cm and the test cells were measured for absorbance over a range of wavelengths ranging from 300 nm to 440 nm using a Cary 4000 UV spectrophotometer and a plot of absorbance vs. wavelength was obtained. The integrated extinction coefficient for each material tested was then determined by converting the absorption measurements to extinction coefficient and integrating the resultant plot over 320-420 nm using Igor program (distributed by WaveMetrics, Inc.).

Table 1: Absorption Test Data

| Example No. | Name | Conc. (M) | Area 320-420nm | Integrated Extinction Coeff. (nm x $mol^{-1}$ x $cm^{-1}$) |
|---|---|---|---|---|
| **1** | As set forth in Example 1 | $1.45 \times 10^{-4}$ | 195.8 | $1.4 \times 10^{6}$ |
| **2** | As set forth in Example 2 | $1.30 \times 10^{-4}$ | 173.9 | $1.3 \times 10^{6}$ |
| **3** | As set forth in Example 3 | $1.28 \times 10^{-4}$ | 175.5 | 1.4 X $10^{6}$ |
| **4** | As set forth in Example 4 | $1.36 \times 10^{-4}$ | 193.8 | $1.4 \times 10^{6}$ |
| **5** | As set forth in Example 5 | $1.26 \times 10^{-4}$ | 151.8 | $1.2 \times 10^{6}$ |
| **6** | As set forth in Example 6 | $1.16 \times 10^{-4}$ | 206.4 | $1.8 \times 10^{6}$ |
| **7** | As set forth in Example 7 | $1.24 \times 10^{-4}$ | 166.5 | $1.3 \times 10^{6}$ |
| **8** | As set forth in Example 8 | $1.28 \times 10^{-4}$ | 161.5 | $1.3 \times 10^{6}$ |
| **9** | As set forth in Example 9 | $1.33 \times 10^{-4}$ | 272.6 | $2.0 \times 10^{6}$ |
| **10** | As set forth in Example 10 | $1.23 \times 10^{-4}$ | 161.4 | $1.3 \times 10^{6}$ |
| **11** | As set forth in Example 11 | $1.02 \times 10^{-4}$ | 162.9 | $1.6 \times 10^{6}$ |
| **12** | As set forth in Example 12 | $7.52 \times 10^{-5}$ | 162.5 | $2.2 \times 10^{6}$ |
| **13** | As set forth in Example 13 | $8.78 \times 10^{-5}$ | 108.5 | $1.2 \times 10^{6}$ |
| **14** | As set forth in Example 14 | $1.25 \times 10^{-4}$ | 246.4 | $2.0 \times 10^{6}$ |
| **15** | As set forth in Example 15 | $2.32 \times 10^{-5}$ | 38.4 | $1.7 \times 10^{6}$ |
| **16** | 3,3-di(4-methoxyphenyl)-11-methoxycarboxy-13,13-dimethyl-3H,13H-indeno[2',3':3,4] naphtho[1,2-b]pyran | $1.52 \times 10^{-4}$ | 177.4 | $1.2 \times 10^{6}$ |
| **17** | 3-(4-morpholinophenyl)-3-phenyl-6,7-dimethoxy-11-carboxy-13,13-dimethyl-3H, 13H-indeno[2',3':3,4]naphtho [1,2-b] pyran | $1.30 \times 10^{-4}$ | 187.2 | $1.4 \times 10^{6}$ |
| **18** | 3-(4-morpholinophenyl)-3-phenyl-6,7-dimethoxy-11-methoxycarbonyl-13,13-dimethyl-3H, 13H-indeno[2',3':3,4] naphtho[1,2-b]pyran | $1.36 \times 10^{-4}$ | 201.9 | $1.5 \times 10^{6}$ |
| **19** | 3-(4-moipholinophenyl)-3-(4-metboxyphenyl)-6,7-dimethoxy-l1-(4-fluorophenyl)-13,13-dimethyl-3H,13H-indeno[2',3':3,4] naphtho[1,2-b]pyran | 1.24 x 10- | 152.0 | $1.2 \times 10^{6}$ |
| **20** | 3-(4-fluorophenyl)-3-(4-methoxyphenyl)-6,7-dimethoxy-11-cyano-13,13-dimethyl-3H,13H-indeno[2',3':3,4]naphtho[1,2-b] pyran | $1.46 \times 10^{-4}$ | 189.0 | $1.3 \times 10^{6}$ |

(continued)

| Example No. | Name | Conc. (M) | Area 320-420nm | Integrated Extinction Coeff. (nm x $mol^{-1}$ x $cm^{-1}$) |
|---|---|---|---|---|
| **21** | 3-(4-morpholinophenyl)-3-(4-methoxyphenyl)-11-(2-phenylethynyl)-13,13-dimethyl-3H,13H-indeno[2',3':3,4] naphtho[1,2-b]pyran | 1.29 x $10^{-4}$ | 277.5 | 2.1 x $10^{6}$ |
| **22** | 3,3-di(4-methoxyphenyl)-6,7-dimethoxy-11-(4-dimethylaminophenyl)-13,13-dimethyl-3H,13H-indeno[2',3':3,4]naphtho[1,2-b] pyran | 1.25 x $10^{-4}$ | 275.9 | 2.2 x $10^{6}$ |
| **23** | 3,3-di(4-methoxyphenyl)-6,7-dimethoxy-11-(4-methoxyphenyl)-13,13-dimethyl-3H,13H-indeno[2',3':3,4]naphtho[1,2-b]pyran | 1.26 x $10^{-4}$ | 185.4 | 1.5 x $10^{6}$ |
| **24** | 3,3-di(4-methoxyphenyl)-6-methoxy-7-morpholino-11-phenyl-13-butyl-13-(2-(2-hydroxyethoxy)ethoxy)-3H,13H-indeno[2',3':3,4]naphtho [1,2-b]pyran | 1.03 x $10^{-4}$ | 170.7 | 1.7 x $10^{6}$ |
| **25** | 3-(4-fluorophenyl)-3-(4-methoxyphenyl)-6-methoxy-7-morpholino-11-phenyl-13-butyl-13-(2-(2-hydroxyethoxy) ethoxy)-3H,13H-indeno[2',3':3,4]naphtho[1,2-b]pyran | 1.03 x $10^{-4}$ | 168.2 | 1.6 x $10^{6}$ |
| **26** | 3,3-di(4-fluorophenyl)-11-cyano-13,13 dimethyl-3H,13H-indeno[2',3':3,4] naphthyl [1,2-b]pyran | 1.62 x $10^{-4}$ | 181.5 | 1.1 x $10^{6}$ |
| **CE1** | As set forth in Comparative Example 1 | 1.88 x $10^{-4}$ | 109.8 | 5.8 x $10^{5}$ |
| **CE2** | As set forth in Comparative Example 2 | 1.63 x$10^{-4}$ | 93.9 | 5.8 x $10^{5}$ |
| **CE3** | As set forth in Comparative Example 3 | 1.44 x $10^{-4}$ | 144.1 | 1.0 x $10^{6}$ |
| **CE4** | As set forth in Comparative Example 4 | 1.64 x $10^{-4}$ | 94.1 | 5.7 x $10^{5}$ |

As can be seen from the data in Table 1, the photochromic materials according to various non-limiting embodiments disclosed herein (Example Nos. 1-26) all had integrated extinction coefficients greater than 1.0 x $10^{6}$ nm x $mol^{-1}$ x $cm^{-1}$, whereas the photochromic materials of comparative examples CE1-CE4 did not.

<u>Photochromic Performance Testing</u>

The photochromic performance of the photochromic materials of Examples 1-15, Comparative Examples CE1-CE4, as well as the eleven additional photochromic materials (Examples 16-26, listed above in Table 1) were tested as follows.

A quantity of the photochromic material to be tested calculated to yield a 1.5 x $10^{-3}$ M solution was added to a flask containing 50 grams of a monomer blend of 4 parts ethoxylated bisphenol A dimethacrylate (BPA 2EO DMA), 1 part poly(ethylene glycol) 600 dimethacrylate, and 0.033 weight percent 2,2'-azobis(2-methyl propionitrile) (AIBN). The photochromic material was dissolved into the monomer blend by stirring and gentle heating. After a clear solution was obtained, it was vacuum degassed before being poured into a flat sheet mold having the interior dimensions of 2.2 mm x 6 inches (15.24 cm) x 6 inches (15.24 cm). The mold was sealed and placed in a horizontal air flow, programmable oven programmed to increase the temperature from 40°C to 95°C over a 5 hour interval, hold the temperature at 95°C for 3 hours and then lower it to 60°C for at least 2 hours. After the mold was opened, the polymer sheet was cut using a diamond blade saw into 2 inch (5.1 cm) test squares.

The photochromic test squares prepared as described above were tested for photochromic response on an optical bench. Prior to testing on the optical bench, the photochromic test squares were exposed to 365 nm ultraviolet light for about 15 minutes to cause the photochromic material to transform from the unactived (or bleached) state to an activated (or colored) state, and then placed in a 75°C oven for about 15 minutes to allow the photochromic material to revert back to the bleached state. The test squares were then cooled to room temperature, exposed to fluorescent room lighting for at least 2 hours, and then kept covered (that is, in a dark environment) for at least 2 hours prior to testing on an optical bench maintained at 23°C (73°F). The bench was fitted with a 300-watt xenon arc lamp, a remote controlled shutter, a Melles Griot KG2 filter that modifies the UV and IR wavelengths and acts as a heat-sink, neutral density filter (s) and a sample holder, situated within a water bath, in which the square to be tested was inserted. A collimated beam of light from a tungsten lamp was passed through the square at a small angle (approximately 30°) normal to the square. After passing through the square, the light from the tungsten lamp was directed to a collection sphere, where the light was blended, and on to an Ocean Optics S2000 spectrometer where the spectrum of the measuring beam was collected and analyzed. The $\lambda_{max-vis}$ is the wavelength in the visible spectrum at which the maximum absorption of the activated (colored) form of the photochromic compound in a test square occurs. The $\lambda_{max-vis}$ wavelength was determined by testing the photochromic test squares in a Varian Cary 300 UV-Visible spectrophotometer; it may also be calculated from the spectrum obtained by the S2000 spectrometer on the optical bench.

The saturated optical density ("Sat'd OD") for each test square was determined by opening the shutter from the xenon lamp and measuring the transmittance after exposing the test chip to UV radiation for 30 minutes. The $\lambda_{max-vis}$ at the Sat'd OD was calculated from the activated data measured by the S2000 spectrometer on the optical bench. The First Fade Half Life ("T1/2") is the time interval in seconds for the absorbance of the activated form of the photochromic material in the test squares to reach one half the Sat'd OD absorbance value at room temperature 23°C (73°F), after removal of the source of activating light. Results for the photochromic materials tested are listed below in Table 2.

Table 2: Photochromic Test Data

| Example No. | T1/2 (at $\lambda_{max-vis}$) | Sat'd OD (at $\lambda_{max-vis}$) | $\lambda_{max-vis}$ | Example No. | T1/2 (at $\lambda_{max-vis}$) | Sat'd OD (at $\lambda_{max-vis}$) | $\lambda_{max-vis}$ |
|---|---|---|---|---|---|---|---|
| 1 | 66 | 0.58 | 459 | 16 | 50 | 0.42 | 560 |
| 2 | 121 | 0.80 | 455 | 17 | 220 | 0.85 | 603 |
| 3 | 116 | 0.79 | 457 | 18 | 199 | 0.81 | 603 |
| 4 | 112 | 0.37 | 456 | 19 | 180 | 0.57 | 607 |
| 5 | 238 | 1.09 | 452 | 20 | 134 | 0.86 | 449 |
| 6 | 242 | 1.01 | 452 | 21 | 41 | 0.48 | 605 |
| 7 | 245 | 1.15 | 451 | 22 | 415 | 0.87 | 451 |
| 8 | 197 | 0.93 | 457 | 23 | 325 | 0.64 | 451 |
| 9 | 183 | 0.89 | 453 | 24 | 91 | 0.79 | 476 |
| 10 | 94 | 0.60 | 458 | 25 | 123 | 1.08 | 469 |
| 11 | 480 | 0.97 | 448 | 26 | 130 | 0.69 | 530 |
| 12 | 593 | 0.67 | 475 | CE1 | 99 | 0.68 | 569 |
| 13 | 921 | 0.65 | 580 | CE2 | * | * | * |
| 14 | 896 | 0.86 | 589 | CE3 | 129 | 0.81 | 572 |

(continued)

| Example No. | T1/2 (at $\lambda_{max-vis}$) | Sat'd OD (at $\lambda_{max-vis}$) | $\lambda_{max-vis}$ | Example No. | T1/2 (at $\lambda_{max-vis}$) | Sat'd OD (at $\lambda_{max-vis}$) | $\lambda_{max-vis}$ |
|---|---|---|---|---|---|---|---|
| 15 | 866 | 0.69 | 602 | CE4 | 236 | 1.27 | 451 |
| *Not tested | | | | | | | |

PART 3: MODELED SYSTEMS

Modeled 3H,13H-Indeno[2',3':3,4]naphtho[1,2-b]pyrans

The substituent effect on UV absorption and intensity at the 11-position of the 3H,13H-indeno[2',3':3,4]naphtho[1,2-b]pyrans were calculated using density functional theory implemented in Gaussian98 software, which is purchased from Gaussian, Inc. of Wallingford, CT. Model systems were designed based on the 3H,13H-indeno[2',3':3,4]naphtho[1,2-b]pyrans with substitution at the 11-position of the indeno-fused naphthopyran (substituents at the 3-position were replaced with hydrogen atoms for ease of modeling). Geometry was first optimized using Becke's parameter functional in combination with the Lee, Yang, and Parr (LYP) correlation function and the 6-31G(d) basis set (B3LYP/6-31G(d)). The absorption spectra were calculated using time dependent density functional theory (TDDFT) with B3LYP functional and 6-31+G(d) basis set. The longest absorption ($\lambda_{max}$) and correspondent intensity calculated by TDDFT/6-31+G(d) are shown below in Table 3. All structures were optimized using B3LYP/6-31G(d).

Table 3: Modeled Intensity Data for Closed Form of Model Photochromic Materials

| Modeled Photochromic Material | $\lambda_{max}$ (nm) | Modeled Intensity at $\lambda_{max}$ | Modeled Photochromic Material | $\lambda_{max}$ (nm) | Modeled Intensity at $\lambda_{max}$ |
|---|---|---|---|---|---|
| 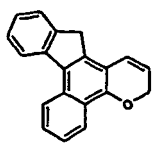 MPM1 | 383 | 0.12 | 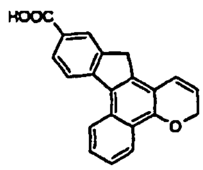 MPM2 | 388 | 0.31 |
| 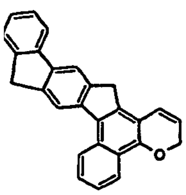 MPM3 | 402 | 0.31 | 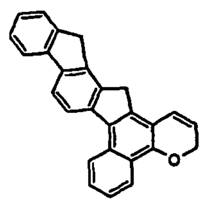 MPM4 | 399 | 0.28 |
| 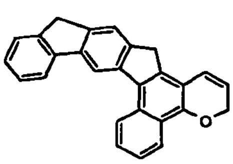 MPM5 | 391 | 0.17 | 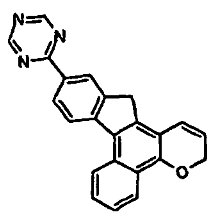 MPM6 | 419 | 0.57 |
| 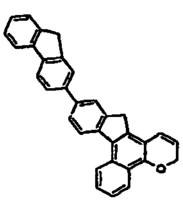 MPM7 | 400 | 0.48 | 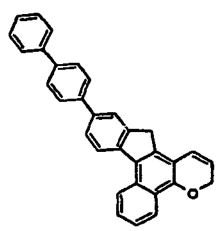 MPM8 | 397 | 0.44 |

(continued)

| Modeled Photochromic Material | $\lambda_{max}$ (nm) | Modeled Intensity at $\lambda_{max}$ | Modeled Photochromic Material | $\lambda_{max}$ (nm) | Modeled Intensity at $\lambda_{max}$ |
|---|---|---|---|---|---|
| 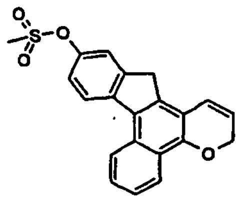 MPM9 | 382 | 0.17 | 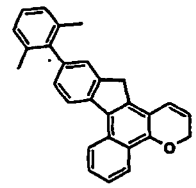 MPM10 | 385 | 0.16 |
| 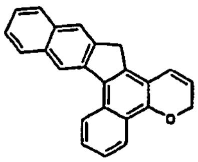 MPM11 | 395 | 0.19 | 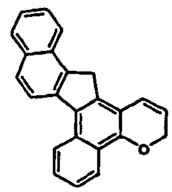 MPM12 | 393 | 0.20 |
| 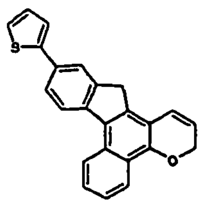 MPM13 | 405 | 0.38 | 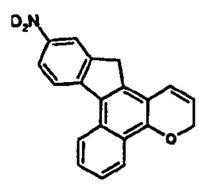 MPM14 | 445 | 0.37 |
| 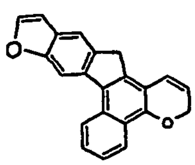 MPM15 | 395 | 0.18 | | | |

The modeling data indicates that groups that extend the pi-conjugated system of the 3H,13H-indeno[2',3':3,4] naphtho[1,2-b]pyrans bonded at the 11-position thereof have an increased modeled intensity and a bathochromic shift in $\lambda_{max}$ as compared to comparable photochromic materials without a group that extends the pi-conjugated system of the indeno-fused naphthopyran bonded at the 11-position thereof (for example MPM1).

Further, modeled photochromic materials having a group bonded at the 11-position but that does not extend the pi-conjugated system of the indeno-fused naphtho pyran along the 11-position, for example MPM5, MPM9, and MPM10, do not appear to have a significant increase in modeled intensity as compared to MPM1. Modeled photochromic materials having a fused-group that is bonded at both the 11-position and the 10-position or the 11-position and 12-position of the indeno-fused naphthopyran, wherein the fused group extends the pi-conjugated system of the indeno-fused naphthopyran at both bonding positions (for example, MPM11 and MPM12) generally had a smaller increase in modeled intensity than those modeled photochromic materials that had a fused group that extends the pi-conjugated systems of the indeno-fused naphthopyran only at the 11-position (for example, MPM3 and MPM4) or indeno-fused naphthopyrans having a group that extends the pi-conjugated system thereof bonded at the 11-position only. The modeled intensity data for MPM2, MPM8 and MPM12 is consistent with the integrated extinction coefficient measurements for similar compounds as described above.

<u>Modeled 2H,13H-Indeno[1',2':4.3]naphtho[2.1-b]pyrans</u>

The substituent effect on UV absorption and intensity at the 11-position of the 2H,13H-indeno[1',2';4,3]naphtho [2,1-b]pyran was calculated using the same procedure as described for the 3H,13H-indeno[2',3':3,4]naphtho[1,2-b] pyrans. Model systems were designed based on the 2H,13H-indeno[1',2':4,3]naphtho[2,1-b]pyrans with substitution at the 11-position of the indeno-fused naphthopyran (substituents at the 2-position were replaced with hydrogen atoms for

ease of modeling). The absorption spectra were calculated using time dependent density functional theory (TDDFT) with B3LYP functional and 6-31+G(d) basis set. The longest absorption ($\lambda_{max}$) and correspondent intensity calculated by TDDFT/6-31+G(d) are shown below in Table 4. All structures were optimized using B3LYP/6-31 G(d). As shown in Table 4, extending the conjugation at the 11-position increases the absorption intensity.

Table 4: Modeled Intensity Data for Closed Form of Model Photochromic Materials

| **Modeled Photochromic Material** | **$\lambda_{max}$ (nm)** | **Modeled Intensity at $\lambda_{max}$** |
|---|---|---|
| 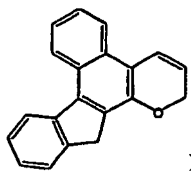 MPM16 | 383 | 0.33 |
| 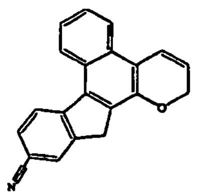 MPM17 | 402 | 0.42 |
| 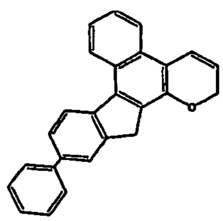 MPM18 | 396 | 0.57 |

As can be seen from Table 4, both MPM 17 and MPM 18 (which had a cyano and a phenyl group, respectively, extending the pi-conjugated system of the indeno-fused naphthopyran bonded at the 11-position thereof) had higher modeled intensities and a bathochromically shifted $\lambda_{max}$ as compared to MPM16, which did not have a group that extended the pi-conjugated system of the indeno-fused naphthopyran bonded at the 11-position thereof.

Modeled 3H,13H- benzothieno [2',3':3,4]naphtho[1,2-b]pyrans

The substituent effect on UV absorption and intensity at the 11-position of the 3H,13H-benzothieno[2',3':3,4]naphtho[1,2-b]pyran was calculated using the same procedure as described for the 3H,13H-indeno[2',3':3,4]naphtho[1,2-b]pyrans. Model systems were designed based on the 3H,13H-benzothieno[2',3':3,4]naphtho[1,2-b]pyrans with substitution at the 11-position of the benzothioeno-fused naphthopyran (substituents at the 3-position were replaced with hydrogen atoms for ease of modeling). The absorption spectra were calculated using time dependent density functional theory (TDDFT) with B3LYP functional and 6-31+G(d) basis set. The longest absorption ($\lambda_{max}$) and correspondent intensity calculated by TDDFT/6-31+G(d) are shown below in Table 5. All structures were optimized using B3LYP/6-31G(d). As shown in Table 5, extending the conjugation at the 11-position increases the absorption intensity.

Table 5: Modeled Intensity Data for Closed Form of Model Photochromic Materials

| **Modeled Photochromic Material** | **$\lambda_{max}$ (nm)** | **Modeled Intensity at $\lambda_{max}$** |
|---|---|---|
| 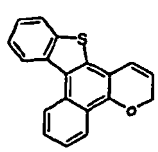 MPM19 | 373 | 0.10 |

(continued)

| Modeled Photochromic Material | $\lambda_{max}$ (nm) | Modeled Intensity at $\lambda_{max}$ |
|---|---|---|
| 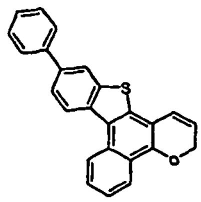 MPM20 | 383 | 0.22 |

As can be seen from Table 5, MPM 20 (which had a phenyl group, extending the pi-conjugated system of the benzothieno-fused naphthopyran bonded at the 11-position thereof) had a higher modeled intensity and a bathochromically shifted $\lambda_{max}$ as compared to MPM19, which did not have a group that extended the pi-conjugated system of the benzothieno-fused naphthopyran bonded at the 11-position thereof.

It is to be understood that the present description illustrates aspects of the invention relevant to a clear understanding of the invention. Certain aspects of the invention that would be apparent to those of ordinary skill in the art and that, therefore, would not facilitate a better understanding of the invention have not been presented in order to simplify the present description.

## Claims

1. A photochromic material comprising:

    (i) an indeno-fused naphthopyran; and
    (ii) a group that extends the pi-conjugated system of the indeno-fused naphthopyran bonded at the 11-position thereof, provided that if the group bonded at the 11-position of the indeno-fused naphthopyran and a group bonded at the 10-position or 12-position of the indeno-fused naphthopyran together form a fused group, said fused group is not a benzo-fused group; and

    wherein the 13-position of the indeno-fused naphthopyran is unsubstituted, mono-substituted or di-substituted, provided that if the 13-position of the indeno-fused naphthopyran is di-substituted, the substituents do not together form norbornyl.

2. The photochromic material of claim 1 wherein the photochromic material comprises an indeno[2',3':3,4]naphtho[1,2-b]pyran, an indeno[1',2':4,3]naphtho[2,1-b]pyran or a mixture thereof.

3. The photochromic material of claim 1 wherein the photochromic material comprises at least one of a reactive substituent and a compatibilizing substituent, each of said reactive substituent or compatibilizing substituent being independently represented by one of:

    -A'-D-E-G-J;

    -G-E-G-J;

    -D-E-G-J;

    -A'-D-J;

    -D-G-J;

    -D-J;

    -A'-G-J;

-G-J; and

-A'-J;

wherein:

(i) each -A'- is independently -O-, -C(=O)-, $-CH_2-$, -OC(=O)- or -NHC(=O)-, provided that if -A'- is -O-, -A'- forms at least one bond with -J:
(ii) each -D- is independently:

(a) a diamine residue or a derivative thereof, said diamine residue being an aliphatic diamine residue, a cyclo aliphatic diamine residue, a diazacycloalkane residue, an azacyclo aliphatic amine residue, a diazacrown ether residue or an aromatic diamine residue, wherein a first amino nitrogen of said diamine residue forms a bond with -A'-, the group that extends the pi-conjugated system of the indeno-fused naphthopyran bonded at the 11-position thereof, or a substituent or an available position on the indeno-fused naphthopyran, and a second amino nitrogen of said diamine residue forms a bond with -E-, -G- or -J; or
(b) an amino alcohol residue or a derivative thereof, said amino alcohol residue being an aliphatic amino alcohol residue, a cyclo aliphatic amino alcohol residue, an azacyclo aliphatic alcohol residue, a diazacyclo aliphatic alcohol residue or an aromatic amino alcohol residue, wherein an amino nitrogen of said amino alcohol residue forms a bond with -A'-, the group that extends the pi-conjugated system of the indeno-fused naphthopyran bonded at the 11-position thereof, or a substituent or an available position on the indeno-fused naphthopyran, and an alcohol oxygen of said amino alcohol residue forms a bond with -E-, -G- or -J, or said amino nitrogen of said amino-alcohol residue forms a bond with -E-, -G- or -J, and said alcohol oxygen of said amino alcohol residue forms a bond with -A'-, the group that extends the pi-conjugated system of the indeno-fused naphthopyran bonded at the 11-position thereof, or a substituent or an available position on the indeno-fused naphthopyran;

(iii) each -E- is independently a dicarboxylic acid residue or a derivative thereof, said dicarboxylic acid residue being an aliphatic dicarboxylic acid residue, a cycloaliphatic dicarboxylic acid residue or an aromatic dicarboxylic acid residue, wherein a first carbonyl group of said dicarboxylic acid residue forms a bond with -G- or -D-, and a second carbonyl group of said dicarboxylic acid residue forms a bond with -G-;
(iv) each -G- is independently:

(a) $-[(OC_2H_4)_x(OC_3H_6)_y(OC_4H_8)_z]-O-$, wherein x, y and z are each independently chosen and range from 0 to 50, and a sum of x, y, and z ranges from 1 to 50;
(b) a polyol residue or a derivative thereof, said polyol residue being an aliphatic polyol residue, a cyclo aliphatic polyol residue or an aromatic polyol residue, wherein a first polyol oxygen of said polyol residue forms a bond with -A'-, -D-, -E-, the group that extends the pi-conjugated system of the indeno-fused naphthopyran bonded at the 11-position thereof, or a substituent or an available position on the indeno-fused naphthopyran, and a second polyol oxygen of said polyol residue forms a bond with -E- or -J; or
(c) a combination thereof, wherein the first polyol oxygen of the polyol residue forms a bond with a group $-[(OC_2H_4)_x(OC_3H_6)_y(OC_4H_6)_z]-$ and the second polyol oxygen forms a bond with -E- or -J; and

(v) each -J is independently:

(a) a group -K, wherein -K is $-CH_2COOH$, $-CH(CH_3)COOH$, $-C(O)(CH_2)_wCOOH$, $-C_6H_4SO_3H$, $-C_5H_{10}SO_3H$, $-C_4H_8SO_3H$, $-C_3H_6SO_3H$, $-C_2H_4SO_3H$ or $-SO_3H$, wherein w ranges from 1 to 18;
(b) hydrogen, provided that if -J is hydrogen, -J is bonded to an oxygen of -D- or -G-, or a nitrogen of -D-; or
(c) a group -L or residue thereof, wherein -L is acryl, methacryl, crotyl, 2-(methacryloxy)ethylcarbamyl, 2-(methacryloxy)-ethoxycarbonyl, 4-vinylphenyl, vinyl, 1-chlorovinyl or epoxy.

**4.** The photochromic material of any of claims 1 to 3 wherein the group that extends the pi-conjugated system of the indeno-fused naphthopyran bonded at the 11-position thereof is a substituted or unsubstituted aryl; a substituted or unsubstituted heteroaryl; or a group represented by -X=Y or -X'≡ Y', wherein:

(i) X is $-CR^1$, -N, -NO, $-SR^1$, $-S(=O)R^1$ or $-P(=O)R^1$, wherein $R^1$ is amino, dialkyl amino, diaryl amino, acyloxy, acylamino, a substituted or unsubstituted $C_1$-$C_{20}$ alkyl, a substituted or unsubstituted $C_2$-$C_{20}$ alkenyl, a substituted or unsubstituted $C_2$-$C_{20}$ alkynyl, halogen, hydrogen, hydroxy, oxygen, a polyol residue, a substituted or

unsubstituted phenoxy, a substituted or unsubstituted benzyloxy, a substituted or unsubstituted alkoxy, a substituted or unsubstituted oxyalkoxy, alkylamino, mercapto, alkylthio, a substituted or unsubstituted aryl, a substituted or unsubstituted heteroaryl, a substituted or unsubstituted heterocyclic group, a reactive substituent or a compatibilizing substituent as defined in claim 3 or a photochromic material, provided that:

(a) if X is $-CR^1$ or -N, Y is $C(R^2)_2$, $NR^2$, O or S, wherein each $R^2$ is independently chosen for each occurrence from amino, dialkyl amino, diaryl amino, acyloxy, acylamino, a substituted or unsubstituted $C_1$-$C_{20}$ alkyl, a substituted or unsubstituted $C_2$-$C_{20}$ alkenyl, a substituted or unsubstituted $C_2$-$C_{20}$ alkynyl, halogen, hydrogen, hydroxy, oxygen, a polyol residue, a substituted or unsubstituted phenoxy, a substituted or unsubstituted benzyloxy, a substituted or unsubstituted alkoxy, a substituted or unsubstituted oxyalkoxy, alkylamino, mercapto, alkylthio, a substituted or unsubstituted aryl, a substituted or unsubstituted heteroaryl, a substituted or unsubstituted heterocyclic group, a reactive substituent or a compatibilizing substituent as defined in claim 3 and a photochromic material; and
(b) if X is -NO, $-SR^1$, $-S(=O)R^1$ or $-P(=O)R^1$, Y is O; and

(ii) X' is -C or $-N^+$, and Y' is $CR^3$ or N; wherein $R^3$ is amino, dialkyl amino, diaryl amino, acyloxy, acylamino, a substituted or unsubstituted $C_1$-$C_{20}$ alkyl, a substituted or unsubstituted $C_2$-$C_{20}$ alkenyl, a substituted or unsubstituted $C_2$-$C_{20}$ alkynyl, halogen, hydrogen, hydroxy, oxygen, a polyol residue, a substituted or unsubstituted phenoxy, a substituted or unsubstituted benzyloxy, a substituted or unsubstituted alkoxy, a substituted or unsubstituted oxyalkoxy, alkylamino, mercapto, alkylthio, a substituted or unsubstituted aryl, a substituted or unsubstituted heteroaryl, a substituted or unsubstituted heterocyclic group, a reactive substituent or a compatibilizing substituent as defined in claim 3 or a photochromic material; or
the group that extends the pi-conjugated system of the indeno-fused naphthopyran bonded at the 11-position of the indeno-fused naphthopyran together with a group bonded at the 12-position of the indeno-fused naphthopyran or together with a group bonded at the 10-position of the indeno-fused naphthopyran form a fused group, said fused group being indeno, dihydronaphthalene, indole, benzofuran, benzopyran or thianaphthene.

**5.** The photochromic material of claim 4 in as much claim 4 depends on claim 1, wherein the group that extends the pi-conjugated system of the indeno-fused naphthopyran is: a substituted or unsubstituted $C_2$-$C_{20}$ alkenyl; a substituted or unsubstituted $C_2$-$C_{20}$ alkynyl; a substituted or unsubstituted aryl; a substituted or unsubstituted heteroaryl; $-C(=O)R^1$; or -N(=Y) or $-N^+(\equiv Y')$, wherein Y is $C(R^2)_2$, $NR^2$, O or S, and Y' is $CR^3$ or N.

**6.** The photochromic material of claim 5 wherein the group that extends the pi-conjugated system of the indeno-fused naphthopyran bonded at the 11-position thereof is an aryl group or a heteroaryl group that is unsubstituted or substituted with at least one of a substituted or unsubstituted alkyl, a substituted or unsubstituted alkoxy, a substituted or unsubstituted oxyalkoxy, amide, a substituted or unsubstituted amino, a substituted or unsubstituted aryl, a substituted or unsubstituted heteroaryl, azide, carbonyl, carboxy, ester, ether, halogen, hydroxy, a polyol residue, a substituted or unsubstituted phenoxy, a substituted or unsubstituted benzyloxy, cyano, nitro, sulfonyl, thiol, a substituted or unsubstituted heterocyclic group, a reactive substituent or a compatibilizing substituent as defined in claim 3, or a photochromic material, provided that if the aryl group or the heteroaryl group comprises more than one substituent, each substituent may be independently chosen.

**7.** The photochromic material of claim 5 wherein the group that extends the pi-conjugated system of the indeno-fused naphthopyran bonded at the 11-position thereof is $-C(=O)R^1$, wherein $R^1$ is acylamino, acyloxy, a substituted or unsubstituted $C_1$-$C_{20}$ alkyl, a substituted or unsubstituted alkoxy, a substituted or unsubstituted oxyalkoxy, amino, dialkyl amino, diaryl amino, a substituted or unsubstituted aryl, a substituted or unsubstituted heteroaryl, a substituted or unsubstituted heterocyclic group, halogen, hydrogen, hydroxy, oxygen, a polyol residue, a substituted or unsubstituted phenoxy, a substituted or unsubstituted benzyloxy, a reactive substituent as defined in claim 3 or a photochromic material.

**8.** The photochromic material of claim 3 in as much claim 3 depends on claim 1 wherein the photochromic material comprises an indeno[2',3':3,4]naphtho[1,2-b]pyran and at least one of the 6-position, the 7-position, the 13-position, the 3-position, and the group that extends the pi-conjugated system of the indeno[2',3':3,4]naphtho[1,2-b]pyran bonded at the 11-position thereof comprises a reactive substituent as defined in claim 3.

**9.** The photochromic material of claim 1 wherein the indeno-fused naphthopyran is free of spiro-cyclic groups at the 13-position of the indeno-fused naphthopyran.

**10.** The photochromic material of claim 1 wherein the indeno-fused naphthopyran is an indeno[2',3':3,4]naphtho[1,2-b]pyran, and wherein:

(i) the 6-position of the indeno[2',3':3,4]naphtho[1,2-b]pyran is substituted with a nitrogen containing group or an oxygen containing group;
(ii) the 7-position of indeno[2',3':3,4]naphtho[1,2-b]pyran is substituted with a nitrogen containing group or an oxygen containing group; and
(iii) the 13-position of the indeno[2',3':3,4]naphtho[1,2-b]pyran is di-substituted, provided that each of the substituents at the 13-position is independently hydrogen, $C_1$-$C_6$ alkyl, $C_3$-$C_7$ cycloalkyl, allyl, a substituted or unsubstituted phenyl, a substituted or unsubstituted benzyl, a substituted or unsubstituted amino or -C(O)$R^{30}$ wherein $R^{30}$ is hydrogen, hydroxy, $C_1$-$C_6$ alkyl, $C_1$-$C_6$ alkoxy, an unsubstituted, mono- or di-substituted phenyl or naphthyl, phenoxy, a mono- or di-($C_1$-$C_6$)alkyl substituted phenoxy or a mono-or di-($C_1$-$C_6$)alkoxy substituted phenoxy.

**11.** A photochromic composition comprising the photochromic material of claim 1 incorporated into at least a portion of an organic material, said organic material being a polymeric material, an oligomeric material, a monomeric material or a mixture or combination thereof.

**12.** The photochromic composition of claim 11 wherein the organic material is a polymeric material, said polymeric material being a copolymer of ethylene and vinyl acetate; a copolymer of ethylene and vinyl alcohol; a copolymer of ethylene, vinyl acetate and vinyl alcohol; cellulose acetate butyrate; poly(urethane); poly(acrylate); poly(methacrylate); epoxy; an aminoplast functional polymer; poly(anhydride); poly(urea urethane); a N-alkoxymethyl(meth) acrylamide functional polymer; poly(siloxane); poly(silane); or a mixture or combination thereof.

**13.** The photochromic composition of claim 11 wherein the photochromic composition comprises at least one of a complementary photochromic material, a photoinitiator, a thermal initiator, a polymerization inhibitor, a solvent, a light stabilizer, a heat stabilizer, a mold release agent, a rheology control agent, a leveling agent, a free radical scavenger, and an adhesion promoter.

**14.** The photochromic composition of claim 11 wherein the photochromic composition is a coating composition.

**15.** A photochromic article comprising a substrate and a photochromic material according to claim 1 connected to at least a portion of the substrate.

**16.** The photochromic article of claim 15 wherein the photochromic article is an optical element, said optical element being at least one of an ophthalmic element, a display element, a window, a mirror and a liquid crystal cell element.

**17.** The photochromic articles of claim 16 wherein the optical element is an ophthalmic element, said ophthalmic element being at least one of a corrective lens, a non-corrective lens, a magnifying lens, a protective lens, a visor, goggles and a lens for an optical instrument.

**18.** The photochromic article of claim 15 wherein the substrate comprises a polymeric material and the photochromic material is incorporated into at least a portion of the polymeric material.

**19.** The photochromic article of claim 18 wherein the photochromic material is at least one of blended with at least a portion of the polymeric material, bonded to at least a portion of the polymeric material, and imbibed into at least a portion of the polymeric material.

**20.** The photochromic article of claim 15 wherein the photochromic article comprises an at least partial coating connected to at least a portion of the substrate, said at least partial coating comprising the photochromic material.

**21.** The photochromic article of claim 20 wherein the substrate is a polymeric material or glass.

**22.** The photochromic article of claim 15 wherein at least one at least partial coating or film is connected to at least a portion of the substrate, the at least one at least partial coating or film being at least one of a primer coating or film, a protective coating or film, an anti-reflective coating or film, a conventional photochromic coating or film, and a polarizing coating or film.

**23.** The photochromic article of claim 15 wherein the photochromic article comprises at least one of a complementary photochromic material, a photoinitiator, a thermal initiator, a polymerization inhibitor, a solvent, a light stabilizer, a heat stabilizer, a mold release agent, a rheology control agent, a leveling agent, a free radical scavenger, and an adhesion promoter.

**24.** A method of making a photochromic article comprising connecting a photochromic material according to claim 1 to at least a portion of a substrate, wherein connecting the photochromic material to at least a portion of the substrate comprises at least one of in-mold casting, coating, imbibition, lamination and casting-in-place.

**25.** A photochromic material according to claim 1 represented by:

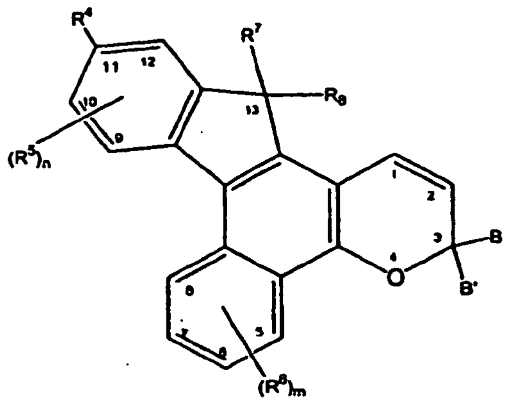
,
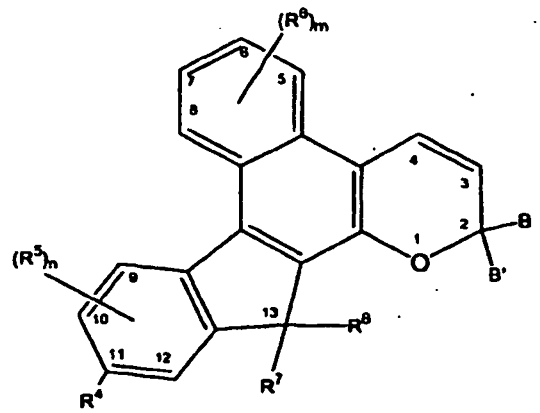

or a mixture thereof, wherein:

(i) $R^4$ is a substituted or unsubstituted aryl; a substituted or unsubstituted heteroaryl; or a group represented by -X=Y or -X'≡ Y', wherein:

(a) X is $-CR^1$, -N, -NO, $-SR^1$ $-S(=O)R^1$ or $-P(=O)R^1$, wherein $R^1$ is amino, dialkyl amino, diaryl amino, acyloxy, acylamino, a substituted or unsubstituted $C_1$-$C_{20}$ alkyl, a substituted or unsubstituted $C_2$-$C_{20}$ alkenyl, a substituted or unsubstituted $C_2$-$C_{20}$ alkynyl, halogen, hydrogen, hydroxy, oxygen, a polyol residue, a substituted or unsubstituted phenoxy, a substituted or unsubstituted benzyloxy, a substituted or unsubstituted alkoxy, a substituted or unsubstituted oxyalkoxy, alkylamino, mercapto, alkylthio, a substituted or unsubstituted aryl, a substituted or unsubstituted heteroaryl, a substituted or unsubstituted heterocyclic group, a reactive substituent or a compatibilizing substituent as defined in claim 3, or a photochromic material, provided that:

(1) if X is $-CR^1$ or -N, Y is $C(R^2)_2$, $NR^2$, O, or S, wherein each $R^2$ is independently chosen for each occurrence from amino, dialkyl amino, diaryl amino, acyloxy, acylamino, a substituted or unsubstituted $C_1$-$C_{20}$ alkyl, a substituted or unsubstituted $C_2$-$C_{20}$ alkenyl, a substituted or unsubstituted $C_2$-$C_{20}$ alkynyl, halogen, hydrogen, hydroxy, oxygen, a polyol residue, a substituted or unsubstituted phenoxy, a substituted or unsubstituted benzyloxy, a substituted or unsubstituted alkoxy, a substituted or unsubstituted oxyalkoxy, alkylamino, mercapto, alkylthio, a substituted or unsubstituted aryl, a substituted or unsubstituted heteroaryl, a substituted or unsubstituted heterocyclic group, a reactive substituent or a compatibilizing substituent as defined in claim 3; and a photochromic material; and
(2) if X is -NO, $-SR^1$, $-S(=O)R^1$ or $-P(=O)R^1$, Y is O; and

(b) X' is -C or $-N^+$, and Y' is $CR^3$ or N; wherein $R^3$ is amino, dialkyl amino, diaryl amino, acyloxy, acylamino, a substituted or unsubstituted $C_1$-$C_{20}$ alkyl, a substituted or unsubstituted $C_2$-$C_{20}$ alkenyl, a substituted or unsubstituted $C_2$-$C_{20}$ alkynyl, halogen, hydrogen, hydroxy, oxygen, a polyol residue, a substituted or unsubstituted phenoxy, a substituted or unsubstituted benzyloxy, a substituted or unsubstituted alkoxy, a substituted or unsubstituted oxyalkoxy, alkylamino, mercapto, alkylthio, a substituted or unsubstituted aryl, a substituted or unsubstituted heteroaryl, a substituted or unsubstituted heterocyclic group, a reactive substituent or a compatibilizing substituent as defined in claim 3; or a photochromic material; or

$R^4$ together with an $R^5$ group bonded at the 12-position of the indeno-fused naphthopyran or together with an $R^5$ group bonded at the 10-position of the indeno-fused naphthopyran form a fused group, said fused group being indeno, dihydronaphthalene, indole, benzofuran, benzopyran or thianaphthene;

(ii) n ranges from 0 to 3;

(iii) m ranges from 0 to 4;

(iv) each $R^5$ and $R^6$ is independently chosen for each occurrence from: a reactive substituent or a compatibilizing substituent as defined in claim 3; hydrogen; $C_1$-$C_6$ alkyl; chloro; fluoro; $C_3$-$C_7$ cycloalkyl; a substituted or unsubstituted phenyl, said phenyl substituents being $C_1$-$C_6$ alkyl or $C_1$-$C_6$ alkoxy; $-OR^{10}$ or $-OC(=O)R^{10}$ wherein $R^{10}$ is S, hydrogen, amine, $C_1$-$C_6$ alkyl, phenyl($C_1$-$C_3$)alkyl, mono($C_1$-$C_6$)alkyl substituted phenyl($C_1$-$C_3$)alkyl, mono($C_1$-$C_6$)alkoxy substituted phenyl($C_1$-$C_3$)alkyl , ($C_1$-$C_6$)alkoxy($C_2$-$C_4$)alkyl, $C_3$-$C_7$ cycloalkyl or mono ($C_1$-$C_4$)alkyl substituted $C_3$-$C_7$ cycloalkyl; a mono-substituted phenyl, said phenyl having a substituent located at the para position, the substituent being a dicarboxylic acid residue or derivative thereof, a diamine residue or derivative thereof, an amino alcohol residue or derivative thereof, a polyol residue or derivative thereof, -($CH_2$)-, $-(CH_2)_r-$ or $-[O-(CH_2)_t]_k-$, wherein t ranges from 2 to 6, and k ranges from 1 to 50, and wherein the substituent is connected to an aryl group on another photochromic material $-N(R^{11})R^{12}$, wherein $R^{11}$ and $R^{12}$ are each independently hydrogen, $C_1$-$C_8$ alkyl, phenyl, naphthyl, furanyl, benzofuran-2-yl, benzofuran-3-yl, thienyl, benzothien-2-yl, benzothien-3-yl, dibenzofuranyl, dibenzothienyl, benzopyridyl and fluorenyl, $C_1$-$C_8$ alkylaryl, $C_3$-$C_{20}$ cycloalkyl, $C_4$-$C_{20}$ bicycloalkyl, $C_5$-$C_{20}$ tricycloalkyl or $C_1$-$C_{20}$ alkoxyalkyl, or $R^{11}$ and $R^{12}$ come together with the nitrogen atom to form a $C_3$-$C_{20}$ hetero-bicycloalkyl ring or a $C_4$-$C_{20}$ hetero-tricycloalkyl ring; a nitrogen containing ring represented by:

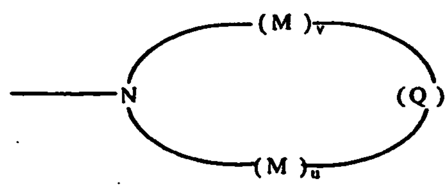

wherein each -M- is independently chosen for each occurrence from $-CH_2-$, $-CH(R^{13})-$, $-C(R^{13})_2-$, -CH(aryl)-, $-C(aryl)_2-$ and $-C(R^{13})(aryl)-$, and -Q- is -M-, -O-, -S-, -S(O)-, $-SO_2-$, -NH-, $-N(R^{13})-$ or -N(aryl)-, wherein each $R^{13}$ is independently $C_1$-$C_6$ alkyl, each (aryl) is independently phenyl or naphthyl, u ranges from 1 to 3, and v ranges from 0 to 3, provided that if v is 0, -Q- is -M-; a group represented by:

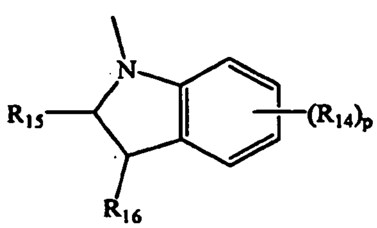

or

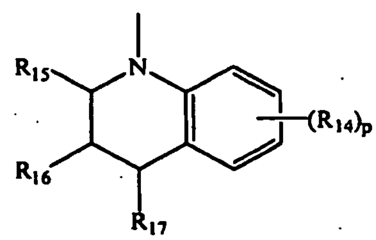

wherein each $R^{15}$, $R^{16}$ and $R^{17}$ is independently hydrogen, $C_1$-$C_6$ alkyl, phenyl or naphthyl, or $R^{15}$ and $R^{16}$ together form a ring of 5 to 8 carbon atoms, each $R^{14}$ is independently $C_1$-$C_6$ alkyl, $C_1$-$C_6$ alkoxy, fluoro or chloro, and p ranges from 0 to 3; and a substituted or unsubstituted $C_4$-$C_{18}$ spirobicyclic amine or a substituted or unsubstituted $C_4$-$C_{18}$ spirotricyclic amine, wherein said substituents are independently aryl, $C_1$-$C_6$ alkyl, $C_1$-$C_6$ alkoxy or phenyl($C_1$-$C_6$)alkyl; or

an $R^6$ group in the 6-position and an $R^6$ group in the 7-position together form a group represented by:

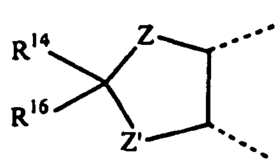

or

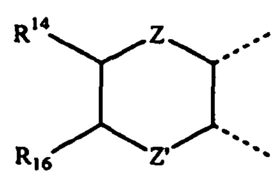

wherein each Z and Z' is independently oxygen or the group $-NR^{11}-$ wherein $R^{11}$, $R^{14}$ and $R^{16}$ are as set forth above;

(v) $R^7$ and $R^8$ are each independently:

a reactive substituent or a compatibilizing substituent as defined in claim 3; hydrogen; hydroxy; $C_1$-$C_6$ alkyl; $C_3$-$C_7$ cycloalkyl; allyl; a substituted or unsubstituted phenyl or benzyl, wherein each of said phenyl and benzyl substituents is independently $C_1$-$C_6$ alkyl or $C_1$-$C_6$ alkoxy; chloro; fluoro; a substituted or unsubstituted amino; $-C(O)R^9$ wherein $R^9$ is hydrogen, hydroxy. $C_1$-$C_6$ alkyl, $C_1$-$C_6$ alkoxy, an unsubstituted, mono- or di-substituted phenyl or naphthyl wherein each of said substituents is independently $C_1$-$C_6$ alkyl or $C_1$-$C_6$ alkoxy, phenoxy, mono- or di-($C_1$-$C_6$)alkyl substituted phenoxy, mono- or di-($C_1$-$C_6$)alkoxy substituted phenoxy, amino, mono- or di-($C_1$-$C_6$)alkylamino, phenylamino, mono- or di-($C_1$-$C_6$)alkyl substituted phenylamino or mono- or di-($C_1$-$C_6$)alkoxy substituted phenylamino; $-OR^{18}$ wherein $R^{18}$ is $C_1$-$C_6$ alkyl, phenyl($C_1$-$C_3$)alkyl, mono($C_1$-$C_6$)alkyl substituted phenyl($C_1$-$C_3$)alkyl, mono($C_1$-$C_6$)alkoxy substituted phenyl($C_1$-$C_3$)alkyl, $C_1$-$C_6$ alkoxy($C_2$-$C_4$)alkyl, $C_3$-$C_7$ cycloalkyl, mono($C_1$-$C_4$)alkyl substituted $C_3$-$C_7$ cycloalkyl. $C_1$-$C_6$ chloroalkyl, $C_1$-$C_6$ fluoroalkyl, allyl or $-CH(R^{19})T$ wherein $R^{19}$ is hydrogen or $C_1$-$C_3$ alkyl, T is CN, $CF_3$ or $COOR^{20}$ wherein $R^{20}$ is hydrogen or $C_1$-$C_3$ alkyl, or wherein $R^{18}$ is -C(=O)U wherein U is hydrogen, $C_1$-$C_6$ alkyl, $C_1$-$C_6$ alkoxy, an unsubstituted, mono- or di-substituted phenyl or naphthyl, wherein each of said substituents are independently $C_1$-$C_6$ alkyl or $C_1$-$C_6$ alkoxy, phenoxy, mono- or di-($C_1$-$C_6$)alkyl substituted phenoxy, mono- or di-($C_1$-$C_6$)alkoxy substituted phenoxy, amino, mono- or di-($C_1$-$C_6$)alkylamino, phenylamino, mono- or di-($C_1$-$C_6$)alkyl substituted phenylamino or mono- or di-($C_1$-$C_6$)alkoxy substituted phenylamino; and a mono-substituted phenyl, said phenyl having a substituent located at the para position, the substituent being a dicarboxylic acid residue or derivative thereof, a diamine residue or derivative thereof, an amino alcohol residue or derivative thereof, a polyol residue or derivative thereof, $-(CH_2)-$, $-(CH_2)_t-$ or $-[O-(CH_2)_t]_k-$, wherein t ranges from 2 to 6 and k ranges from 1 to 50, and wherein the substituent is connected to an aryl group on another photochromic material; or $R^7$ and $R^8$ together form an oxo group; a spiro-carbocyclic group containing 3 to 6 carbon atoms, provided that the spiro-carbocyclic group is not norbornyl; or a spiro-heterocyclic group containing 1 to 2 oxygen atoms and 3 to 6 carbon atoms including the spirocarbon atom, said spiro-carboxyclic and spiro-heterocyclic groups being annellated with 0,1, or 2 benzene rings; and

(vi) B and B' are each independently:

an aryl group that is mono-substituted with a reactive substituent or a compatibilizing substituent as defined in claim 3; an unsubstituted, mono-, di- or tri-substituted aryl group; 9-julolidinyl; an unsubstituted, mono- or di-substituted heteroaromatic group chosen from pyridyl, furanyl, benzofuran-2-yl, benzofuran-3-yl, thienyl, benzothien-2-yl, benzothien-3-yl, dibenzofuranyl, dibenzothienyl, carbazoyl, benzopyridyl, indolinyl and fluorenyl; wherein the aryl and heteroaromatic substituents are each independently: hydroxy, aryl, mono- or di-($C_1$-$C_{12}$)alkoxyaryl, mono- or di-($C_1$-$C_{12}$)alkylaryl, haloaryl, $C_3$-$C_7$ cycloalkylaryl, $C_3$-$C_7$ cycloalkyl, $C_3$-$C_7$ cycloalkyloxy, $C_3$-$C_7$ cycloalkyloxy($C_1$-$C_{12}$)alkyl, $C_3$-$C_7$ cycloalkyloxy($C_1$-$C_{12}$)alkoxy, aryl($C_1$-$C_{12}$)alkyl, aryl($C_1$-$C_{12}$)alkoxy, aryloxy, aryloxy($C_1$-$C_{12}$)alkyl, aryloxy($C_1$-$C_{12}$)alkoxy, mono- or di-($C_1$-$C_{12}$)alkylaryl($C_1$-$C_{12}$)alkyl, mono- or di-($C_1$-$C_{12}$)alkoxyaryl-($C_1$-$C_{12}$)alkyl, mono- or di-($C_1$-$C_{12}$)alkylaryl($C_1$-$C_{12}$)alkoxy, mono- or di-($C_1$-$C_{12}$)alkoxyaryl($C_1$-$C_{12}$)alkoxy, amino, mono- or di-($C_1$-$C_{12}$)-alkylamino, diarylamino, piperazino, N-($C_1$-$C_{12}$)alkylpiperazino, N-arylpiperazino, aziridino, indolino, piperidino, morpholino, thiomorpholino, tetrahydroquinolino, tetrahydroisoquinolino, pyrrolidyl, $C_1$-$C_{12}$ alkyl, $C_1$-$C_{12}$ haloalkyl, $C_1$-$C_{12}$ alkoxy, mono($C_1$-$C_{12}$ )alkoxy($C_1$-$C_{12}$)alkyl, acryloxy, methacryloxy, halogen, or $-C(=O)R^{21}$ wherein $R^{21}$ is $-OR^{22}$, $-N(R^{23})R^{24}$, piperidino or morpholino, wherein $R^{22}$ is allyl, $C_1$-$C_6$ alkyl, phenyl, mono($C_1$-$C_6$)alkyl substituted phenyl, mono($C_1$-$C_6$)alkoxy substituted phenyl, phenyl($C_1$-$C_3$)alkyl , mono($C_1$-$C_6$)alkyl substituted phenyl($C_1$-$C_3$)alkyl, mono($C_1$-$C_6$)alkoxy substituted phenyl($C_1$-$C_3$)alkyl, $C_1$-$C_6$ alkoxy($C_2$-$C_4$)alkyl or $C_1$-$C_6$ haloalkyl, and $R^{23}$ and $R^{24}$ are each independently $C_1$-$C_6$ alkyl, $C_5$-$C_7$ cycloalkyl or a substituted or unsubstituted phenyl, said phenyl substituents independently being $C_1$-$C_6$ alkyl or $C_1$-$C_6$ alkoxy; an unsubstituted or mono-substituted group chosen from pyrazolyl, imidazolyl, pyrazolinyl, imidazolinyl, pyrrolinyl, phenothiazinyl, phenoxazinyl, phenazinyl and acridinyl, said substituents being $C_1$-$C_{12}$ alkyl, $C_1$-$C_{12}$ alkoxy, phenyl or halogen; a mono-substituted phenyl, said phenyl having a substituent located at the para position, the substituent being a dicarboxylic acid residue or derivative thereof, a diamine residue or derivative thereof, an amino alcohol residue or derivative thereof, a polyol residue or derivative thereof, $-(CH_2)-$, $-(CH_2)_t-$ or $-[O-(CH_2)_t]_k-$, wherein t ranges form 2 to 6 and k ranges from 1 to 50, and wherein the substituent is connected to an aryl group on another photochromic material; a group represented by:

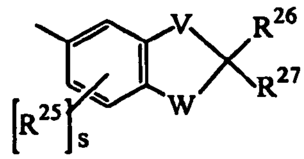

or

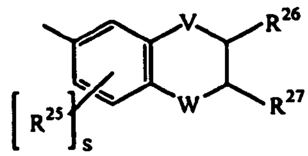

wherein V is $-CH_2-$ or -O-, W is oxygen or substituted nitrogen, provided that when W is substituted nitrogen, V is $-CH_2-$, the substituted nitrogen substituents being hydrogen, $C_1$-$C_{12}$ alkyl or $C_1$-$C_{12}$ acyl, each $R^{25}$ independently being $C_1$-$C_{12}$ alkyl, $C_1$-$C_{12}$ alkoxy, hydroxy or halogen, $R^{26}$ and $R^{27}$ are each independently hydrogen or $C_1$-$C_{12}$ alkyl, and s ranges from 0 to 2; or a group represented by:

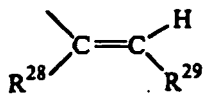

wherein $R^{28}$ is hydrogen or $C_1$-$C_{12}$ alkyl, and $R^{29}$ is an unsubstituted, mono- or di-substituted naphthyl, phenyl, furanyl or thienyl, said substituents being $C_1$-$C_{12}$ alkyl, $C_1$-$C_{12}$ alkoxy or halogen; or B and B' taken together form a fluoren-9-ylidene or mono- or di-substituted fluoren-9-ylidene, each of said fluoren-9-ylidene substituents independently being $C_1$-$C_{12}$ alkyl, $C_1$-$C_{12}$ alkoxy or halogen.

**26.** The photochromic material of claim 25 wherein at least one of an $R^6$ group at the 6-position, an $R^6$ group at the 7-position, B, B', $R^7$, $R^8$ and $R^4$ comprises a reactive substituent as defined in claim 3.

**27.** The photochromic material of claim 3 wherein the indeno-fused naphthopyran is an indeno[2',3':3,4]naphtho[1,2-b]pyran and wherein:

(i) each of an $R^6$ group at the 7-position and an $R^6$ group at the 6-position of the indeno[2',3':3,4]naphtho[1,2-b]pyran is independently $-OR^{10}$ wherein $R^{10}$ is $C_1$-$C_6$ alkyl, a substituted or unsubstituted phenyl, said phenyl substituents being $C_1$-$C_6$ alkyl or $C_1$-$C_6$ alkoxy, phenyl($C_1$-$C_3$)alkyl, mono($C_1$-$C_6$)alkyl substituted phenyl($C_1$-$C_3$) alkyl, mono($C_1$-$C_6$)alkoxy substituted phenyl($C_1$-$C_3$)alkyl, ($C_1$-$C_6$)alkoxy($C_2$-$C_4$)alkyl, $C_3$-$C_7$ cycloalkyl or mono ($C_1$-$C_4$)alkyl substituted $C_3$-$C_7$ cycloalkyl; $-N(R^{11})R^{12}$ wherein $R^{11}$ and $R^{12}$ are each independently hydrogen, $C_1$-$C_8$ alkyl, $C_1$-$C_8$ alkylaryl, $C_3$-$C_{20}$ cycloalkyl, $C_4$-$C_{20}$ bicycloalkyl, $C_5$-$C_{20}$ tricycloalkyl or $C_1$-$C_{20}$ alkoxyalkyl, wherein said aryl group is phenyl or naphthyl; a nitrogen containing ring represented by:

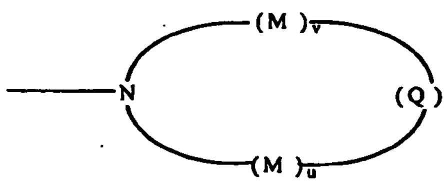

wherein each -M- is independently chosen for each occurrence from $-CH_2-$, $-CH(R^{13})-$, $-C(R^{13})_2-$, -CH(aryl)-, $-C(aryl)_2-$ and $-C(R^{13})(aryl)-$, and -Q- is -M-, -O-, -S-, -NH-, $-N(R^{13})-$ or -N(aryl)-, wherein each $R^{13}$ is independently $C_1$-$C_6$ alkyl, each (aryl) is independently phenyl or naphthyl, u ranges from 1 to 3, and v ranges from 0 to 3, provided that if v is 0, -Q- is -M-; or a reactive substituent or a compatibilizing substituent as defined in claim 3, provided that the reactive or compatibilizing substituent comprises a linking group comprising an aliphatic amino alcohol residue, a cyclo aliphatic amino alcohol residue, an azacyclo aliphatic alcohol residue, a diazacyclo aliphatic alcohol residue, a diamine residue, an aliphatic diamine residue, a cyclo aliphatic diamine residue, a diazacycloalkane residue, an azacyclo aliphatic amine residue; an oxyalkoxy group, an aliphatic polyol residue or a cyclo aliphatic polyol residue that forms a bond with the indeno[2',3':3,4]naphtho[1,2-b]pyran at the 6-position or the 7-position; or

(ii) an $R^6$ group in the 6-position and an $R^6$ group in the 7-position of the indeno[2',3':3,4]naphtho[1,2-b]pyran together form a group represented by:

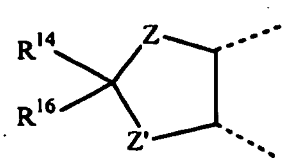

or

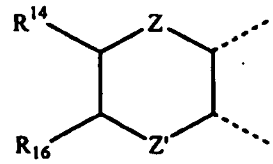

wherein Z and Z' are each independently oxygen or $-NR^{11}-$, wherein $R^{11}$ is as set forth above in (i).

**28.** The photochromic material of claim 3 wherein the photochromic material is chosen from:

(i) a 3,3-di(4-methoxyphenyl)-6,7-dimethoxy-11-cyano-13,13-dimethyl-3H,13H-indeno[2',3':3,4]naphtho[1,2-b]pyran;
(ii) a 3,3-di(4-methoxyphenyl)-6,7-dimethoxy-11-carboxy-13,13-dimethyl-3H,13H-indeno[2',3':3,4]naphtho[1,2-b]pyran;
(iii) a 3,3-di(4-methoxyphenyl)-6,7-dimethoxy-11-methoxycarbonyl-13,13-dimethyl-3H,13H-indeno[2',3':3,4]naphtho[1,2-b]pyran;
(iv) a 3.3-di(4-methoxyphenyl)-6,7-dimethoxy-11-(2-(2-hydroxyethoxy)ethoxycarbonyl-13,13-dimethyl-3H,13H-indeno[2',3':3,4]naphtho[1,2-b]pyran;
(v) a 3,3-di(4-methoxyphenyl)-6,7-dimethoxy-11-(4-fluorophenyl)-13,13-dimethyl-3H,13H-indeno[2',3':3,4]naphtho[1,2-b]pyran;
(vi) a 3.3-di(4-methoxyphenyl)-6,7-dimethoxy-11-(4-(phenyl)phenyl)-13,13-dimethyl-3H,13H-indeno[2',3':3,4]naphtho[1,2-b]pyran;
(vii) a 3,3-di(4-methoxyphenyl)-6,7-dimethoxy-11-(4-(hydroxymethyl)phenyl)-13,13-dimethyl-3H,13H-indeno[2',3':3,4]naphtho[1,2-b]pyran;
(viii) a 3,3-di(4-methoxyphenyl)-6,7-dimethoxy-11-(3-hydroxy-3-methylbutynyl)-13,13-dimethyl-3H,13H-indeno[2',3':3,4]naphtho[1,2-b]pyran;
(ix) a 3,3-di(4-methoxyphenyl)-6,7-dimethoxy-11-(2-phenylethynyl) 13,13-dimethyl-3H,13H-indeno[2',3':3,4]naphtho[1,2-b]pyran;
(x) a 3,3-di(4-methoxyphenyl)-8,7-dimethoxy-11-phenyl-13-ethyl, 13-methoxy-3H,13H-indeno[2',3':3,4]naphtho[1,2-b]pyran;
(xi) a 3-phenyl-3-(4-methoxyphenyl)-6,7-dimethoxy-11-(4-(2-methacryloxyethoxy)carbonylphenyl)-13,13-dimethyl-3H,13H-indeno[2',3':3,4] naphtho[1,2-b]pyran;
(xii) a 3,3-di(4-methoxyphenyl)-6-methoxy-7-((3-(2-methacryloxyethyl)carbamyloxymethylenepiperidino)-1-yl)-11-(4-(phenyl)phenyl)-13,13-dimethyl-3H,13H-indeno[2',3':3,4]naphtho[1,2-b]pyran;
(xiii) a 3-phenyl-3-(4-(2-(2-methacryloxyethyl)carbamyloxyethoxy)phenyl)-6-methoxy-11-phenyl-13,13-dimethyl-3H,13H-indeno[2',3':3,4]naphtho[1,2-b]pyran;
(xiv) a 3-phenyl-3-(4-methoxyphenyl)-6,7-dirnethoxy-13,13-dimethyl-11-(2-(4-(3-phenyl-6,11-dimethoxy-13,13-dimethyl-3H,13H-indeno[2',3':3,4]naphtho[1,2-b]pyran-3-yl)phenoxy)ethoxycarbonyl)-3H,13H-indeno[2',3':3,4]naphtho[1,2-b]pyran;
(xv) a 3-phenyl-3-(4-(2-methacryloxyethyl)carbamyloxyphenyl)-6,7-dimethoxy-13,13-dimethyl-11-((1-(4-(3-phenyl-6,11-dimethoxy-13,13-dimethyl-3H,13H-indeno[2',3':3,4]naphtho[1,2-b]pyran-3-yl)phenyl)piperazino-4-yl)carbonyl)-3H,13H-indeno[2',3':3,4]naphtho[1,2-b]pyran;
(xvi) a 3,3-di(4-methoxyphenyl)-11-methoxycarboxy-13,13-dimethyl-3H,13H-indeno[2',3':3,4]naphtho[1,2-b]pyran;
(xvii) a 3-(4-morpholinophenyl)-3-phenyl-6,7-dimethoxy-11-carboxy-13,13-dimethyl-3H,13H-indeno[2',3':3,4]naphtho[1,2-b]pyran;
(xviii) a 3-(4-morpholinophenyl)-3-phenyl-6,7-dimethoxy-11-methoxycarbonyl-13,13-dimethyl-3H,13H-indeno[2',3':3,4]naphtho[1,2-b]pyran;
(xix) a 3-(4-morpholinophenyl)-3-(4-methoxyphenyl)-6,7-dimethoxy-11-(4-fluorophenyl)-13,13-dimethyl-3H,13H-indeno[2',3':3,4]naphtho[1,2-b]pyran;
(xx) a 3-(4-fluorophenyl)-3-(4-methoxyphenyl)-6,7-dimethoxy-11-cyano-13,13-dimethyl-3H,13H-indeno[2',3':3,4]naphtho[1,2-b]pyran;
(xxi) a 3-(4-morpholinophenyl)-3-(4-methoxyphenyl)-11-(2-phenylethynyl)-13,13-dimethyl-3H,13H-indeno[2',3':3,4]naphtho[1.2-b]-pyran;
(xxii) a 3,3-di(4-methoxyphenyl)-6,7-dimethoxy-11-(4-dimethylaminophenyl)-13,13-dimethyl-3H, 13H-indeno[2',3':3,4]naphtho[1,2-b]pyran;

(xxiii) a 3,3-di(4-methoxyphenyl)-6,7-dimethoxy-11-(4-methoxyphenyl)-13,13-dimethyl-3H,13H-indeno[2',3':3,4]naphtho[1,2-b]pyran;
(xxiv) a 3,3-di(4-methoxyphenyl)-6-methoxy-7-morpholino-11-phenyl-13-butyl-13-(2-(2-hydroxyethoxy)ethoxy)-3H,13H-indeno[2',3':3,4] naphtho[1,2-b]pyran;
(xxv) a 3-(4-fluorophenyl)-3-(4-methoxyphenyl)-6-methoxy-7-morpholino-11-phenyl-13-butyl-13-(2-(2-hydroxyethoxy)ethoxy)-3H,13H-indeno[2',3':3,4]naphtho[1,2-b]pyran;
(xxvi) a 3,3-di(4-fluorophenyl)-11-cyano-13, 13-dimethyl-3H,13H-indeno[2',3':3,4]naphtho[1,2-b]pyran;
(xxvii) a 3-(4-morpholinophenyl)-3-phenyl-6-methoxy-7-(3-(2-methacryloxyethyl)carbamyloxymethylenepiperidino-1-yl)-11-phenyl-13,13-dimethyl-3H,13H-indeno[2',3':3,4]naphtho[1,2-b]pyran;
(xxviii) a 3-(4-(2-(2-methacryloxyethyl)carbamylethoxy)phenyl)-3-phenyl-6,7-d imethoxy-11-phenyl-13,13-dimethyl-3H,13H-indeno[2',3':3,4]naphtho[1,2-b]pyran; and mixtures thereof.

**Patentansprüche**

1. Photochromes Material, enthaltend:

   (i) ein indenokondensiertes Naphthopyran und
   (ii) eine Gruppe, die das konjugierte $\pi$-System des indenokondensierten Naphthopyrans erweitert und an dessen 11-Position gebunden ist, vorausgesetzt, dass, wenn die Gruppe, die an die 11-Position des indenokondensierten Naphthopyrans gebunden ist, und eine Gruppe, die an die 10-Position oder 12-Position des indenokondensierten Naphthopyrans gebunden ist, zusammen eine kondensierte Gruppe bilden, diese kondensierte Gruppe keine benzokondensierte Gruppe ist, und

   wobei die 13-Position des indenokondensierten Naphthopyrans unsubstituiert, monosubstituiert oder disubstituiert ist, vorausgesetzt, dass, wenn die 13-Position des indenokondensierten Naphthopyrans disubstituiert ist, die Substituenten zusammen kein Norbornyl bilden.

2. Photochromes Material nach Anspruch 1, wobei das photochrome Material ein Indeno[2',3':3,4]naphtho[1,2-b]pyran, ein Indeno[1',2':4,3]naphtho[2,1-b]pyran oder eine Mischung davon enthält.

3. Photochromes Material nach Anspruch 1, wobei das photochrome Material wenigstens eines von einem reaktiven Substituenten und einem kompatibilisierenden Substituenten enthält, wobei jeder dieser reaktiven Substituenten oder kompatibilisierenden Substituenten unabhängig voneinander ausgewählt ist aus einem von:

   -A'-D-E-G-J;

   -G-E-G-J;

   -D-E-G-J;

   -A'-D-J;

   -D-G-J;

   -D-J;

   -A'-G-J;

   -G-J;

   und

   -A'-J;

   worin:

   (i) jedes -A'- unabhängig -O-, -C(=O)-, $-CH_2-$, -OC(=O)- oder -NHC(=O)- ist, vorausgesetzt, dass wenn -A'-

gleich -O- ist, -A'-wenigstens eine Bindung mit -J bildet,
(ii) jedes -D- unabhängig voneinander:

(a) ein Diaminrest oder ein Derivat davon ist, wobei dieser Diaminrest ein aliphatischer Diaminrest, ein cycloaliphatischer Diaminrest, ein Diazacycloalkanrest, ein azacycloaliphatischer Aminrest, ein Diazakronetherrest oder ein aromatischer Diaminrest ist, wobei ein erster Aminostickstoff dieses Diaminrests eine Bindung mit -A'-, der Gruppe, die das konjugierte π-System des indenokondensierten Naphthopyrans erweitert und an dessen 11-Position gebunden ist, oder einem Substituenten oder einer zugänglichen Position an dem indenokondensierten Naphthopyran bildet und ein zweiter Aminostickstoff dieses Diaminrests eine Bindung mit -E-, -G- oder -J bildet, oder
(b) ein Aminoalkoholrest oder ein Derivativ davon ist, wobei dieser Aminoalkoholrest ein aliphatischer Aminoalkoholrest, ein cycloaliphatischer Aminoalkoholrest, ein azacycloaliphatischer Aminoalkoholrest, ein diazacycloaliphatischer Aminoalkoholrest oder ein aromatischer Aminoalkoholrest ist, wobei ein Aminostickstoff dieses Aminoalkoholrests eine Bindung mit -A'-, der Gruppe, die das konjugierte π-system des indenokondensierten Naphthopyrans erweitert und an dessen 11-Position gebunden ist, oder einem Substituenten oder einer zugänglichen Position an dem indenokondensierten Naphthopyran bildet und ein Alkoholsauerstoff dieses Aminoalkoholrests eine Bindung mit -E-, -G- oder -J bildet oder dieser Aminostickstoff dieses Aminoalkoholrests eine Bindung mit -E-, -G- oder -J bildet und dieser Alkoholsauerstoff dieses Aminoalkoholrests eine Bindung mit -A'-, der Gruppe, die das konjugierte π-System des indenokondensierten Naphthopyrans erweitert und an dessen 11-Position gebunden ist, oder einem Substituenten oder einer zugänglichen Position an dem indenokondensierten Naphthopyran bildet,

(iii) jedes -E- unabhängig voneinander ein Dicarbonsäurerest oder ein Derivat davon ist, wobei dieser Dicarbonsäurerest ein aliphatischer Dicarbonsäurerest, ein cycloaliphatischer Dicarbonsäurerest oder ein aromatischer Dicarbonsäurerest ist, wobei eine erste Carbonylgruppe dieses Dicarbonsäurerests eine Bindung mit -G- oder -D-bildet und eine zweite Carbonylgruppe dieses Dicarbonsäurerests eine Bindung mit -G- bildet,
(iv) jedes -G- unabhängig voneinander:

(a) $-[(OC_2H_4)_x(OC_3H_6)_y(OC_4H_8)_z]-O-$ ist, worin x, y und z jeweils unabhängig voneinander ausgewählt sind und von 0 bis 50 reichen und eine Summe von x, y und z von 1 bis 50 reicht,
(b) ein Polyolrest oder ein Derivat davon ist, wobei dieser Polyolrest ein aliphatischer Polyolrest, ein cycloaliphatischer Polyolrest oder ein aromatischer Polyolrest ist, wobei ein erster Polyolsauerstoff dieses Polyolrests eine Bindung mit -A'-, -D-, -E-, der Gruppe, die das konjugierte π-System des indenokondensierten Naphthopyrans erweitert und an dessen 11-Position gebunden ist, oder einem Substituenten oder einer zugänglichen Position an dem indenokondensierten Naphthopyran bildet und ein zweiter Polyolsauerstoff dieses Polyolrests eine Bindung mit -E- oder -J bildet, oder
(c) eine Kombination davon ist, wobei der erste Polyolsauerstoff des Polyolrests eine Bindung mit einer Gruppe $-[(OC_2H_4)_x(OC_3H_6)_y(OC_4H_8)_z]-$ bildet und der zweite Polyolsauerstoff eine Bindung mit -E- oder-J bildet, und

(v) jedes -J unabhängig voneinander:

(a) eine Gruppe -K ist, worin -K gleich $-CH_2COOH$, $-CH(CH_3)COOH$, $-C(O)(CH_2)_wCOOH$, $-C_6H_4SO_3H$,-$C_5H_{10}SO_3H$, $-C_4H_8SO_3H$, $-C_3H_6SO_3H$, $-C_2H_4SO_3H$ oder $-SO_3H$ ist, worin w von 1 bis 18 reicht,
(b) Wasserstoff ist, vorausgesetzt, dass wenn -J gleich Wasserstoff ist, -J an ein Sauerstoff von -D- oder -G- oder ein Stickstoff von -D- gebunden ist, oder
(c) eine Gruppe -L oder ein Rest davon ist, worin -L gleich Acryl, Methacryl, Crotyl, 2-(Methacryloxy)ethylcarbamyl, 2-(Methacryloxy)ethoxycarbonyl, 4-Vinylphenyl, Vinyl, 1-Chlorvinyl oder Epoxy ist.

**4.** Photochromes Material nach einem der Ansprüche 1 bis 3, wobei die Gruppe, die das konjugierte π-System des indenokondensierten Naphthopyrans erweitert und an dessen 11-Position gebunden ist, ein substituiertes oder unsubstituiertes Aryl, ein substituiertes oder unsubstituiertes Heteroaryl oder eine Gruppe, wiedergegeben durch -X=Y oder -X'≡ Y' , ist, worin:

(i) X gleich $-CR^1$, -N, -NO, $-SR^1$, $-S(=O)R^1$ oder $-P(=O)R^1$ ist, worin $R^1$ Amino, Dialkylamino, Diarylamino, Acyloxy, Acylamino, ein substituiertes oder unsubstituiertes $C_1$- bis $C_{20}$-Alkyl, ein substituiertes oder unsubstituiertes $C_2$- bis $C_{20}$-Alkenyl, ein substituiertes oder unsubstituiertes $C_2$- bis $C_{20}$-Alkinyl, Halogen, Wasserstoff, Hydroxy, Sauerstoff, ein Polyolrest, ein substituiertes oder unsubstituiertes Phenoxy, ein substituiertes oder

unsubstituiertes Benzyloxy, ein substituiertes oder unsubstituiertes Alkoxy, ein substituiertes oder unsubstituiertes Oxyalkoxy, Alkylamino, Mercapto, Alkylthio, ein substituiertes oder unsubstituiertes Aryl, ein substituiertes oder unsubstituiertes Heteroaryl, eine substituierte oder unsubstituierte heterocyclische Gruppe, ein reaktiver Substituent oder ein kompatibilisierender Substituent wie in Anspruch 3 definiert oder ein photochromes Material ist, vorausgesetzt dass:

(a) wenn X gleich $-CR^1$ oder -N ist, Y gleich $C(R^2)_2$, $NR^2$, O oder S ist, worin jedes $R^2$ unabhängig voneinander bei jedem Auftreten ausgewählt ist aus Amino, Dialkylamino, Diarylamino, Acyloxy, Acylamino, einem substituierten oder unsubstituierten $C_1$- bis $C_{20}$-Alkyl, einem substituierten oder unsubstituierten $C_2$- bis $C_{20}$-Alkenyl, einem substituierten oder unsubstituierten $C_2$- bis $C_{20}$-Alkinyl, Halogen, Wasserstoff, Hydroxy, Sauerstoff, einem Polyolrest, einem substituierten oder unsubstituierten Phenoxy, einem substituierten oder unsubstituierten Benzyloxy, einem substituierten oder unsubstituierten Alkoxy, einem substituierten oder unsubstituierten Oxyalkoxy, Alkylamino, Mercapto, Alkylthio, einem substituierten oder unsubstituierten Aryl, einem substituierten oder unsubstituierten Heteroaryl, einer substituierten oder unsubstituierten heterocyclischen Gruppe, einem reaktiven Substituenten oder einem kompatibilisierenden Substituenten wie in Anspruch 3 definiert und einem photochromen Material, und
(b) wenn X gleich -NO, $-SR^1$, $-S(=O)R^1$ oder $-P(=O)R^1$ ist, Y gleich O ist und

(ii) X' gleich -C oder $-N^+$ ist und Y' gleich $CR^3$ oder N ist, worin $R^3$ gleich Amino, Dialkylamino, Diarylamino, Acyloxy, Acylamino, ein substituiertes oder unsubstituiertes $C_1$- bis $C_{20}$-Alkyl, ein substituiertes oder unsubstituiertes $C_2$- bis $C_{20}$-Alkenyl, ein substituiertes oder unsubstituiertes $C_2$- bis $C_{20}$-Alkinyl, Halogen, Wasserstoff, Hydroxy, Sauerstoff, ein Polyolrest, ein substituiertes oder unsubstituiertes Phenoxy, ein substituiertes oder unsubstituiertes Benzyloxy, ein substituiertes oder unsubstituiertes Alkoxy, ein substituiertes oder unsubstituiertes Oxyalkoxy, Alkylamino, Mercapto, Alkylthio, ein substituiertes oder unsubstituiertes Aryl, ein substituiertes oder unsubstituiertes Heteroaryl, eine substituierte oder unsubstituierte heterocyclische Gruppe, ein reaktiver Substituent oder ein kompatibilisierender Substituent wie in Anspruch 3 definiert oder ein photochromes Material ist, oder die Gruppe, die das konjugierte $\pi$-System des indenokondensierten Naphthopyrans erweitert und an die 11-Position des indenokondensierten Naphthopyrans gebunden ist, zusammen mit einer Gruppe, die an die 12-Position des indenokondensierten Naphthopyrans gebunden ist, oder zusammen mit einer Gruppe, die an die 10-Position des indenokondensierten Naphthopyrans gebunden ist, eine kondensierte Gruppe bildet, wobei diese kondensierte Gruppe Indeno, Dihydronaphthalin, Indol, Benzofuran, Benzopyran oder Thianaphthin ist.

**5.** Photochromes Material nach Anspruch 4, insofern Anspruch 4 auf Anspruch 1 zurückbezogen ist, worin die Gruppe, die das konjugierte $\pi$-System des indenokondensierten Naphthopyrans erweitert, ein substituiertes oder unsubstituiertes $C_2$- bis $C_{20}$-Alkenyl, ein substituiertes oder unsubstituiertes $C_2$- bis $C_{20}$-Alkinyl, ein substituiertes oder unsubstituiertes Aryl, ein substituiertes oder unsubstituiertes Heteroaryl, $-C(=O)R^1$ oder -N(=Y) oder $-N^+(\equiv Y')$ ist, worin Y gleich $C(R^2)_2$, $NR^2$, O oder S ist und Y' gleich $CR^3$ oder N ist.

**6.** Photochromes Material nach Anspruch 5, wobei die Gruppe, die das konjugierte $\pi$-System des indenokondensierten Naphthopyrans erweitert und an dessen 11-Position gebunden ist, eine Arylgruppe oder eine Heteroarylgruppe ist, die unsubstituiert ist oder mit wenigstens einem substituierten oder unsubstituierten Alkyl, einem substituierten oder unsubstituierten Alkoxy, einem substituierten oder unsubstituierten Oxyalkoxy, Amid, einem substituierten oder unsubstituierten Amino, einem substituierten oder unsubstituierten Aryl, einem substituierten oder unsubstituierten Heteroaryl, Azid, Carbonyl, Carboxy, Ester, Ether, Halogen, Hydroxy, einem Polyolrest, einem substituierten oder unsubstituierten Phenoxy, einem substituierten oder unsubstituierten Benzyloxy, Cyano, Nitro, Sulfonyl, Thiol, einer substituierten oder unsubstituierten heterocyclischen Gruppe, einem reaktiven Substituenten oder einem kompatibilisierenden Substituenten wie in Anspruch 3 definiert oder einem photochromen Material substituiert ist, vorausgesetzt, dass wenn die Arylgruppe oder die Heteroarylgruppe mehr als einen Substituenten enthält, jeder Substituent unabhängig voneinander ausgewählt werden kann.

**7.** Photochromes Material nach Anspruch 5, wobei die Gruppe, die das konjugierte $\pi$-system des indenokondensierten Naphthopyrans erweitert und an dessen 11-Position gebunden ist, gleich $-C(=O)R^1$ ist, worin $R^1$ gleich Acylamino, Acyloxy, ein substituiertes oder unsubstituiertes $C_1$- bis $C_{20}$-Alkyl, ein substituiertes oder unsubstituiertes Alkoxy, ein substituiertes oder unsubstituiertes Oxyalkoxy, Amino, Dialkylamino, Diarylamino, ein substituiertes oder unsubstituiertes Aryl, ein substituiertes oder unsubstituiertes Heteroaryl, eine substituierte oder unsubstituierte heterocyclische Gruppe, Halogen, Wasserstoff, Hydroxy, Sauerstoff, ein Polyolrest, ein substituiertes oder unsubstituiertes Phenoxy, ein substituiertes oder unsubstituiertes Benzyloxy, ein reaktiver Substituent wie in Anspruch 3

definiert oder ein photochromes Material ist.

**8.** Photochromes Material nach Anspruch 3, insofern Anspruch 3 auf Anspruch 1 zurückbezogen ist, wobei das photochrome Material ein Indeno[2',3':3,4]naphtho[1,2-b]pyran enthält und wenigstens eine der 6-Position, der 7-Position, der 13-Position, der 3-Position und der Gruppe, die das konjugierte $\pi$-System des Indeno[2',3':3,4]naphtho [1,2-b]pyrans erweitert und an dessen 11-Position gebunden ist, einen reaktiven Substituenten wie in Anspruch 3 definiert enthält.

**9.** Photochromes Material nach Anspruch 1, wobei das indenokondensierte Naphthopyran frei von spirocyclischen Gruppen an der 13-Position des indenokondensierten Naphthopyrans ist.

**10.** Photochromes Material nach Anspruch 1, wobei das indenokondensierte Naphthopyran ein Indeno[2',3':3,4]naphtho [1,2-b]pyran ist und worin:

(i) die 6-Position des Indeno[2',3':3,4]naphtho[1,2-b]pyrans mit einer stickstoffhaltigen Gruppe oder einer sauerstoffhaltigen Gruppe substituiert ist,
(ii) die 7-Position des Indeno[2',3':3,4]naphtho[1,2-b]pyrans mit einer stickstoffhaltigen Gruppe oder einer sauerstoffhaltigen Gruppe substituiert ist und
(iii) die 13-Position des Indeno[2',3':3,4]naphtho[1,2-b]pyrans disubstituiert ist, vorausgesetzt, dass jeder der Substituenten an der 13-Position unabhängig voneinander Wasserstoff, $C_1$- bis $C_6$-Alkyl, $C_3$- bis $C_7$-Cycloalkyl, Allyl, ein substituiertes oder unsubstituiertes Phenyl, ein substituiertes oder unsubstituiertes Benzyl, ein substituiertes oder unsubstituiertes Amino oder -C(O)$R^{30}$ ist, worin $R^{30}$ gleich Wasserstoff, Hydroxy, $C_1$- bis $C_6$-Alkyl, $C_1$- bis $C_6$-Alkoxy, ein unsubstituiertes, mono- oder disubstituiertes Phenyl oder Naphthyl, Phenoxy, ein mono- oder di($C_1$-$C_6$)alkylsubstituiertes Phenoxy oder ein mono- oder di($C_1$-$C_6$)alkoxysubstituiertes Phenoxy ist.

**11.** Photochrome Zusammensetzung, enthaltend das photochrome Material nach Anspruch 1, eingebracht in wenigstens einen Teil eines organischen Materials, wobei dieses organische Material ein polymeres Material, ein oligomeres Material, ein monomeres Material oder eine Mischung oder Kombination davon ist.

**12.** Photochrome Zusammensetzung nach Anspruch 11, wobei das organische Material ein polymeres Material ist und dieses polymere Material ein Copolymer aus Ethylen und Vinylacetat, ein Copolymer aus Ethylen und Vinylalkohol, ein Copolymer aus Ethylen, Vinylacetat und Vinylalkohol, Celluloseacetatbutyrat, Poly(urethan), Poly(acrylat), Poly (methacrylat), Epoxy, ein aminoplastfunktionelles Polymer, Poly(anhydrid), Poly(harnstoffurethan), ein N-alkoxymethyl(meth)acrylamidfunktionelles Polymer, Poly(siloxan), Poly(silan) oder eine Mischung oder Kombination davon ist.

**13.** Photochrome Zusammensetzung nach Anspruch 11, wobei die photochrome Zusammensetzung wenigstens eines aus einem komplementären photochromen Material, einem Photoinitiator, einem thermischen Initiator, einem Polymerisationsinhibitor, einem Lösungsmittel, einem Lichtstabilisator, einem Wärmestabilisator, einem Formtrennmittel, einem Rheologiekontrollmittel, einem Verlaufmittel, einem Radikalfänger und einem Haftvermittler enthält.

**14.** Photochrome Zusammensetzung nach Anspruch 11, wobei die photochrome Zusammensetzung eine Beschichtungszusammensetzung ist.

**15.** Photochromer Gegenstand, enthaltend ein Substrat und ein photochromes Material gemäß Anspruch 1, das mit wenigstens einem Teil des Substrats verbunden ist.

**16.** Photochromer Gegenstand nach Anspruch 15, wobei der photochrome Gegenstand ein optisches Element ist, wobei dieses optische Element wenigstens eines aus einem ophthalmischen Element, einem Anzeigeelement, einem Fenster, einem Spiegel und einem Flüssigkristallzellenelement ist.

**17.** Photochromer Gegenstand nach Anspruch 16, wobei das optische Element ein ophthalmisches Element ist und dieses ophthalmische Element wenigstens eines aus einer Korrekturlinse, einer nicht-korrigierenden Linse, einer Vergrößerungslinse, einer Schutzlinse, ein Visier, Brillen oder eine Linse für ein optisches Instrument ist.

**18.** Photochromer Gegenstand nach Anspruch 15, wobei das Substrat ein polymeres Material enthält und das photochrome Material in wenigstens einen Teil des polymeren Materials eingebracht ist.

**19.** Photochromer Gegenstand nach Anspruch 18, wobei das photochrome Material wenigstens eines von gemischt mit wenigstens einem Teil des polymeren Materials, gebunden an wenigstens einen Teil des polymeren Materials und imbibiert in wenigstens einen Teil des polymeren Materials ist.

**20.** Photochromer Gegenstand nach Anspruch 15, wobei der photochrome Gegenstand eine wenigstens teilweise Beschichtung aufweist, die an wenigstens einen Teil des Substrats gebunden ist, wobei die wenigstens teilweise Beschichtung das photochrome Material enthält.

**21.** Photochromer Gegenstand nach Anspruch 20, wobei das Substrat ein polymeres Material oder Glas ist.

**22.** Photochromer Gegenstand nach Anspruch 15, wobei wenigstens eine wenigstens teilweise Beschichtung oder ein wenigstens teilweiser Film an wenigstens einen Teil des Substrats gebunden ist, wobei die wenigstens eine wenigstens teilweise Beschichtung oder der wenigstens eine wenigstens teilweise Film wenigstens eins aus einer Grundierungsbeschichtung oder -film, einer Schutzbeschichtung oder -film, einer antireflektierenden Beschichtung oder Film, einer konventionellen photochromen Beschichtung oder Film und einer polarisierenden Beschichtung oder Film ist.

**23.** Photochromer Gegenstand nach Anspruch 15, wobei der photochrome Gegenstand wenigstens eins aus einem komplementären photochromen Material, einem Photoinitiator, einem thermischen Initiator, einem Polymerisationsinhibitor, einem Lösungsmittel, einem Lichtstabilisator, einem Wärmestabilisator, einem Formtrennmittel, einem Rheologiekontrollmittel, einem Verlaufmittel, einem Radikalfänger und einem Haftvermittler enthält.

**24.** Verfahren zur Herstellung eines photochrome Gegenstands, umfassend Verbinden eines photochromen Materials gemäß Anspruch 1 mit wenigstens einem Teil eines Substrats, wobei Verbinden des photochromen Materials mit wenigstens einem Teil des Sustrats wenigstens eines von Formgießen, Beschichten, Imbibieren, Laminieren und Vor-Ort-Gießen umfasst.

**25.** Photochromes Material nach Anspruch 1, wiedergegeben durch:

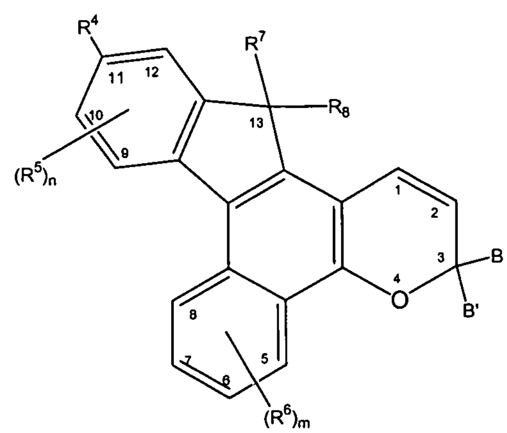

,

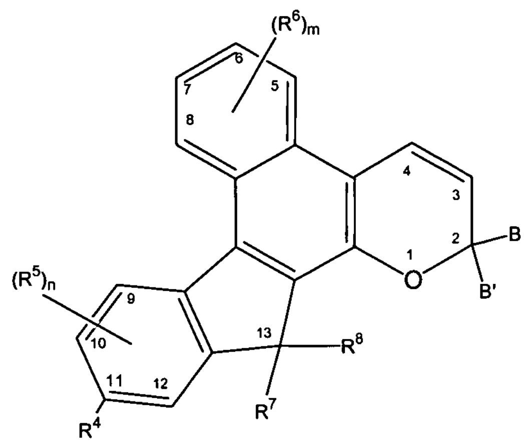

oder eine Mischung davon, worin:

(i) $R^4$ ein substituiertes oder unsubstituiertes Aryl, ein substituiertes oder unsubstituiertes Heteroaryl oder eine Gruppe, wiedergegeben durch -X=Y oder -X'≡ Y' ist, worin:

(a) X gleich $-CR^1$, -N, -NO, $-SR^1$, $-S(=O)R^1$ oder $-P(=O)R^1$ ist, worin $R^1$ Amino, Dialkylamino, Diarylamino, Acyloxy, Acylamino, ein substituiertes oder unsubstituiertes $C_1$- bis $C_{20}$-Alkyl, ein substituiertes oder unsubstituiertes $C_2$- bis $C_{20}$-Alkenyl, ein substituiertes oder unsubstituiertes $C_2$- bis $C_{20}$-Alkinyl, Halogen, Wasserstoff, Hydroxy, Sauerstoff, ein Polyolrest, ein substituiertes oder unsubstituiertes Phenoxy, ein substituiertes oder unsubstituiertes Benzyloxy, ein substituiertes oder unsubstituiertes Alkoxy, ein substituiertes oder unsubstituiertes Oxyalkoxy, Alkylamino, Mercapto, Alkylthio, ein substituiertes oder unsubstituiertes Aryl, ein substituiertes oder unsubstituiertes Heteroaryl, eine substituierte oder unsubstituierte heterocyclische Gruppe, ein reaktiver Substituent oder ein kompatibilisierender Substituent wie in Anspruch 3 definiert

oder ein photochromes Material ist, vorausgesetzt dass:

(1) wenn X gleich $-CR^1$ oder -N ist, Y gleich $C(R^2)_2$, $NR^2$, O oder S ist, worin jedes $R^2$ unabhängig voneinander bei jedem Auftreten ausgewählt ist aus Amino, Dialkylamino, Diarylamino, Acyloxy, Acylamino, einem substituierten oder unsubstituierten $C_1$- bis $C_{20}$-Alkyl, einem substituierten oder unsubstituierten $C_2$- bis $C_{20}$-Alkenyl, einem substituierten oder unsubstituierten $C_2$- bis $C_{20}$-Alkinyl, Halogen, Wasserstoff, Hydroxy, Sauerstoff, einem Polyolrest, einem substituierten oder unsubstituierten Phenoxy, einem substituierten oder unsubstituierten Benzyloxy, einem substituierten oder unsubstituierten Alkoxy, einem substituierten oder unsubstituierten Oxyalkoxy, Alkylamino, Mercapto, Alkylthio, einem substituierten oder unsubstituierten Aryl, einem substituierten oder unsubstituierten Heteroaryl, einer substituierten oder unsubstituierten heterocyclischen Gruppe, einem reaktiven Substituenten oder einem kompatibilisierenden Substituenten wie in Anspruch 3 definiert und einem photochromen Material, und

(2) wenn X gleich -NO, $-SR^1$, $-S(=O)R^1$ oder $-P(=O)R^1$ ist, Y gleich O ist und

(b) X' gleich -C oder $-N^+$ ist und Y' gleich $CR^3$ oder N ist, worin $R^3$ gleich Amino, Dialkylamino, Diarylamino, Acyloxy, Acylamino, ein substituiertes oder unsubstituiertes $C_1$- bis $C_{20}$-Alkyl, ein substituiertes oder unsubstituiertes $C_2$- bis $C_{20}$-Alkenyl, ein substituiertes oder unsubstituiertes $C_2$- bis $C_{20}$-Alkinyl, Halogen, Wasserstoff, Hydroxy, Sauerstoff, ein Polyolrest, ein substituiertes oder unsubstituiertes Phenoxy, ein substituiertes oder unsubstituiertes Benzyloxy, ein substituiertes oder unsubstituiertes Alkoxy, ein substituiertes oder unsubstituiertes Oxyalkoxy, Alkylamino, Mercapto, Alkylthio, ein substituiertes oder unsubstituiertes Aryl, ein substituiertes oder unsubstituiertes Heteroaryl, eine substituierte oder unsubstituierte heterocyclische Gruppe, ein reaktiver Substituent oder ein kompatibilisierender Substituent wie in Anspruch 3 definiert oder ein photochromes Material ist, oder

$R^4$ zusammen mit einer $R^5$-Gruppe, die an die 12-Position des indenokondensierten Naphthopyrans gebunden ist, oder zusammen mit einer $R^5$-Gruppe, die an die 10-Position des indenokondensierten Naphthopyrans gebunden ist, eine kondensierte Gruppe bildet, wobei diese kondensierte Gruppe Indeno, Dihydronaphthalin, Indol, Benzofuran, Benzopyran oder Thianaphthin ist,
(ii) n von 0 bis 3 reicht,
(iii) m von 0 bis 4 reicht,
(iv) jedes $R^5$ und $R^6$ unabhängig voneinander bei jedem Auftreten ausgewählt sind aus: einem reaktiven Substituenten oder einem kompatibilisierenden Substituenten wie in Anspruch 3 definiert, Wasserstoff, $C_1$- bis $C_6$-Alkyl, Chlor, Fluor, $C_3$- bis $C_7$-Cycloalkyl, einem substituierten oder unsubstituierten Phenyl, wobei diese Substituenten des Phenyls gleich $C_1$- bis $C_6$-Alkyl oder $C_1$- bis $C_6$-Alkoxy sind, $-OR^{10}$ oder $-OC(=O)R^{10}$, worin $R^{10}$ gleich S, Wasserstoff, Amin, $C_1$- bis $C_6$-Alkyl, Phenyl($C_1$-$C_3$)alkyl, mono($C_1$-$C_6$)alkylsubstituiertes Phenyl ($C_1$-$C_3$)alkyl, mono($C_1$-$C_6$)alkoxysubstituiertes Phenyl($C_1$-$C_3$)alkyl, ($C_1$-$C_6$)Alkoxy($C_2$-$C_4$)alkyl, $C_3$- bis $C_7$-Cycloalkyl oder mono($C_1$-$C_4$)alkylsubstituiertes $C_3$- bis $C_7$-Cycloalkyl ist, einem monosubstituierten Phenyl, wobei dieses Phenyl einen Substituenten, angeordnet in der para-Position aufweist, wobei der Substituent ein Dicarbonsäurerest oder Derivat davon, ein Diaminrest oder Derivat davon, ein Aminoalkoholrest oder Derivat davon, ein Polyolrest oder Derivat davon, $-(CH_2)-$, $-(CH_2)_t-$ oder $-[O-(CH_2)_t]_k-$ ist, worin t von 2 bis 6 reicht und k von 1 bis 50 reicht und wobei der Substituent mit einer Arylgruppe eines anderen photochromen Material verbunden ist, $-N(R^{11})R^{12}$, worin $R^{11}$ und $R^{12}$ jeweils unabhängig voneinander Wasserstoff, $C_1$- bis $C_8$-Alkyl, Phenyl, Naphthyl, Furanyl, Benzofuran-2-yl, Benzofuran-3-yl, Thienyl, Benzothien-2-yl, Benzothien-3-yl, Dibenzofuranyl, Dibenzothienyl, Benzopyridyl und Fluorenyl, $C_1$- bis $C_8$-Alkylaryl, $C_3$- bis $C_{20}$-Cycloalkyl, $C_4$- bis $C_{20}$-Bicycloalkyl, $C_5$- bis $C_{20}$-Tricycloalkyl oder $C_1$-bis $C_{20}$-Alkoxyalkyl sind oder $R^{11}$ und $R^{12}$ mit dem Stickstoffatom zusammenkommen, um ein $C_3$- bis $C_{20}$ heterobicyclischen Alkylring oder einen $C_4$- bis $C_{20}$ heterotricyclischen Alkylring zu bilden, einen stickstofhaltigen Ring, der wiedergegeben ist durch:

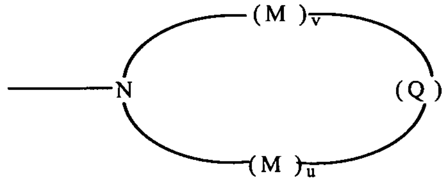

worin jedes -M- unabhängig voneinander bei jedem Auftreten ausgewählt ist aus $-CH_2-$, $-CH(R^{13})-$, $-C(R^{13})_2-$,

-CH(aryl)-, $-C(aryl)_2-$ und $-C(R^{13})(aryl)-$ und -Q- gleich -M-, -O-, -S-, -S(O)-, $-SO_2-$, -NH-, $-N(R^{13})-$ oder -N(aryl)- ist, worin jedes $R^{13}$ unabhängig $C_1$- bis $C_6$-Alkyl ist, jedes (Aryl) unabhängig voneinander Phenyl oder Naphthyl ist, u von 1 bis 3 reicht und v von 0 bis 3 reicht, unter der Voraussetzung, dass wenn v gleich 0 ist, -Q- gleich -M- ist, einer Gruppe, die wiedergegeben ist durch:

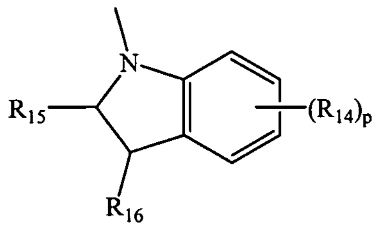

oder

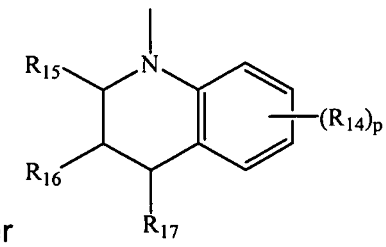

worin jedes $R^{15}$, $R^{16}$ und $R^{17}$ unabhängig voneinander Wasserstoff, $C_1$- bis $C_6$-Alkyl, Phenyl oder Naphthyl ist oder $R^{15}$ und $R^{16}$ zusammen einen Ring mit 5 bis 8 Kohlenstoffatomen bilden, jedes $R^{14}$ unabhängig voneinander $C_1$- bis $C_6$-Alkyl, $C_1$- bis $C_6$-Alkoxy, Fluor oder Chlor ist und p von 0 bis 3 reicht, und einem substituierten oder unsubstituierten $C_4$- bis $C_{18}$-spirobicyclischem Amin oder einem substituierten oder unsubstituierten $C_4$- bis $C_{18}$-spirotricyclischen Amin, wobei diese Substituenten unabhängig voneinander Aryl, $C_1$- bis $C_6$-Alkyl, $C_1$- bis $C_6$-Alkoxy oder Phenyl($C_1$-$C_6$)alkyl sind, oder eine $R^6$-Gruppe in der 6-Position und eine $R^6$-Gruppe in der 7-Position zusammen eine Gruppe bilden, die wiedergegeben ist durch:

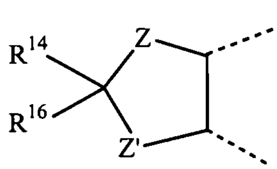

oder

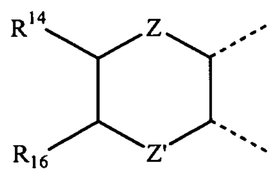

worin jedes Z und Z' unabhängig voneinander Sauerstoff oder die Gruppe $-NR^{11}-$ ist, worin $R^{11}$, $R^{14}$ und $R^{16}$ wie oben festgelegt sind,

(v) $R^7$ und $R^8$ jeweils unabhängig voneinander sind: ein reaktiver Substituent oder ein kompatibilisierender Substituent wie in Anspruch 3 definiert, Wasserstoff, Hydroxy, $C_1$- bis $C_6$-Alkyl, $C_3$- bis $C_7$-Cycloalkyl , Allyl, ein substituiertes oder unsubstituiertes Phenyl oder Benzyl, worin jeder dieser Substituenten des Phenyls und Benzyls unabhängig voneinder $C_1$- bis $C_6$-Alkyl oder $C_1$- bis $C_6$-Alkoxy ist, Chlor, Fluor, ein substituiertes oder unsubstituiertes Amino, $-C(O)R^9$, worin $R^9$ Wasserstoff, Hydroxy, $C_1$- bis $C_6$-Alkyl, $C_1$- bis $C_6$-Alkoxy, ein unsubstituiertes, mono- oder disubstituiertes Phenyl oder Naphthyl ist, worin jeder dieser Substituenten unabhängig voneinander $C_1$- bis $C_6$-Alkyl oder $C_1$- bis $C_6$-Alkoxy, Phenoxy, mono- oder di($C_1$-$C_6$)alkylsubstituiertes Phenoxy, mono- oder di($C_1$-$C_6$)alkoxysubstituiertes Phenoxy, Amino, Mono- oder Di($C_1$-$C_6$)alkylamino, Phenylamino, mono- oder di($C_1$-$C_6$)alkylsubstituiertes Phenylamino oder mono- oder di($C_1$-$C_6$)alkoxysubstituiertes Phenylamino ist, $-OR^{18}$, worin $R^{18}$ gleich $C_1$- bis $C_6$-Alkyl, Phenyl($C_1$-$C_3$)alkyl, mono($C_1$-$C_6$)alkylsubstituiertes Phenyl($C_1$-$C_3$)alkyl, mono($C_1$-$C_6$)alkoxysubstituiertes Phenyl($C_1$-$C_3$)alkyl, $C_1$- bis $C_6$-Alkoxy($C_2$-$C_4$)alkyl, $C_3$- bis $C_7$-Cycloalkyl, mono($C_1$-$C_4$)alkylsubstituiertes $C_3$- bis $C_7$-Cycloalkyl, $C_1$- bis $C_6$-Chloralkyl, $C_1$- bis $C_6$-Fluoralkyl, Allyl oder $-CH(R^{19})T$ ist, worin $R^{19}$ gleich Wasserstoff oder $C_1$- bis $C_3$-Alkyl ist, T gleich CN, $CF_3$ oder $COOR^{20}$ ist, worin $R^{20}$ gleich Wasserstoff oder $C_1$- bis $C_3$-Alkyl ist oder worin $R^{18}$ gleich -C(=O)U ist, worin U gleich Wasserstoff, $C_1$- bis $C_6$-Alkyl, $C_1$-bis $C_6$-Alkoxy, ein unsubstituiertes, mono- oder disubstituiertes Phenyl oder Naphthyl ist, worin jeder dieser Substituenten unabhängig voneinander $C_1$- bis $C_6$-Alkyl oder $C_1$- bis $C_6$-Alkoxy, Phenoxy, mono- oder di($C_1$-$C_6$)alkylsubstituiertes Phenoxy, mono- oder di($C_1$-$C_6$)alkoxysubstituiertes Phenoxy, Amino, Mono- oder Di($C_1$-$C_6$)alkylamino, Phenylamino, mono- oder di($C_1$-$C_6$)alkylsubstituiertes Phenylamino oder mono- oder di($C_1$-$C_6$)alkoxysubstituiertes Phenylamino ist, und ein monosubstituiertes Phenyl, wobei dieses Phenyl einen Substituenten, angeordnet in der para-Position, aufweist, wobei der Substituent ein Dicarbonsäurerest oder Derivat davon, ein Diaminrest oder Derivat davon, ein Aminoalkoholrest oder Derivat davon, ein Polyolrest oder Derivat davon, $-(CH_2)-$, $-(CH_2)_t-$ oder $-[O-(CH_2)_t]_k-$ ist, worin t von 2 bis 6 reicht, und k von 1 bis 50 reicht, und wobei der Substituent mit einer Arylgruppe eines anderen photochromen Material verbunden ist, oder $R^7$ und $R^8$ bilden zusammen eine Oxogruppe, eine spirocarbocyclische Gruppe mit 3 bis 6 Kohlenstoffatomen, unter der Voraussetzung, dass die spirocarbocyclische Gruppe nicht Norbornyl ist, oder

eine spiroheterocyclische Gruppe mit 1 bis 2 Sauerstoffatomen und 3 bis 6 Kohlenstoffatomen einschließlich des Spirokohlenstoffatoms, wobei diese spirocarbocyclischen und spiroheterocyclischen Gruppen mit 0, 1 oder 2 Benzolringen anelliert sind, und

(vi) B und B' jeweils unabhängig voneinander sind:

eine Arylgruppe, die mit einem reaktiven Substituenten oder einem kompatibilisierenden Substituenten wie in Anspruch 3 definiert monosubstituiert ist, eine unsubstituierte, mono-, di- oder trisubstituierte Arylgruppe, 9-Julolidinyl, eine unsubstituierte, mono- oder disubstituierte heteroaromatische Gruppe, ausgewählt aus Pyridyl, Furanyl, Benzofuran-2-yl, Benzofuran-3-yl, Thienyl, Benzothien-2-yl, Benzothien-3-yl, Dibenzofuranyl, Dibenzothienyl, Carbazoyl, Benzopyridyl, Indolinyl und Fluorenyl, worin die Substituenten des Aryls und der heteroaromatischen Gruppe unabhängig voneinander sind: Hydroxy, Aryl, Mono- oder Di($C_1$-$C_{12}$) alkoxyaryl, Mono- oder Di($C_1$-$C_{12}$)alkylaryl, Halogenaryl, $C_3$- bis $C_7$-Cycloalkylaryl, $C_3$- bis $C_7$-Cycloalkyl, $C_3$- bis $C_7$-Cycloalkyloxy, $C_3$- bis $C_7$-Cycloalkyloxy($C_1$-$C_{12}$)alkyl, $C_3$- bis $C_7$-Cycloalkyloxy($C_1$-$C_{12}$)alkoxy, Aryl($C_1$-$C_{12}$)alkyl, Aryl($C_1$-$C_{12}$)alkoxy, Aryloxy, Aryloxy($C_1$-$C_{12}$)alkyl, Aryloxy($C_1$-$C_{12}$)alkoxy, Mono- oder Di($C_1$-$C_{12}$)alkylaryl($C_1$-$C_{12}$)alkyl, Mono- oder Di($C_1$-$C_{12}$)alkoxyaryl($C_1$-$C_{12}$)alkyl, Mono- oder Di($C_1$-$C_{12}$) alkylaryl($C_1$-$C_{12}$)alkoxy, Mono- oder Di($C_1$-$C_{12}$)alkoxyaryl($C_1$-$C_{12}$)alkoxy, Amino, Mono- oder Di($C_1$-$C_{12}$) alkylamino, Diarylamino, Piperazino, N-($C_1$-$C_{12}$)Alkylpiperazino, N-Arylpiperazino, Aziridino, Indolino, Piperidino, Morpholino, Thiomorpholino, Tetrahydrochinolino, Tetrahydroisochinolino, Pyrrolidyl, $C_1$- bis $C_{12}$-Alkyl, $C_1$- bis $C_{12}$-Halogenalkyl, $C_1$- bis $C_{12}$-Alkoxy, Mono($C_1$-$C_{12}$)alkoxy($C_1$-$C_{12}$)alkyl, Acryloxy, Methacryloxy, Halogen oder -C(=O)$R^{21}$, worin $R^{21}$ gleich -O$R^{22}$, -N($R^{23}$)$R^{24}$, Piperidino oder Morpholino ist, worin $R^{22}$ gleich Allyl, $C_1$- bis $C_6$-Alkyl, Phenyl, mono($C_1$-$C_6$)alkylsubstituiertes Phenyl, mono($C_1$-$C_6$)alkoxysubstituiertes Phenyl, Phenyl($C_1$-$C_3$)alkyl, mono($C_1$-$C_6$)alkylsubstituiertes Phenyl($C_1$-$C_3$)alkyl, mono($C_1$-$C_6$) alkoxysubstituiertes Phenyl($C_1$-$C_3$)alkyl, $C_1$- bis $C_6$-Alkoxy($C_2$-$C_4$)alkyl oder $C_1$- bis $C_6$-Halogenalkyl ist und $R^{23}$ und $R^{24}$ jeweils unabhängig voneinander $C_1$- bis $C_6$-Alkyl, $C_5$- bis $C_7$-Cycloalkyl oder ein substituiertes oder unsubstituiertes Phenyl sind, wobei die Substituenten des Phenyls unabhängig voneinander $C_1$- bis $C_6$-Alkyl oder $C_1$- bis $C_6$-Alkoxy sind,

eine unsubstituierte oder monosubstituierte Gruppe, ausgewählt aus Pyrazolyl, Imidazolyl, Pyrazolinyl, Imidazolinyl, Pyrrolinyl, Phenothiazinyl, Phenoxazinyl, Phenazinyl und Acridinyl, wobei die Substituenten $C_1$- bis $C_{12}$-Alkyl, $C_1$- bis $C_{12}$-Alkoxy, Phenyl oder Halogen sind, ein monosubstituiertes Phenyl, wobei dieses Phenyl einen Substituenten, angeordnet in der para-Position, aufweist, und dieser Substituent ein Dicarbonsäurerest oder Derivat davon, ein Diaminrest oder Derivat davon, ein Aminoalkoholrest oder Derivat davon, ein Polyolrest oder Derivat davon, -($CH_2$)-, -$(CH_2)_t$- oder -$[O-(CH_2)_t]_k$- ist, worin t von 2 bis 6 reicht und k von 1 bis 50 reicht, und wobei der Substituent mit einer Arylgruppe eines anderen photochromen Materials verbunden ist, eine Gruppe, die wiedergegeben ist durch:

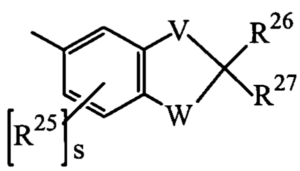

oder

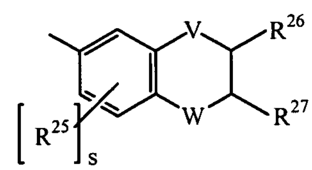

worin V gleich -$CH_2$- oder -O- ist, W gleich Sauerstoff oder substituierter Stickstoff ist, unter der Voraussetzung, dass wenn W substituierter Stickstoff ist, V gleich -$CH_2$- ist, die substituierten Stickstoffsubstituenten Wasserstoff, $C_1$- bis $C_{12}$-Alkyl oder $C_1$- bis $C_{12}$-Acyl sind, jedes $R^{25}$ unabhängig voneinander $C_1$- bis $C_{12}$-Alkyl, $C_1$- bis $C_{12}$-Alkoxy, Hydroxy oder Halogen ist, $R^{26}$ und $R^{27}$ jeweils unabhängig voneinander Wasserstoff oder $C_1$- bis $C_{12}$-Alkyl sind und s von 0 bis 2 reicht, oder eine Gruppe, die wiedergegeben ist durch:

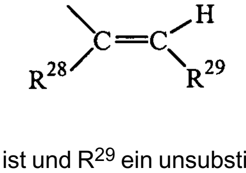

worin $R^{28}$ Wasserstoff oder $C_1$- bis $C_{12}$-Alkyl ist und $R^{29}$ ein unsubstituiertes, mono- oder disubstituiertes Naphthyl, Phenyl, Furanyl oder Thienyl ist, wobei diese Substituenten $C_1$- bis $C_{12}$-Alkyl, $C_1$- bis $C_{12}$-Alkoxy oder Halogen sind, oder B und B' zusammengenommen ein Fluoren-9-yliden oder mono- oder disubstituiertes Fluoren-9-yliden

bilden, wobei jeder der Substituenten dieses Fluoren-9-ylidens unabhänging voneinander $C_1$- bis $C_{12}$-Alkyl, $C_1$- bis $C_{12}$-Alkoxy oder Halogen ist.

**26.** Photochromes Material nach Anspruch 25, wobei die wenigstens eine von einer $R^6$-Gruppe an der 6-Position, einer $R^6$-Gruppe an der 7-Position, B, B', $R^7$, $R^8$ und $R^4$ einen reaktiven Substituenten wie in Anspruch 3 definiert aufweisen.

**27.** Photochromes Material nach Anspruch 3, wobei das indenokondensierte Naphthopyran ein Indeno[2',3':3,4]naphtho [1,2-b]pyran ist und worin:

(i) jedweils eine $R^6$-Gruppe an der 7-Position und eine $R^6$-Gruppe an der 6-Position des Indeno[2',3':3,4]naphtho [1,2-b]pyrans unabhängig voneinander $-OR^{10}$ sind, worin $R^{10}$ gleich $C_1$- bis $C_6$-Alkyl, ein substituiertes oder unsubstituiertes Phenyl, wobei diese Substituenten des Phenyls $C_1$- bis $C_6$-Alkyl oder $C_1$- bis $C_6$-Alkoxy, Phenyl $(C_1-C_3)$alkyl, mono$(C_1-C_6)$alkylsubstituiertes Phenyl$(C_1-C_3)$alkyl, mono$(C_1-C_6)$alkoxysubstituiertes Phenyl $(C_1-C_3)$alkyl, $(C_1-C_6)$Alkoxy$(C_2-C_4)$alkyl, $C_3$- bis $C_7$-Cycloalkyl oder mono$(C_1-C_4)$alkylsubstituiertes $C_3$- bis $C_7$-Cycloalkyl sind, $-N(R^{11})R^{12}$, worin $R^{11}$ und $R^{12}$ jeweils unabhängig voneinander Wasserstoff, $C_1$- bis $C_8$-Alkyl, $C_1$- bis $C_8$-Alkylaryl, $C_3$- bis $C_{20}$-Cycloalkyl, $C_4$- bis $C_{20}$-Bicycloalkyl, $C_5$- bis $C_{20}$-Tricycloalkyl oder $C_1$- bis $C_{20}$-Alkoxyalkyl sind, wobei diese Arylgruppe gleich Phenyl oder Naphthyl ist, ein stickstoffhaltiger Ring, der wiedergegeben ist durch:

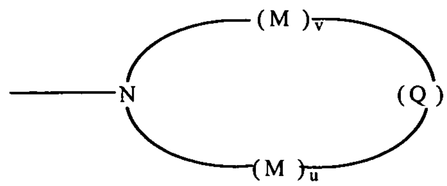

worin jedes -M- unabhängig voneinander bei jedem Auftreten ausgewählt ist aus $-CH_2-$, $-CH(R^{13})-$, $-C(R^{13})_2-$, -CH(Aryl)-, $-C(Aryl)_2-$ und $-C(R^{13})(Aryl)-$ und -Q- gleich -M-, -O-, -S-, -NH-, $-N(R^{13})-$ oder -N(Aryl)- ist, worin jedes $R^{13}$ unabhängig voneinander $C_1$- bis $C_6$-Alkyl ist, jedes (Aryl) unabhängig voneinander Phenyl oder Naphthyl ist, u von 1 bis 3 und v von 0 bis 3 reicht, unter der Voraussetzung, dass wenn v gleich 0 ist, -Q- gleich -M- ist, oder ein reaktiver Substituent oder ein kompatibilisierender Substituent wie in Anspruch 3 definiert ist, vorausgesetzt, dass der reaktive oder kompatibilisierende Substituent eine Verbindungsgruppe enthält, die einen aliphatischen Aminoalkoholrest, einen cycloaliphatischen Aminoalkoholrest, einen azacyclischen aliphatischen Alkoholrest, einen diazacyclischen aliphatischen Alkoholrest, einen Diaminrest, einen aliphatischen Diaminrest, einen cycloaliphatischen Diaminrest, einen Diazacycloalkanrest, einen azacyclischen aliphatischen Aminrest, eine Oxyalkoxygruppe, einen aliphatischen Polyolrest oder einen cycloaliphatischen Polyolrest aufweist, der eine Bindung mit dem Indeno[2',3':3,4]naphtho[1,2-b]pyran an der 6-Position oder der 7-Position bildet, oder

(ii) eine $R^6$-Gruppe in der 6-Position und eine $R^6$-Gruppe in der 7- Position des Indeno [2', 3': 3,4] naphtho [1,2-b] pyrans zusammen eine Gruppe bilden, die wiedergegeben ist durch:

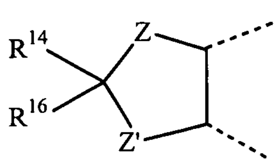

oder

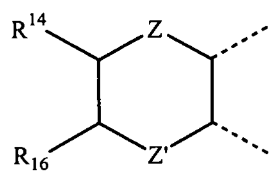

worin Z und Z' jeweils unabhängig voneinander Sauerstoff oder $-NR^{11}-$ sind, worin $R^{11}$ wie oben unter (i) festgelegt ist.

**28.** Photochromes Material nach Anspruch 3, wobei das photochrome Material ausgewählt ist aus:

(i) einem 3,3-Di(4-methoxyphenyl)-6,7-dimethoxy-11-cyano-13,13-dimethyl-3H,13H-indeno[2',3':3,4]naphtho [1,2-b]pyran;

(ii) einem 3,3-Di(4-methoxyphenyl)-6,7-dimethoxy-11-carboxy-13,13-dimethyl-3H,13H-indeno[2',3':3,4]naphtho[1,2-b]pyran;

(iii) einem 3,3-Di(4-methoxyphenyl)-6,7-dimethoxy-11-methoxycarbonyl-13,13-dimethyl-3H,13H-indeno[2',3':3,4]naphtho[1,2-b]pyran;

(iv) einem 3,3-Di(4-methoxyphenyl)-6,7-dimethoxy-11-(2-(2-hydroxyethoxy)ethoxycarbonyl-13,13-dimethyl-3H,13H-indeno[2',3':3,4]naphtho[1,2-b]pyran;

(v) einem 3,3-Di(4-methoxyphenyl)-6,7-dimethoxy-11-(4-fluorphenyl)-13,13-dimethyl-3H,13H-indeno[2',3':3,4]naphtho[1,2-b]pyran;

(vi) einem 3,3-Di(4-methoxyphenyl)-6,7-dimethoxy-11-(4-(phenyl)-phenyl)-13,13-dimethyl-3H,13H-indeno[2',3':3,4]naphtho[1,2-b]-pyran;

(vii) einem 3,3-Di(4-methoxyphenyl)-6,7-dimethoxy-11-(4-(hydroxymethyl)phenyl)-13,13-dimethyl-3H,13H-indeno[2',3':3,4]naphtho[1,2-b]pyran;

(viii) einem 3,3-Di(4-methoxyphenyl)-6,7-dimethoxy-1 1-(3-hydroxy-3-methylbutinyl)-13,13-dimethyl-3H,13H-indeno[2',3':3,4]naphtho-[1,2-b]pyran;

(ix) einem 3,3-Di(4-methoxyphenyl)-6,7-dimethoxy-11-(2-phenylethinyl)-13,13-dimethyl-3H,13H-indeno[2',3':3,4]naphtho[1,2-b]pyran;

(x) einem 3,3-Di(4-methoxyphenyl)-6,7-dimethoxy-11-phenyl-13-ethyl,13-methoxy-3H,13H-indeno[2',3':3,4]naphtho[1,2-b]pyran;

(xi) einem 3-Phenyl-3-(4-methoxyphenyl)-6,7-dimethoxy-11-(4-(2-methacryloxyethoxy)carbonylphenyl)-13,13-dimethyl-3H,13H-indeno[2',3':3,4] naphtho[1,2-b]pyran;

(xii) einem 3,3-Di(4-methoxyphenyl)-6-methoxy-7-((3-(2-methacryloxyethyl)carbamyloxymethylenpiperidino)-1-yl)-11-(4-(phenyl)phenyl)-13,13-dimethyl-3H,13H-indeno[2',3':3,4]naphtho[1,2-b]pyran;

(xiii) einem 3-Phenyl-3-(4-(2-(2-methacryloxyethyl)carbamyloxyethoxy)phenyl)-6-methoxy-11-phenyl-13,13-dimethyl-3H,13H-indeno[2',3':3,4]naphtho[1,2-b]pyran;

(xiv) einem 3-Phenyl-3-(4-methoxyphenyl)-6,7-dimethoxy-13,13-dimethyl-11-(2-(4-(3-phenyl-6,11-dimethoxy-13,13-dimethyl-3H,13H-indeno[2',3':3,4]naphtho[1,2-b]pyran-3-yl)phenoxy)ethoxycarbonyl)-3H,13H-indeno[2',3':3,4]naphtho[1,2-b]pyran;

(xv) einem 3-Phenyl-3-(4-(2-methacryloxyethyl)carbamyloxyphenyl)-6,7-dimethoxy-13,13-dimethyl-11-((1-(4-(3-phenyl-6,11-dimethoxy-13,13-dimethyl-3H,13H-indeno[2',3':3,4]naphtho[1,2-b]pyran-3-yl)phenyl)piperazino-4-yl)carbonyl)-3H,13H-indeno[2',3':3,4]-naphtho[1,2-b]pyran;

(xvi) einem 3,3-Di(4-methoxyphenyl)-11-methoxycarboxy-13,13-dimethyl-3H,13H-indeno[2',3':3,4]naphtho[1,2-b]pyran;

(xvii) einem 3-(4-Morpholinophenyl)-3-phenyl-6,7-dimethoxy-11-carboxy-13,13-dimethyl-3H,13H-indeno[2',3':3,4]naphtho[1,2-b]pyran;

(xviii) einem 3-(4-Morpholinophenyl)-3-phenyl-6,7-dimethoxy-11-methoxycarbonyl-13,13-dimethyl-3H,13H-indeno[2',3':3,4]-naphtho[1,2-b]pyran;

(xix) einem 3-(4-Morpholinophenyl)-3-(4-methoxyphenyl)-6,7-dimethoxy-11-(4-fluorophenyl)-13,13-dimethyl-3H,13H-indeno[2',3':3,4]naphtho-[1,2-b]pyran;

(xx) einem 3-(4-Fluorphenyl)-3-(4-methoxyphenyl)-6,7-dimethoxy-11-cyano-13,13-dimethyl-3H,13H-indeno[2',3':3,4]naphtho[1,2-b]pyran;

(xxi) einem 3-(4-Morpholinophenyl)-3-(4-methoxyphenyl)-11-(2-phenylethinyl)-13,13-dimethyl-3H,13H-indeno[2',3':3,4]naphtho[1,2-b]-pyran;

(xxii) einem 3,3-Di(4-methoxyphenyl)-6,7-dimethoxy-11-(4-dimethylaminophenyl)-13,13-dimethyl-3H,13H-indeno[2',3':3,4]naphtho[1,2-b]-pyran;

(xxiii) einem 3,3-Di(4-methoxyphenyl)-6,7-dimethoxy-11-(4-methoxyphenyl)-13,13-dimethyl-3H,13H-indeno[2',3':3,4]-naphtho[1,2-b]pyran;

(xxiv) einem 3,3-Di(4-methoxyphenyl)-6-methoxy-7-morpholino-11-phenyl-13-butyl-13-(2-(2-hydroxyethoxy)ethoxy)-3H,13H-indeno[2',3':3,4]-naphtho[1,2-b]pyran;

(xxv) einem 3-(4-Fluorphenyl)-3-(4-methoxyphenyl)-6-methoxy-7-morpholino-11-phenyl-13-butyl-13-(2-(2-hydroxyethoxy)ethoxy)-3H,13H-indeno[2',3':3,4]naphtho[1,2-b]pyran;

(xxvi) einem 3,3-Di(4-Fluorphenyl)-11-cyano-13,13-dimethyl-3H,13H-indeno[2',3':3,4]naphtho[1,2-b]pyran;

(xxvii) einem 3-(4-Morpholinophenyl)-3-phenyl-6-methoxy-7-(3-(2-methacryloxyethyl)carbamyloxymethylenpiperidino-1-yl)-11-phenyl-13,13-dimethyl-3H,13H-indeno[2',3':3,4]naphtho[1,2-b]pyran;

(xxviii) einem 3-(4-(2-(2-Methacryloxyethyl)carbamylethoxy)phenyl)-3-phenyl-6,7-dimethoxy-11-phenyl-13,13-dimethyl-3H,13H-indeno[2',3':3,4]naphtho[1,2-b]pyran und Mischungen davon.

## Revendications

1. Matériau photochromique comprenant :

   (i) un naphtopyrane indénocondensé ; et
   (ii) un groupe qui prolonge le système pi-conjugué du naphtopyrane indénocondensé lié à la position 11 de celui-ci, à condition que si le groupe lié à la position 11 du naphtopyrane indénocondensé et un groupe lié à la position 10 ou à la position 12 du naphtopyrane indénocondensé forment ensemble un groupe condensé, ledit groupe condensé n'est pas un groupe benzocondensé ; et

   dans lequel la position 13 du naphtopyrane indénocondensé est non substituée, monosubstituée ou disubstituée, à condition que si la position 13 du naphtopyrane indénocondensé est disubstituée, les substituants ne forment pas ensemble un groupe norbornyle.

2. Matériau photochromique selon la revendication 1, dans lequel le matériau photochromique comprend un indéno [2',3':3,4]naphto[1,2-b]pyrane, un indéno[1',2':4,3]-naphto[2,1-b]pyrane ou un mélange de ceux-ci.

3. Matériau photochromique selon la revendication 1, dans lequel le matériau photochromique comprend au moins un d'un substituant réactif et d'un substituant de compatibilisation, chacun dudit substituant réactif ou substituant de compatibilisation étant indépendamment représenté par l'un de :

   -A'-D-E-G-J ;

   -G-E-G-J ;

   -D-E-G-J ;

   -A'-D-J ;

   -D-G-J ;

   -D-J ;

   -A'-G-J ;

   -G-J ;

   et

   -A'-J ;

   où

   (i) chaque -A'- est indépendamment -O-, -C(=O)-, $-CH_2-$, -OC(=O)- ou -NHC(=O)-, à condition que si -A'- est -O-, -A'-forme au moins une liaison avec -J ;
   (ii) chaque -D- est indépendamment :

   (a) un résidu diamine ou un dérivé de celui-ci, ledit résidu diamine étant un résidu diamine aliphatique, un résidu diamine cycloaliphatique, un résidu diazacycloalcane, un résidu amine azacycloaliphatique, un résidu éther diazacouronne ou un résidu diamine aromatique, dans lequel un premier azote aminé dudit résidu diamine forme une liaison avec -A'-, le groupe qui prolonge le système pi-conjugué du naphtopyrane indénocondensé lié à la position 11 de celui-ci, ou un substituant ou une position disponible sur le naphtopyrane indénocondensé et un deuxième azote aminé dudit résidu diamine forme une liaison avec -E-, -G- ou -J ; ou
   (b) un résidu alcool aminé ou un dérivé de celui-ci, ledit résidu alcool aminé étant un résidu alcool aminé aliphatique, un résidu alcool aminé cycloaliphatique, un résidu alcool azacycloaliphatique, un résidu alcool diazacycloaliphatique ou un résidu alcool aminé aromatique, dans lequel un azote aminé dudit résidu alcool aminé forme une liaison avec -A'-, le groupe qui prolonge le système pi-conjugué du naphtopyrane indénocondensé lié à la position 11 de celui-ci ou un substituant ou une position disponible sur le naphtopyrane indénocondensé et un oxygène alcoolique dudit résidu alcool aminé forme une liaison avec -E-, -G- ou -J ou ledit azote aminé dudit résidu alcool aminé forme une liaison avec -E-, -G- ou -J et ledit oxygène alcoolique dudit résidu alcool aminé forme une liaison avec -A'-, le groupe qui prolonge le système pi-conjugué du naphtopyrane indénocondensé lié à la position 11 de celui-ci ou un substituant ou une position disponible sur le naphtopyrane indénocondensé ;

(iii) chaque -E- est indépendamment un résidu acide dicarboxylique ou un dérivé de celui-ci, ledit résidu acide dicarboxylique étant un résidu acide dicarboxylique aliphatique, un résidu acide dicarboxylique cycloalipahtique ou un résidu acide dicarboxylique aromatique, dans lequel un premier groupe carbonyle dudit résidu acide dicarboxylique forme une liaison avec -G- ou -D- et un deuxième groupe carbonyle dudit résidu acide dicarboxylique forme une liaison avec -G- ;
(iv) chaque -G- est indépendamment :

(a) $-[(OC_2H_4)_x(OC_3H_6)_y(OC_4H_8)_z]-O-$, où x, y et z sont chacun indépendamment choisis et compris dans la plage allant de 0 à 50 et une somme de x, y et z est comprise dans la plage allant de 1 à 50 ;
(b) un résidu polyol ou un dérivé de celui-ci, ledit résidu polyol étant un résidu polyol aliphatique, un résidu polyol cycloaliphatique ou un résidu polyol aromatique, dans lequel un premier oxygène polyalcoolique dudit résidu polyol forme une liaison avec -A'-, -D-, -E-, le groupe qui prolonge le système pi-conjugué du naphtopyrane indénocondensé lié à la position 11 de celui-ci ou un substituant ou une position disponible sur le naphtopyrane indénocondensé et un deuxième oxygène polyalcoolique dudit résidu polyol forme une liaison avec -E- ou -J ; ou
(c) une de leurs combinaisons, dans laquelle le premier oxygène polyalcoolique du résidu polyol forme une liaison avec un groupe $-[(OC_2H_4)_x(OC_3H_6)_y(OC_4H_8)_z]$ - et le deuxième oxygène polyalcoolique forme une liaison avec -E- ou -J ; et

(v) chaque -J est indépendamment :

(a) un groupe -K, où -K est $-CH_2COOH$, $-CH(CH_3)COOH$, $-C(O)(CH_2)_wCOOH$, $-C_6H_4SO_3H$, $-C_5H_{10}SO_3H$, $-C_4H_8SO_3H$, $-C_3H_6SO_3H$,- $C_2H_4SO_3H$ ou $-SO_3H$, où w est compris dans la plage allant de 1 à 18 ;
(b) un atome d'hydrogène, à condition que si -J est un atome d'hydrogène -J soit lié à un atome d'oxygène de -D- ou -G- ou un atome d'azote de -D- ; ou
(c) un groupe -L ou un résidu de celui-ci, où -L est un groupe acryle, méthacryle, crotyle, 2-(méthacryloxy)-éthylcarbamyle, 2-(méthacryloxy)éthoxycarbonyle, 4-vinylphényle, vinyle, 1-chlorovinyle ou époxy.

**4.** Matériau photochromique selon l'une quelconque des revendications 1 à 3, dans lequel le groupe qui prolonge le système pi-conjugué du naphtopyrane indénocondensé lié à la position 11 de celui-ci est un groupe aryle substitué ou non substitué ; un groupe hétéroaryle substitué ou non substitué ; ou un groupe représenté par -X=Y ou -X'=Y', où :

(i) X est $-CR^1$, -N, -NO, $-SR^1$, - $S(=O)R^1$ ou -P (=O) $R^1$, où $R^1$ est un groupe amino, un groupe dialkylamino, un groupe diarylamino, un groupe acyloxy, un groupe acylamino, un groupe alkyle en $C_1$-$C_{20}$ substitué ou non substitué, un groupe alcényle en $C_2$-$C_{20}$ substitué ou non substitué, un groupe alcynyle en $C_2$-$C_{20}$ substitué ou non substitué, un atome d'halogène, un atome d'hydrogène, un groupe hydroxy, un atome d'oxygène, un résidu polyol, un groupe phénoxy substitué ou non substitué, un groupe benzyloxy substitué ou non substitué, un groupe alcoxy substitué ou non substitué, un groupe oxyalcoxy substitué ou non substitué, un groupe alkylamino, un groupe mercapto, un groupe alkylthio, un groupe aryle substitué ou non substitué, un groupe hétéroaryle substitué ou non substitué, un groupe hétérocyclique substitué ou non substitué, un substituant réactif ou un substituant de compatibilisation comme défini selon la revendication 3 ou un matériau photochromique, à condition que :

(a) si X est $-CR^1$ ou -N, Y soit $C(R^2)_2$, $NR^2$, O ou S, où chaque $R^2$ est indépendamment choisi dans chaque cas parmi un groupe amino, un groupe dialkylamino, un groupe diarylamino, un groupe acyloxy, un groupe acylamino, un groupe alkyle en $C_1$-$C_{20}$ substitué ou non substitué, un groupe alcényle en $C_2$-$C_{20}$ substitué ou non substitué, un groupe alcynyle en $C_2$-$C_{20}$ substitué ou non substitué, un atome d'halogène, un atome d'hydrogène, un groupe hydroxy, un atome d'oxygène, un résidu polyol, un groupe phénoxy substitué ou non substitué, un groupe benzyloxy substitué ou non substitué, un groupe alcoxy substitué ou non substitué, un groupe oxyalcoxy substitué ou non substitué, un groupe alkylamino, un groupe mercapto, un groupe alkylthio, un groupe aryle substitué ou non substitué, un groupe hétéroaryle substitué ou non substitué, un

groupe hétérocyclique substitué ou non substitué, un substituant réactif ou un substituant de compatibilisation comme défini selon la revendication 3 et un matériau photochromique ; et
(b) si X est -NO, -$SR^1$, -S(=O)$R^1$ ou -P(=O)$R^1$ Y soit O ; et

(ii) X' est -C ou -$N^+$, et Y' est -$CR^3$ ou -N, où $R^3$ est un groupe amino, un groupe dialkylamino, un groupe diarylamino, un groupe acyloxy, un groupe acylamino, un groupe alkyle en $C_1$-$C_{20}$ substitué ou non substitué, un groupe alcényle en $C_2$-$C_{20}$ substitué ou non substitué, un groupe alcynyle en $C_2$-$C_{20}$ substitué ou non substitué, un atome d'halogène, un atome d'hydrogène, un groupe hydroxy, un atome d'oxygène, un résidu polyol, un groupe phénoxy substitué ou non substitué, un groupe benzyloxy substitué ou non substitué, un groupe alcoxy substitué ou non substitué, un groupe oxyalcoxy substitué ou non substitué, un groupe alkylamino, un groupe mercapto, un groupe alkylthio, un groupe aryle substitué ou non substitué, un groupe hétéroaryle substitué ou non substitué, un groupe hétérocyclique substitué ou non substitué, un substituant réactif ou un substituant de compatibilisation comme défini selon la revendication 3 ou un matériau photochromique ; ou le groupe qui prolonge le système pi-conjugué du naphtopyrane indénocondensé lié à la position 11 du naphtopyrane indénocondensé avec un groupe lié à la position 12 du naphtopyrane indénocondensé ou avec un groupe lié à la position 10 du naphtopyrane indénocondensé forme un groupe condensé, ledit groupe condensé étant un groupe indéno, dihydronaphtalène, indole, benzofurane, benzopyrane ou thianaphtène.

**5.** Matériau photochromique selon la revendication 4, lorsque la revendication 4 dépend de la revendication 1, dans lequel le groupe qui prolonge le système pi-conjugué du naphtopyrane indénocondensé est un groupe alcényle en $C_2$-$C_{20}$ substitué ou non substitué ; un groupe alcynyle en $C_2$-$C_{20}$ substitué ou non substitué ; un groupe aryle substitué ou non substitué ; un groupe hétéroaryle substitué ou non substitué ; -C(=O)$R^1$ ; ou -N ( =Y ) ou -$N^+$(≡Y'), où Y est C( $R^2)^2$ , $NR^2$, O ou S et Y' est $CR^3$ ou N.

**6.** Matériau photochromique selon la revendication 5, dans lequel le groupe qui prolonge le système pi-conjugué du naphtopyrane indénocondensé lié à la position 11 de celui-ci est un groupe aryle ou un groupe hétéroaryle qui est non substitué ou substitué par au moins un d'un groupe alkyle substitué ou non substitué, d'un groupe alcoxy substitué ou non substitué, d'un groupe oxyalcoxy substitué ou non substitué, d'un groupe amide, d'un groupe amino substitué ou non substitué, d'un groupe aryle substitué ou non substitué, d'un groupe hétéroaryle substitué ou non substitué, d'un groupe azide, d'un groupe carbonyle, d'un groupe carboxy, d'un groupe ester, d'un groupe éther, d'un atome d'halogène, d'un groupe hydroxy, d'un résidu polyol, d'un groupe phénoxy substitué ou non substitué, d'un groupe benzyloxy substitué ou non substitué, d'un groupe cyano, d'un groupe nitro, d'un groupe sulfonyle, d'un groupe thiol, d'un groupe hétérocyclique substitué ou non substitué, d'un substituant réactif ou d'un substituant de compatibilisation comme défini selon la revendication 3 ou d'un matériau photochromique, à condition que si le groupe aryle ou le groupe hétéroaryle comprend plus d'un substituant, chaque substituant puisse être choisi de manière indépendante.

**7.** Matériau photochromique selon la revendication 5, dans lequel le groupe qui prolonge le système pi-conjugué du naphtopyrane indénocondensé lié à la position 11 de celui-ci est -C (=O) $R^1$, où $R^1$ est un groupe acylamino, un groupe acyloxy, un groupe alkyle en $C_1$-$C_{20}$ substitué ou non substitué, un groupe alcoxy substitué ou non substitué, un groupe oxyalcoxy substitué ou non substitué, un groupe amino, un groupe dialkylamino, un groupe diarylamino, un groupe aryle substitué ou non substitué, un groupe hétéroaryle substitué ou non substitué, un groupe hétérocyclique substitué ou non substitué, un atome d'halogène, un atome d'hydrogène, un groupe hydroxy, un atome d'oxygène, un résidu polyol, un groupe phénoxy substitué ou non substitué, un groupe benzyloxy substitué ou non substitué, un substituant réactif comme défini selon la revendication 3 ou un matériau photochromique.

**8.** Matériau photochromique selon la revendication 3, lorsque la revendication 3 dépend de la revendication 1, dans lequel le matériau photochromique comprend un indéno[2',3':3,4]-naphto[1,2-b]pyrane et au moins une des positions 6, 7, 13 et 3, et le groupe qui prolonge le système pi-conjugué du indéno[2',3':3,4]naphto[1,2-b]pyrane lié à la position 11 de celui-ci comprend un substituant réactif comme défini selon la revendication 3.

**9.** Matériau photochromique selon la revendication 1, dans lequel le naphtopyrane indénocondensé est exempt de groupes spirocycliques en position 13 du naphtopyrane indénocondensé.

**10.** Matériau photochromique selon la revendication 1, dans lequel le naphtopyrane indénocondensé est un indéno[2', 3':3,4]naphto[1,2-b]pyrane et dans lequel :

(i) la position 6 de l'indéno [2', 3': 3,4] naphto [1, 2-b]pyrane est substituée par un groupe contenant un atome

d'azote ou un groupe contenant un atome d'oxygène ;
(ii) la position 7 de l'indéno[2',3':3,4]naphto[1,2-b]pyrane est substituée par un groupe contenant un atome d'azote ou un groupe contenant un atome d'oxygène ; et
(iii) la position 13 de l'indéno[2',3':3,4]naphto[1,2-b]pyrane est disubstituée, à condition que chacun des substituants en position 13 soit indépendamment un atome d'hydrogène, un groupe alkyle en $C_1$-$C_6$, un groupe cycloalkyle en $C_3$-$C_7$, un groupe allyle, un groupe phényle substitué ou non substitué, un groupe benzyle substitué ou non substitué, un groupe amino substitué ou non substitué ou -C(O)$R^{30}$ où $R^{30}$ est un atome d'hydrogène, un groupe hydroxy, un groupe alkyle en $C_1$-$C_6$, un groupe alcoxy en $C_1$-$C_6$, un groupe phényle ou naphtyle non substitué, mono- ou disubstitué, un groupe phénoxy, un groupe phénoxy mono- ou disubstitué par un groupe alkyle en $C_1$-$C_6$ ou un groupe phénoxy mono- ou disubstitué par un groupe alcoxy en $C_1$-$C_6$.

**11.** Composition photochromique comprenant le matériau photochromique selon la revendication 1, incorporé dans au moins une partie d'un matériau organique, ledit matériau organique étant un matériau polymère, un matériau oligomère, un matériau monomère ou un mélange ou une combinaison de ceux-ci.

**12.** Composition photochromique selon la revendication 11, dans laquelle le matériau organique est un matériau polymère, ledit matériau polymère étant un copolymère d'éthylène et d'acétate de vinyle ; un copolymère d'éthylène et d'alcool vinylique ; un copolymère d'éthylène, d'acétate de vinyle et d'alcool vinylique ; un acétobutyrate de cellulose ; un poly(uréthane) ; un poly(acrylate) ; un poly(méthacrylate) ; un époxy ; un polymère fonctionnel aminoplaste ; un poly(anhydride) ; un poly(urée-uréthane) ; un polymère fonctionnel N-alcoxyméthyl(méth)acrylamide ; un poly (siloxane) ; un poly(silane) ; ou un mélange ou une combinaison de ceux-ci.

**13.** Composition photochromique selon la revendication 11, dans laquelle la composition photochromique comprend au moins un d'un matériau photochromique complémentaire, d'un photoinitiateur, d'un initiateur thermique, d'un inhibiteur de polymérisation, d'un solvant, d'un photostabilisant, d'un stabilisant thermique, d'un agent de démoulage, d'un agent de contrôle de rhéologie, d'un agent égalisant, d'un piégeur de radicaux libres et d'un promoteur d'adhésion.

**14.** Composition photochromique selon la revendication 11, dans laquelle la composition photochromique est une composition de revêtement.

**15.** Article photochromique comprenant un substrat et un matériau photochromique selon la revendication 1 relié à au moins une partie du substrat.

**16.** Article photochromique selon la revendication 15, dans lequel l'article photochromique est un élément optique, ledit élément optique étant au moins un d'un élément ophtalmique, d'un élément graphique, d'une fenêtre, d'un miroir et d'un élément de cellules à cristaux liquides.

**17.** Article photochromique selon la revendication 16, dans lequel l'élément optique est un élément ophtalmique, ledit élément ophtalmique étant au moins un d'une lentille corrective, d'une lentille non corrective, d'une lentille à effet loupe, d'une lentille protectrice, d'une visière, de lunettes de sécurité et d'une lentille pour un instrument optique.

**18.** Article photochromique selon la revendication 15, dans lequel le substrat comprend un matériau polymère et le matériau photochromique est incorporé dans au moins une partie du matériau polymère.

**19.** Article photochromique selon la revendication 18, dans lequel le matériau photochromique est au moins mélangé avec au moins une partie du matériau polymère, lié à au moins une partie du matériau polymère et/ou imbibé dans au moins une partie du matériau polymère.

**20.** Article photochromique selon la revendication 15, dans lequel l'article photochromique comprend un revêtement au moins partiel relié à au moins une partie du substrat, ledit revêtement au moins partiel comprenant le matériau photochromique.

**21.** Article photochromique selon la revendication 20, dans lequel le substrat est un matériau polymère ou un verre.

**22.** Article photochromique selon la revendication 15, dans lequel au moins un revêtement ou film au moins partiel est relié à au moins une partie du substrat, ledit au moins un revêtement ou film au moins partiels étant au moins un d'un revêtement ou d'un film primaire, d'un revêtement ou d'un film protecteur, d'un revêtement ou d'un film antireflet,

d'un revêtement ou d'un film photochromique classique et d'un revêtement ou d'un film polarisant.

**23.** Article photochromique selon la revendication 15, dans lequel l'article photochromique comprend au moins un d'un matériau photochromique complémentaire, d'un photoinitiateur, d'un initiateur thermique, d'un inhibiteur de polymérisation, d'un solvant, d'un photostabilisant, d'un stabilisant thermique, d'un agent de démoulage, d'un agent de contrôle de rhéologie, d'un agent égalisant, d'un piégeur de radicaux libres et d'un promoteur d'adhésion.

**24.** Procédé de préparation d'un article photochromique comprenant la liaison d'un matériau photochromique selon la revendication 1 à au moins une partie d'un substrat, ladite liaison du matériau photochromique à ladite au moins une partie du substrat comprenant au moins un d'un moulage dans un moule, d'un revêtement, d'une imbibition, d'une stratification et d'un moulage en place.

**25.** Matériau photochromique selon la revendication 1, représenté par :

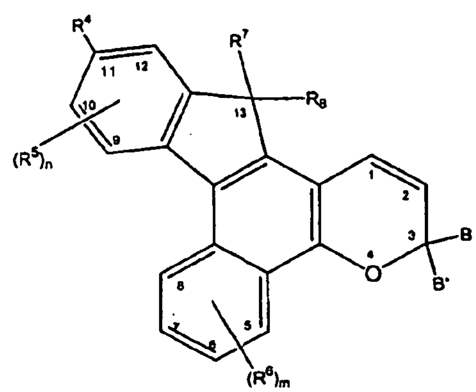

,

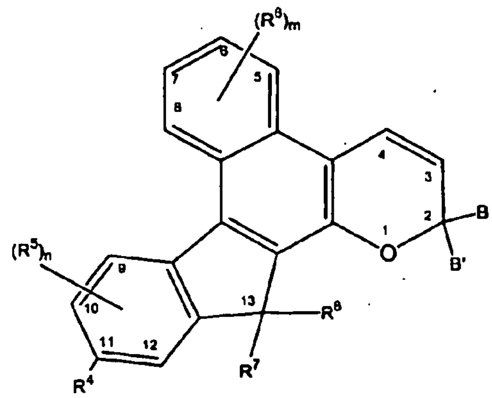

ou un mélange de ceux-ci, où

(i) $R^4$ est un groupe aryle substitué ou non substitué ; un groupe hétéroaryle substitué ou non substitué ; ou un groupe représenté par -X=Y ou -X'=Y', où

(a) X est $-CR^1$, -N, -NO, $-SR^1$, -S (=O) $R^1$ ou $-P(=O)R^1$, où $R^1$ est un groupe amino, un groupe dialkylamino, un groupe diarylamino, un groupe acyloxy, un groupe acylamino, un groupe alkyle en $C_1$-$C_{20}$ substitué ou non substitué, un groupe alcényle en $C_2$-$C_{20}$ substitué ou non substitué, un groupe alcynyle en $C_2$-$C_{20}$ substitué ou non substitué, un atome d'halogène, un atome d'hydrogène, un groupe hydroxy, un atome d'oxygène, un résidu polyol, un groupe phénoxy substitué ou non substitué, un groupe benzyloxy substitué ou non substitué, un groupe alcoxy substitué ou non substitué, un groupe oxyalcoxy substitué ou non substitué, un groupe alkylamino, un groupe mercapto, un groupe alkylthio, un groupe aryle substitué ou non substitué, un groupe hétéroaryle substitué ou non substitué, un groupe hétérocyclique substitué ou non substitué, un substituant réactif ou un substituant de compatibilisation comme défini selon la revendication 3 ou un matériau photochromique, à condition que :

(1) si X est $-CR^1$ ou -N, Y soit $C(R^2)_2$ , $NR^2$, O ou S, où chaque $R^2$ est indépendamment choisi dans chaque cas parmi un groupe amino, un groupe dialkylamino, un groupe diarylamino, un groupe acyloxy, un groupe acylamino, un groupe alkyle en $C_1$-$C_{20}$ substitué ou non substitué, un groupe alcényle en $C_2$-$C_{20}$ substitué ou non substitué, un groupe alcynyle en $C_2$-$C_{20}$ substitué ou non substitué, un atome d'halogène, un atome d'hydrogène, un groupe hydroxy, un atome d'oxygène, un résidu polyol, un groupe phénoxy substitué ou non substitué, un groupe benzyloxy substitué ou non substitué, un groupe alcoxy substitué ou non substitué, un groupe oxyalcoxy substitué ou non substitué, un groupe alkylamino, un groupe mercapto, un groupe alkylthio, un groupe aryle substitué ou non substitué, un groupe hétéroaryle substitué ou non substitué, un groupe hétérocyclique substitué ou non substitué, un substituant réactif ou un substituant de compatibilisation comme défini selon la revendication 3 et un matériau photochromique ; et
(2) si X est -NO, $-SR^1$, -S (=O) $R^1$ ou $-P(=O)R^1$ Y soit O ; et

(b) X' est -C ou $-N^+$ et Y' est $CR^3$ ou N ; où $R^3$ est un groupe amino, un groupe dialkylamino, un groupe diarylamino, un groupe acyloxy, un groupe acylamino, un groupe alkyle en $C_1$-$C_{20}$ substitué ou non substitué, un groupe alcényle en $C_2$-$C_{20}$ substitué ou non substitué, un groupe alcynyle en $C_2$-$C_{20}$ substitué ou non substitué, un atome d'halogène, un atome d'hydrogène, un groupe hydroxy, un atome d'oxygène, un résidu polyol, un groupe phénoxy substitué ou non substitué, un groupe benzyloxy substitué ou non substitué, un groupe alcoxy substitué ou non substitué, un groupe oxyalcoxy substitué ou non substitué, un groupe alkylamino, un groupe mercapto, un groupe alkylthio, un groupe aryle substitué ou non substitué, un groupe hétéroaryle substitué ou non substitué, un groupe hétérocyclique substitué ou non substitué, un substituant réactif ou un substituant de compatibilisation comme défini selon la revendication 3 ou un matériau photochromique ; ou

$R^4$ avec un groupe $R^5$ lié à la position 12 du naphtopyrane indénocondensé ou avec un groupe $R^5$ lié à la position 10 du naphtopyrane indénocondensé forme un groupe condensé, ledit groupe condensé étant un groupe indéno, dihydronaphtalène, indole, benzofurane, benzopyrane ou thianaphtène ;

(ii) n est compris dans la plage allant de 0 à 3 ;

(iii) m est compris dans la plage allant de 0 à 4 ;

(iv) chacun de $R^5$ et $R^6$ est indépendamment choisi dans chaque cas parmi : un substituant réactif ou un substituant de compatibilisation comme défini selon la revendication 3 ; un atome d'hydrogène ; un groupe alkyle en $C_1$-$C_6$ ; un atome de chlore ; un atome de fluor ; un groupe cycloalkyle en $C_3$-$C_7$ ; un groupe phényle substitué ou non substitué, lesdits substituants du groupe phényle étant un groupe alkyle en $C_1$-$C_6$ ou un groupe alcoxy en $C_1$-$C_6$ ; $-OR^{10}$ ou $-OC(=O)R^{10}$ où $R^{10}$ est S, un atome d'hydrogène, un groupe amine, un groupe alkyle en $C_1$-$C_6$, un groupe phénylalkyle en $C_1$-$C_3$, un groupe phénylalkyle en $C_1$-$C_3$ monosubstitué par un groupe alkyle en $C_1$-$C_6$, un groupe phénylalkyle en $C_1$-$C_3$ monosubstitué par un groupe alcoxy en $C_1$-$C_6$, un groupe (alcoxy en $C_1$-$C_6$) alkyle en $C_2$-$C_4$, un groupe cycloalkyle en $C_3$-$C_7$ ou un groupe cycloalkyle en $C_3$-$C_7$ monosubstitué par un groupe alkyle en $C_1$-$C_4$ ; un groupe phényle monosubstitué, ledit groupe phényle ayant un substituant situé en position para, le substituant étant un résidu acide dicarboxylique ou un dérivé de celui-ci, un résidu diamine ou un dérivé de celui-ci, un résidu alcool aminé ou un dérivé de celui-ci, un résidu polyol ou un dérivé de celui-ci, $-(CH_2)-$, $-(CH_2)_t-$ ou $-[O-(CH_2)_t]_k-$, où t est compris dans la plage allant de 2 à 6 et k est compris dans la plage allant de 1 à 50 et où le substituant est relié à un groupe aryle sur un autre matériau photochromique ; $-N(R^{11})R^{12}$, où $R^{11}$ et $R^{12}$ sont chacun indépendamment un atome d'hydrogène, un groupe alkyle en $C_1$-$C_8$, un groupe phényle, un groupe naphtyle, un groupe furanyle, un groupe benzofuran-2-yle, un groupe benzofuran-3-yle, un groupe thiényle, un groupe benzothién-2-yle, un groupe benzothién-3-yle, un groupe dibenzofuranyle, un groupe dibenzothiényle, un groupe benzopyridyle et un groupe fluorényle, un groupe alkylaryle en $C_1$-$C_8$, un groupe cycloalkyle en $C_3$-$C_{20}$, un groupe bicycloalkyle en $C_4$-$C_{20}$, un groupe tricycloalkyle en $C_5$-$C_{20}$ ou un groupe alcoxyalkyle en $C_1$-$C_{20}$, ou $R^{11}$ et $R^{12}$ ensemble avec un atome d'azote forment un cycle hétérobicycloalkyle en $C_3$-$C_{20}$ ou un cycle hétérotricycloalkyle en $C_4$-$C_{20}$ ; un cycle contenant un atome d'azote représenté par :

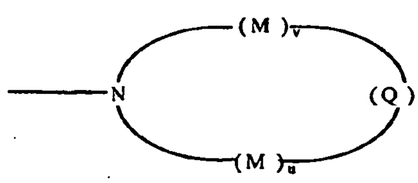

où chaque -M- est indépendamment choisi dans chaque cas parmi $-CH_2-$, $-CH(R^{13})-$, $-C(R^{13})_2-$, -CH (aryle)-, $-C(aryle)_2-$ et $-C(R^{13})(aryle)-$ et -Q- est -M-, -O-, -S-, -S(O)-, $-SO_2-$, -NH-, $-N(R^{13})-$ ou -N(aryle)-, où chaque $R^{13}$ est indépendamment un groupe alkyle en $C_1$-$C_6$, chaque (aryle) est indépendamment un groupe phényle ou naphtyle, u est compris dans la plage allant de 1 à 3 et v est compris dans la plage allant de 0 à 3, à condition que si v vaut 0, -Q- soit -M- ; un groupe représenté par :

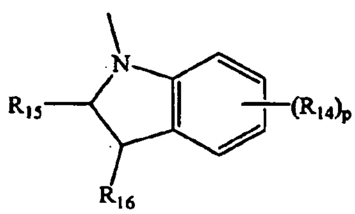

ou

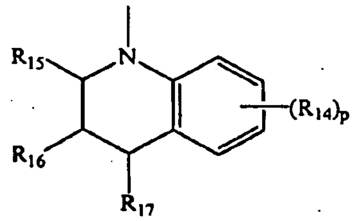

où chacun de $R^{15}$, $R^{16}$ et $R^{17}$ est indépendamment un atome d'hydrogène, un groupe alkyle en $C_1$-$C_6$, un groupe phényle ou un groupe naphtyle, ou $R^{15}$ et $R^{16}$ forment ensemble un cycle de 5 à 8 atomes de carbone, chaque $R^{14}$ est indépendamment un groupe alkyle en $C_1$-$C_6$, un groupe alcoxy en $C_1$-$C_6$, un atome de fluor ou un atome de chlore et p est compris dans la plage allant de 0 à 3 ; et une amine spirobicyclique en $C_4$-$C_{18}$ substituée ou non substituée ou une amine spirotricyclique en $C_4$-$C_{18}$ substituée ou non substituée, où lesdits substituants sont indépendamment un groupe aryle, un groupe alkyle en $C_1$-$C_6$, un groupe alcoxy en $C_1$-$C_6$ ou un groupe phénylalkyle en $C_1$-$C_6$ ; ou

un groupe $R^6$ en position 6 et un groupe $R^6$ en position 7 forment ensemble un groupe représenté par :

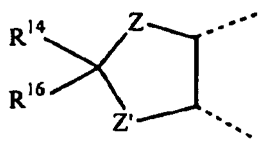

ou

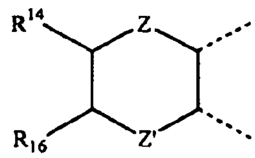

où chacun de Z et Z' est indépendamment un atome d'oxygène ou le groupe $-NR^{11}-$, où $R^{11}$, $R^{14}$ et $R^{16}$ sont comme décrits ci-dessus ;

(v) $R^7$ et $R^8$ sont chacun indépendamment :

un substituant réactif ou un substituant de compatibilisation comme défini selon la revendication 3 ; un atome d'hydrogène ; un groupe hydroxy ; un groupe alkyle en $C_1$-$C_6$ ; un groupe cycloalkyle en $C_3$-$C_7$ ; un groupe allyle ; un groupe phényle ou benzyle substitué ou non substitué, où chacun desdits substituants du groupe phényle et benzyle est indépendamment un groupe alkyle en $C_1$-$C_6$ ou un groupe alcoxy en $C_1$-$C_6$ ; un atome de chlore ; un atome de fluor ; un groupe amino substitué ou non substitué ; $-C(O)R^9$ où $R^9$ est un atome d'hydrogène, un groupe hydroxy, un groupe alkyle en $C_1$-$C_6$, un groupe alcoxy en $C_1$-$C_6$, un groupe phényle ou naphtyle non substitué, mono- ou disubstitué, où chacun desdits substituants est indépendamment un groupe alkyle en $C_1$-$C_6$ ou un groupe alcoxy en $C_1$-$C_6$, un groupe phénoxy, un groupe phénoxy mono- ou disubstitué par un groupe alkyle en $C_1$-$C_6$, un groupe phénoxy mono- ou disubstitué par un groupe alcoxy en $C_1$-$C_6$, un groupe amino, un groupe mono- ou di(alkyle en $C_1$-$C_6$)amino, un groupe phénylamino, un groupe phénylamino mono- ou disubstitué par un groupe alkyle en $C_1$-$C_6$ ou un groupe phénylamino mono- ou disubstitué par un groupe alcoxy en $C_1$-$C_6$ ; $-OR^{18}$ où $R^{18}$ est un groupe alkyle en $C_1$-$C_6$, un groupe phénylalkyle en $C_1$-$C_3$, un groupe phénylalkyle en $C_1$-$C_3$ monosubstitué par un groupe alkyle en $C_1$-$C_6$, un groupe phénylalkyle en $C_1$-$C_3$ monosubstitué par un groupe alcoxy en $C_1$-$C_6$, un groupe (alcoxy en $C_1$-$C_6$) alkyle en $C_2$-$C_4$, un groupe cycloalkyle en $C_3$-$C_7$, un groupe cycloalkyle en $C_3$-$C_7$ monosubstitué par un groupe alkyle en $C_1$-$C_4$, un groupe chloroalkyle en $C_1$-$C_6$, un groupe fluoroalkyle en $C_1$-$C_6$, un groupe allyle ou $-CH(R^{19})T$ où $R^{19}$ est un atome d'hydrogène ou un groupe alkyle en $C_1$-$C_3$, T est CN, $CF_3$ ou $COOR^{20}$ où $R^{20}$ est un atome d'hydrogène ou un groupe alkyle en $C_1$-$C_3$, ou où $R^{18}$ est -C(=O)U où U est un atome d'hydrogène, un groupe alkyle en $C_1$-$C_6$, un groupe alcoxy en $C_1$-$C_6$, un groupe phényle ou naphtyle non substitué, mono- ou disubstitué, où chacun desdits substituants est indépendamment un groupe alkyle en $C_1$-$C_6$ ou un groupe alcoxy en $C_1$-$C_6$, un groupe phénoxy, un groupe phénoxy mono- ou disubstitué par un groupe alkyle en $C_1$-$C_6$, un groupe phénoxy mono- ou disubstitué par un groupe alcoxy en $C_1$-$C_6$, un groupe amino, un groupe mono- ou di(alkyle en $C_1$-$C_6$)amino, un groupe phénylamino, un groupe phénylamino mono- ou disubstitué par un groupe alkyle en $C_1$-$C_6$ ou un groupe phénylamino mono- ou disubstitué par un groupe alcoxy en $C_1$-$C_6$ ; et un groupe phényle monosubstitué, ledit groupe phényle ayant un substituant situé en position para, le substituant étant un résidu acide dicarboxylique ou un dérivé de celui-ci, un résidu diamine ou un dérivé de celui-ci, un résidu alcool aminé ou un dérivé de celui-ci, un résidu polyol ou un dérivé de celui-ci, $-(CH_2)-$, $-(CH_2)_t-$ ou $-[O-(CH_2)_t]_k-$, où t est

compris dans la plage allant de 2 à 6 et k est compris dans la plage allant de 1 à 50, et où le substituant est relié à un groupe aryle sur un autre matériau photochromique ; ou

$R^7$ et $R^8$ forment ensemble un groupe oxo ; un groupe spirocarbocyclique contenant de 3 à 6 atomes de carbone, à condition que le groupe spirocarbocyclique ne soit pas un groupe norbornyle ; ou un groupe spirohétérocyclique contenant de 1 à 2 atomes d'oxygène et de 3 à 6 atomes de carbone incluant l'atome de spirocarbone, lesdits groupes spirocarbocyclique et spirohétérocyclique étant attachés à 0, 1 ou 2 cycles benzéniques ; et

(vi) B et B' sont chacun indépendamment :

un groupe aryle qui est monosubstitué par un substituant réactif ou un substituants de compatibilisation comme défini selon la revendication 3 ; un groupe aryle non substitué, mono-, di- ou trisubstitué ; un groupe 9-julolidinyle ; un groupe hétéroaromatique non substitué, mono- ou disubstitué choisi parmi les groupes pyridyle, furanyle, benzofuran-2-yle, benzofuran-3-yle, thiényle, benzothién-2-yle, benzothién-3-yle, dibenzofuranyle, dibenzothiényle, carbazoyle, benzopyridyle, indolinyle et fluorényle ; où les substituants des groupes aryle et hétéroaromatique sont chacun indépendamment :

un groupe hydroxy, un groupe aryle, un groupe mono- ou di (alcoxy en $C_1$-$C_{12}$)aryle, un groupe mono- ou di (alkyle en $C_1$-$C_{12}$)aryle, un groupe halogénoaryle, un groupe cycloalkylaryle en $C_3$-$C_7$, un groupe cycloalkyle en $C_3$-$C_7$, un groupe cycloalkyloxy en $C_3$-$C_7$, un groupe (cycloalkyloxy en $C_3$-$C_7$)alkyle en $C_1$-$C_{12}$, un groupe (cycloalkyloxy en $C_3$-$C_7$)alcoxy en $C_1$-$C_{12}$, un groupe arylalkyle en $C_1$-$C_{12}$, un groupe arylalcoxy en $C_1$-$C_{12}$, un groupe aryloxy, un groupe aryloxyalkyle en $C_1$-$C_{12}$, un groupe aryloxyalcoxy en $C_1$-$C_{12}$, un groupe mono- ou di(alkyle en $C_1$-$C_{12}$) alkylaryle en $C_1$-$C_{12}$, un groupe mono- ou di(alkyle en $C_1$-$C_{12}$) alkylaryle en $C_1$-$C_{12}$, un groupe mono- ou di(alkyle en $C_1$-$C_{12}$) alcoxyaryle en $C_1$-$C_{12}$, un groupe mono- ou di(alcoxy en $C_1$-$C_{12}$)arylalcoxy en $C_1$-$C_{12}$, un groupe amino, un groupe mono- ou di(alkyle en $C_1$-$C_{12}$)amino, un groupe diarylamino, un groupe pipérazino, un groupe N-(alkyle en $C_1$-$C_{12}$)pipérazino, un groupe N-arylpipérazino, un groupe aziridino, un groupe indolino, un groupe pipéridino, un groupe morpholino, un groupe thiomorpholino, un groupe tétrahydroquinoléino, un groupe tétrahydroisoquinoléino, un groupe pyrrolidyle, un groupe alkyle en $C_1$-$C_{12}$, un groupe halogénoalkyle en $C_1$-$C_{12}$, un groupe alcoxy en $C_1$-$C_{12}$, un groupe mono (alcoxy en $C_1$-$C_{12}$) alkyle en $C_1$-$C_{12}$, un groupe acryloxy, un groupe méthacryloxy, un atome d'halogène ou -C (=O) $R^{21}$ où $R^{21}$ est -$OR^{22}$, -N ($R^{23}$)$R^{24}$, un groupe pipéridino ou un groupe morpholino, où $R^{22}$ est un groupe allyle, un groupe alkyle en $C_1$-$C_6$, un groupe phényle, un groupe phényle monosubstitué par un groupe alkyle en $C_1$-$C_6$, un groupe phényle monosubstitué par un groupe alcoxy en $C_1$-$C_6$, un groupe phénylalkyle en $C_1$-$C_3$, un groupe phénylalkyle en $C_1$-$C_3$ monosubstitué par un groupe alkyle en $C_1$-$C_6$, un groupe phénylalkyle en $C_1$-$C_3$ monosubstitué par un groupe alcoxy en $C_1$-$C_6$, un groupe (alcoxy en $C_1$-$C_6$) alkyle en $C_2$-$C_4$ ou un groupe halogénoalkyle en $C_1$-$C_6$, et $R^{23}$ et $R^{24}$ sont chacun indépendamment un groupe alkyle en $C_1$-$C_6$, un groupe cycloalkyle en $C_5$-$C_7$ ou un groupe phényle substitué ou non substitué, lesdits substituants du groupe phényle étant un groupe alkyle en $C_1$-$C_6$ ou un groupe alcoxy en $C_1$-$C_6$ ; un groupe non substitué ou monosubstitué choisi parmi les groupes pyrazolyle, imidazolyle, pyrazolinyle, imidazolinyle, pyrrolinyle, phénothiazinyle, phénoxazinyle, phénazinyle et acridinyle, lesdits substituants étant un groupe alkyle en $C_1$-$C_{12}$, un groupe alcoxy en $C_1$-$C_{12}$, un groupe phényle ou un atome d'halogène ; un groupe phényle monosubstitué, ledit groupe phényle ayant un substituant situé en position para, le substituant étant un résidu acide dicarboxylique ou un dérivé de celui-ci, un résidu diamine ou un dérivé de celui-ci, un résidu alcool aminé ou un dérivé de celui-ci, un résidu polyol ou un dérivé de celui-ci, $-(CH_2)-$, $-(CH_2)_t-$ ou $-[O-(CH_2)_t]_k-$, où t est compris dans la plage allant de 2 à 6 et k est compris dans la plage allant de 1 à 50, et où le substituant est relié à un groupe aryle sur un autre matériau photochromique ; un groupe représenté par

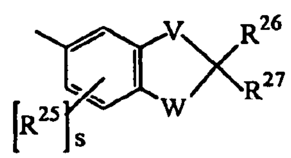

ou

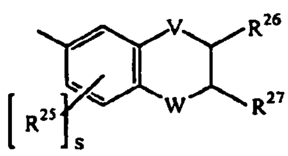

où V est $-CH_2-$ ou -O-, W est un atome d'oxygène ou un atome d'azote substitué, à condition que lorsque W est un

atome d'azote substitué, V soit $-CH_2-$, les substituants de l'atome d'azote substitué étant un atome d'hydrogène, un groupe alkyle en $C_1$-$C_{12}$ ou un groupe acyle en $C_1$-$C_{12}$, chaque $R^{25}$ étant indépendamment un groupe alkyle en $C_1$-$C_{12}$, un groupe alcoxy en $C_1$-$C_{12}$, un groupe hydroxy ou un atome d'halogène, $R^{26}$ et $R^{27}$ sont chacun indépendamment un atome d'hydrogène ou un groupe alkyle en $C_1$-$C_{12}$ et s est compris dans la plage allant de 0 à 2 ; ou un groupe représenté par :

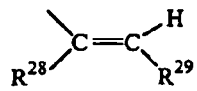

où $R^{28}$ est un atome d'hydrogène ou un groupe alkyle en $C_1$-$C_{12}$ et $R^{29}$ est un groupe naphtyle, phényle, furanyle ou thiényle non substitué, mono- ou disubstitué, lesdits substituants étant un groupe alkyle en $C_1$-$C_{12}$, un groupe alcoxy en $C_1$-$C_{12}$ ou un atome d'halogène ; ou
B et B' forment ensemble un groupe fluorén-9-ylidène ou un groupe fluorén-9-ylidène mono- ou disubstitué, chacun desdits substituants du groupe fluorén-9-ylidène étant indépendamment un groupe alkyle en $C_1$-$C_{12}$, un groupe alcoxy en $C_1$-$C_{12}$ ou un atome d'halogène.

**26.** Matériau photochromique selon la revendication 25, dans lequel au moins un d'un groupe $R^6$ en position 6, d'un groupe $R^6$ en position 7, B, B', $R^7$ $R^8$ et $R^4$ comprend un substituant réactif comme défini selon la revendication 3.

**27.** Matériau photochromique selon la revendication 3, dans lequel le naphtopyrane indénocondensé est un indéno[2',3':3,4]naphto[1,2-b]pyrane et dans lequel :

(i) chacun des groupes $R^6$ en position 7 et $R^6$ en position 6 de l'indéno[2',3':3,4]naphto[1,2-b]pyrane est indépendamment $-OR^{10}$, où $R^{10}$ est un groupe alkyle en $C_1$-$C_6$, un groupe phényle substitué ou non substitué, lesdits substituants du groupe phényle étant un groupe alkyle en $C_1$-$C_6$ ou un groupe alcoxy en $C_1$-$C_6$, un groupe phénylalkyle en $C_1$-$C_3$, un groupe phénylalkyle en $C_1$-$C_3$ monosubstitué par un groupe alkyle en $C_1$-$C_6$, un groupe phénylalkyle en $C_1$-$C_3$ monosubstitué par un groupe alcoxy en $C_1$-$C_6$, un groupe (alcoxy en $C_1$-$C_6$) alkyle en $C_2$-$C_4$, un groupe cycloalkyle en $C_3$-$C_7$ ou un groupe cycloalkyle en $C_3$-$C_7$ monosubstitué par un groupe alkyle en $C_1$-$C_4$ ; $-N(R^{11})R^{12}$, où $R^{11}$ et $R^{12}$ sont chacun indépendamment un atome d'hydrogène, un groupe alkyle en $C_1$-$C_8$, un groupe (alkyle en $C_1$-$C_8$)aryle, un groupe cycloalkyle en $C_3$-$C_{20}$, un groupe bicycloalkyle en $C_4$-$C_{20}$, un groupe tricycloalkyle en $C_5$-$C_{20}$ ou un groupe alcoxyalkyle en $C_1$-$C_{20}$, où ledit groupe aryle est un groupe phényle ou naphtyle ; un cycle contenant un atome d'azote représenté par :

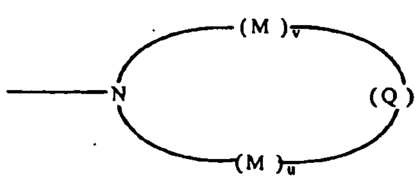

où chaque -M- est indépendamment choisi dans chaque cas parmi $-CH_2-$, $-CH(R^{13})-$, $-C(R^{13})_2-$, -CH(aryl)-, -C(aryl)$_2$- et -C ($R^{13}$) (aryl) - et -Q- est -M-, -O-, -S-, -NH-, $-N(R^{13})-$ ou -N(aryl)-, où chaque $R^{13}$ est indépendamment un groupe alkyle en $C_1$-$C_6$, chaque (aryl) est indépendamment un groupe phényle ou naphtyle, u est compris dans la plage allant de 1 à 3 et v est compris dans la plage allant de 0 à 3, à condition que si v vaut 0, -Q- soit -M- ; ou un substituant réactif ou un substituant de compatibilisation comme défini selon la revendication 3, à condition que le substituant réactif ou de compatibilisation comprenne un groupe liant comprenant un résidu alcool aminé aliphatique, un résidu alcool aminé cycloaliphatique, un résidu alcool azacycloaliphatique, un résidu alcool diazacycloaliphatique, un résidu diamine, un résidu diamine aliphatique, un résidu diamine cycloaliphatique, un résidu diazacycloalcane, un résidu amine azacycloaliphatique ; un groupe oxyalcoxy, un résidu polyol aliphatique ou un résidu polyol cycloaliphatique qui forme une liaison avec l'indéno[2',3':3,4]naphto[1,2-b]pyrane en position 6 ou en position 7 ; ou
(ii) un groupe $R^6$ en position 6 et un groupe $R^6$ en position 7 de l'indéno[2',3':3,4]naphto[1,2-b]pyrane forment ensemble un groupe représenté par :

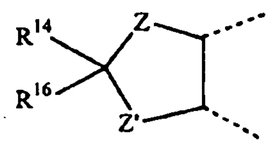

ou

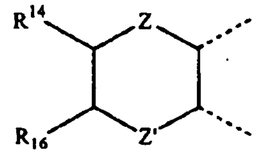

où Z et Z' sont chacun indépendamment un atome d'oxygène ou $-NR^{11}-$, où $R^{11}$ est comme décrit ci-dessus en (i).

**28.** Matériau photochromique selon la revendication 3, dans lequel le matériau photochromique est choisi parmi :

(i) un 3,3-di(4-méthoxyphényl)-6,7-diméthoxy-11-cyano-13,13-diméthyl-3H,13H-indéno[2',3':3,4]naphto[1,2-b]pyrane ;
(ii) un 3,3-di(4-méthoxyphényl)-6,7-diméthoxy-11-carboxy-13,13-diméthyl-3H,13H-indéno[2',3':3,4]naphto[1,2-b]pyrane ;
(iii) un 3,3-di(4-méthoxyphényl)-6,7-diméthoxy-11-méthoxycarbonyl-13,13-diméthyl-3H,13H-indéno[2',3':3,4]naphto-[1,2-b]pyrane ;
(iv) un 3,3-di(4-méthoxyphényl)-6,7-diméthoxy-11-(2-(2-hydroxyéthoxy)éthoxycarbonyl)-13,13-diméthyl-3H,13H-indéno[2',3':3,4]naphto[1,2-b]pyrane ;
(v) un 3,3-di(4-méthoxyphényl)-6,7-diméthoxy-11-(4-fluorophényl)-13,13-diméthyl-3H,13H-indéno[2',3':3,4]naphto-[1,2-b]pyrane ;
(vi) un 3,3-di(4-méthoxyphényl)-6,7-diméthoxy-11-(4-(phényl)phényl)-13,13-diméthyl-3H,13H-indéno[2',3':3,4]naphto-[1,2-b]pyrane ;
(vii) un 3,3-di(4-méthoxyphényl)-6,7-diméthoxy-11-(4-(hydroxyméthyl)phényl)-13,13-diméthyl-3H,13H-indéno[2',3':3,4] naphto[1,2-b]pyrane ;
(viii) un 3,3-di(4-méthoxyphényl)-6,7-diméthoxy-11-(3-hydroxy-3-méthylbutynyl)-13,13-diméthyl-3H,13H-indéno[2',3':3,4]naphto[1,2-b]pyrane ;
(ix) un 3,3-di(4-méthoxyphényl)-6,7-diméthoxy-11-(2-phényléthynyl)-13,13-diméthyl-3H,13H-indéno[2',3':3,4]naphto-[1,2-b]pyrane ;
(x) un 3,3-di(4-méthoxyphényl)-6,7-diméthoxy-11-phényl-13-éthyl-13-méthoxy-3H,13H-indéno[2',3':3,4]naphto[1,2-b]pyrane ;
(xi) un 3-phényl-3-(4-méthoxyphényl)-6,7-diméthoxy-11-(4-(2-méthacryloxyéthoxy)carbonylphényl)-13,13-diméthyl-3H,13H-indéno[2',3':3,4]naphto[1,2-b]pyrane ;
(xii) un 3,3-di(4-méthoxyphényl)-6-méthoxy-7-((3-(2-méthacryloxyéthyl)carbamyloxyméthylènepipéridino)-1-yl)-11-(4-(phényl)phényl)-13,13-diméthyl-3H,13H-indéno[2',3':3,4]naphto-[1,2-b]pyrane ;
(xiii) un 3-phényl-3-(4-(2-(2-méthacryloxyéthyl)-carbamyloxyéthoxy)phényl)-6-méthoxy-11-phényl-13,13-diméthyl-3H,13H-indéno[2',3':3,4]naphto[1,2-b]pyrane ;
(xiv) un 3-phényl-3-(4-méthoxyphényl)-6,7-diméhtoxy-13,13-diméthyl-11-(2-(4-(3-phényl-6,11-diméthoxy-13,13-diméthyl-3H,13H-indéno[2',3':3,4]naphto[1,2-b]pyran-3-yl)phénoxy)éthoxycarbonyl)-3H,13H-indéno[2',3':3,4]naphto[1,2-b]pyrane ;
(xv) un 3-phényl-3-(4-(2-méthacryloxyéthyl)carbamyloxyphényl)-6,7-diméthoxy-13,13-diméthyl-11-((1-(4-(3-phényl-6,11-diméthoxy-13,13-diméthyl-3H,13H-indéno[2',3':3,4]naphto[1,2-b]pyran-3-yl)phényl)pipérazino-4-yl)carbonyl)-3H,13H-indéno[2',3':3,4]naphto[1,2-b]pyrane ;
(xvi) un 3,3-di(4-méthoxyphényl)-11-méthoxycarboxy-13,13-diméthyl-3H,13H-indéno[2',3':3,4]naphto[1,2-b]pyrane ;
(xvii) un 3-(4-morpholinophényl)-3-phényl-6,7-diméthoxy-11-carboxy-13,13-diméthyl-3H,13H-indéno[2',3':3,4]naphto[1,2-b]pyrane ;
(xviii) un 3-(4-morpholinophényl)-3-phényl-6,7-diméthoxy-11-méthoxycarbonyl-13,13-diméthyl-3H,13H-indéno[2',3':3,4]-naphto[1,2-b]pyrane ;
(xix) un 3-(4-morpholinophényl)-3-(4-méthoxyphényl)-6,7-diméthoxy-11-(4-fluorophényl)-13,13-diméthyl-3H,13H-indéno[2',3':3,4]naphto[1, 2-b]pyrane ;
(xx) un 3-(4-fluorophényl)-3-(4-méthoxyphényl)-6,7-diméthoxy-11-cyano-13,13-diméthyl-3H,13H-indéno[2',3':3,4]-naphto[1,2-b]pyrane ;
(xxi) un 3-(4-morpholinophényl)-3-(4-méthoxyphényl)-11-(2-phényléthynyl)-13,13-diméthyl-3H,13H-indéno[2',3':3,4]-naphto[1,2-b]pyrane ;
(xxii) un 3,3-di(4-méthoxyphényl)-6,7-diméthoxy-11-(4-diméthylaminophényl)-13,13-diméthyl-3H,13H-indéno[2',3':3,4]-naphto[1,2-b]pyrane ;

(xxiii) un 3,3-di(4-méthoxyphényl)-6,7-diméthoxy-11-(4-méthoxyphényl)-13,13-diméthyl-3H,13H-indéno[2',3':3,4]naphto-[1,2-b]pyrane ;
(xxiv) un 3,3-di(4-méthoxyphényl)-6-méthoxy-7-morpholino-11-phényl-13-butyl-13-(2-(2-hydroxyéthoxy)éthoxy)-3H,13H-indéno[2',3':3,4]naphto[1,2-b]pyrane ;
(xxv) un 3-(4-fluorophényl)-3-(4-méthoxyphényl)-6-méthoxy-7-morpholino-11-phényl-13-butyl-13-(2-(2-hydroxyéthoxy)éthoxy)-3H,13H-indéno[2',3':3,4]naphto[1,2-b]pyrane;
(xxvi) un 3,3-di(4-fluorophényl)-11-cyano-13,13-diméthyl-3H, 13H-indéno [2',3':3,4]naphto[1,2-b]pyrane ;
(xxvii) un 3-(4-morpholinophényl)-3-phényl-6-méthoxy-7-(3-(2-méthacryloxyéthyl)carbamyloxyméthylènepipéridino-1-yl)-11-phényl-13,13-diméthyl-3H,13H-indéno[2',3':3,4]naphto[1,2-b]pyrane ;
(xxviii) un 3-(4-(2-(2-méthacryloxyéthyl)carbamyléthoxy)-phényl)-3-phényl-6,7-diméthoxy-11-phényl-13,13-diméthyl-3H,13H-indéno[2',3':3,4]naphto[1,2-b]pyrane ; et des mélanges de ceux-ci.

Fig. 1

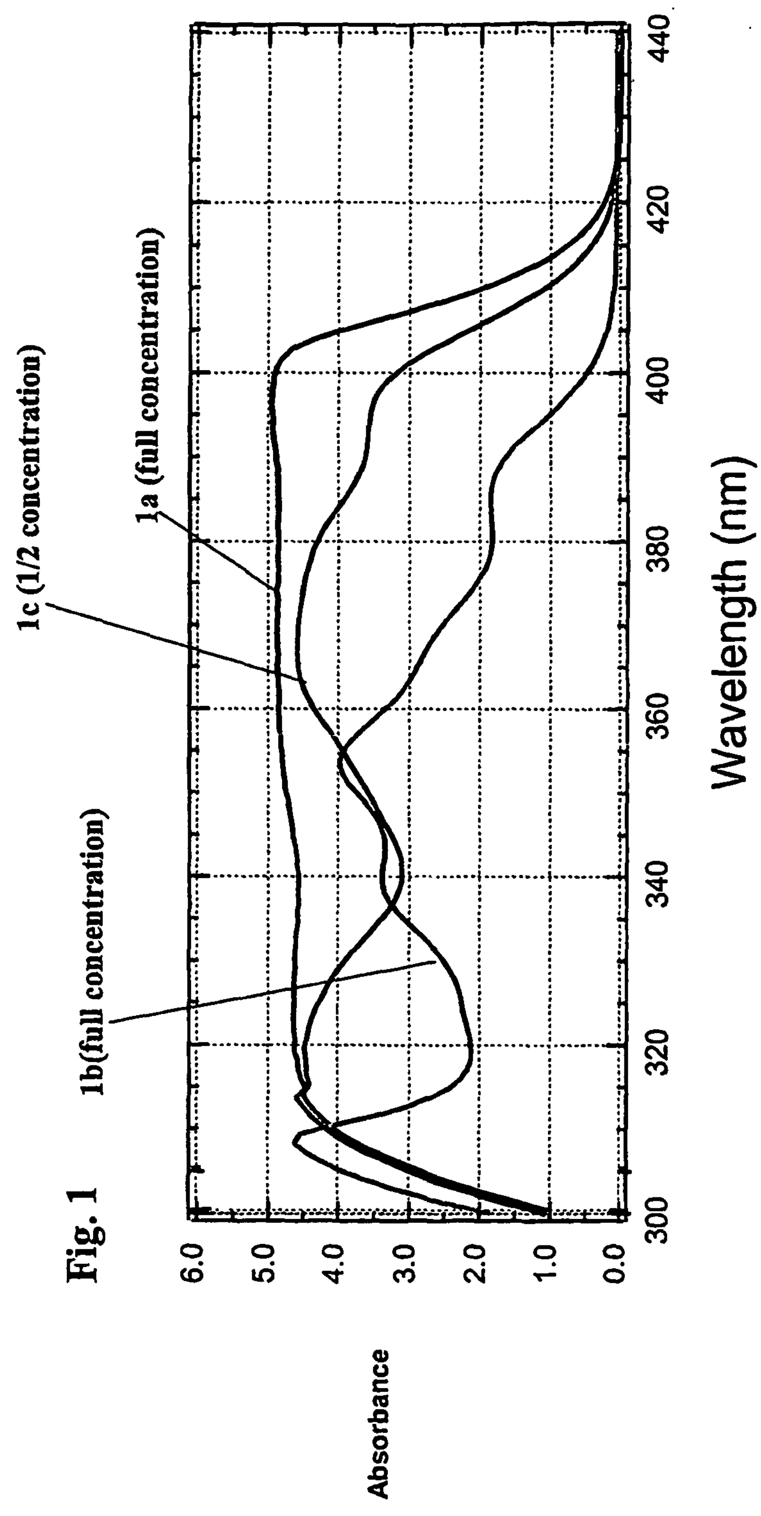
1c (1/2 concentration)
1a (full concentration)
1b(full concentration)
6.0
5.0
4.0
3.0
2.0
1.0
0.0
Absorbance
300
320
340
360
380
400
420
440
Wavelength (nm)

Fig. 2a

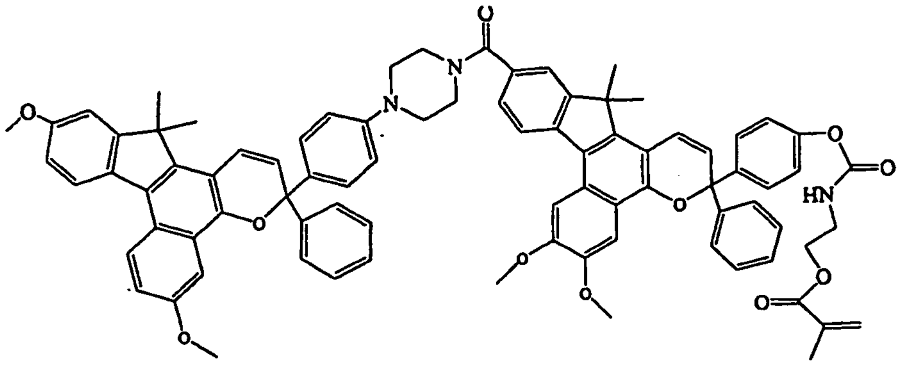

Fig. 2b

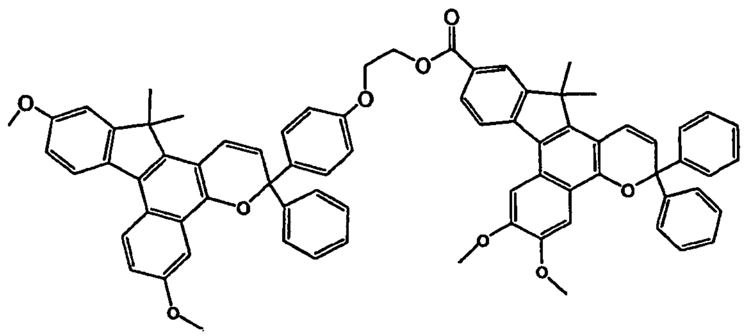

Fig. 3a

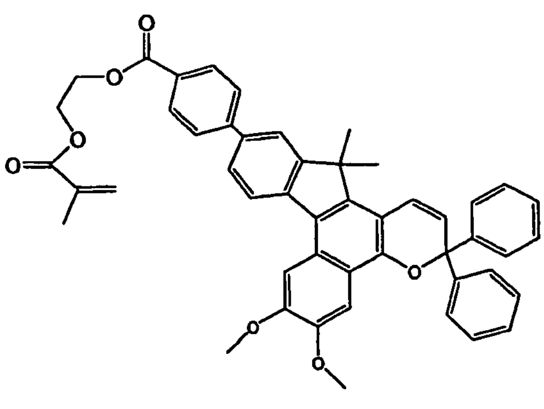

Fig. 3b

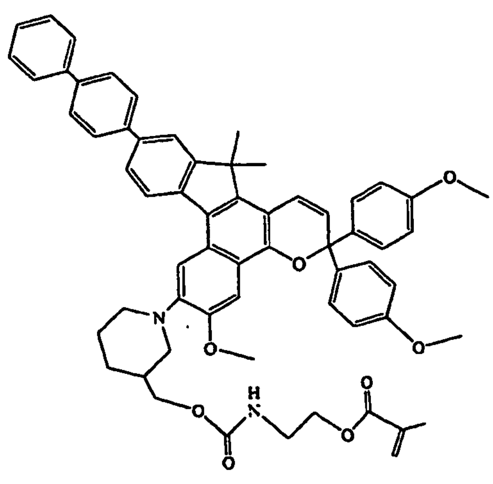

Fig. 4

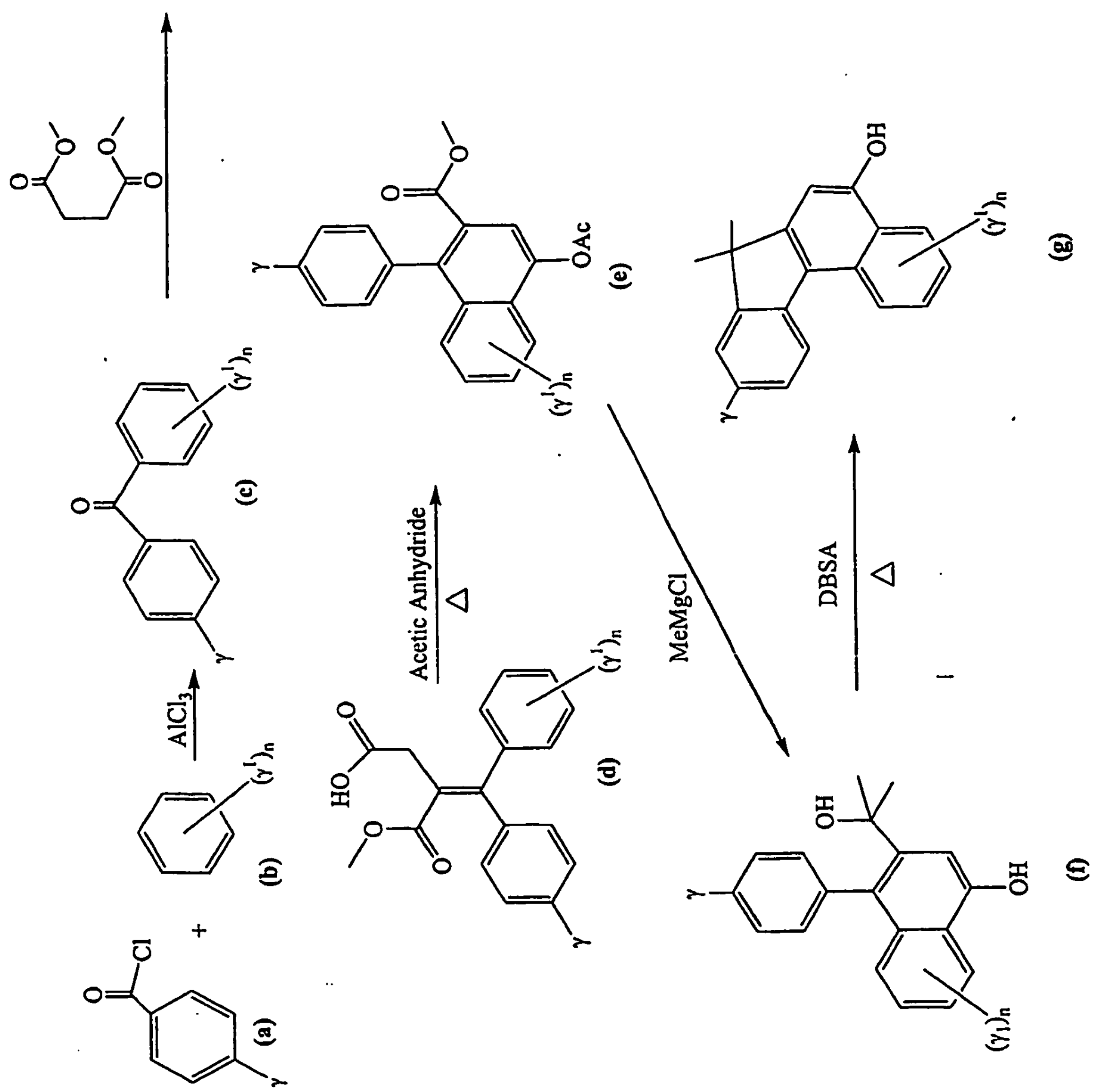


(a) (b) (c) (d) (e) (f) (g)

Fig. 5

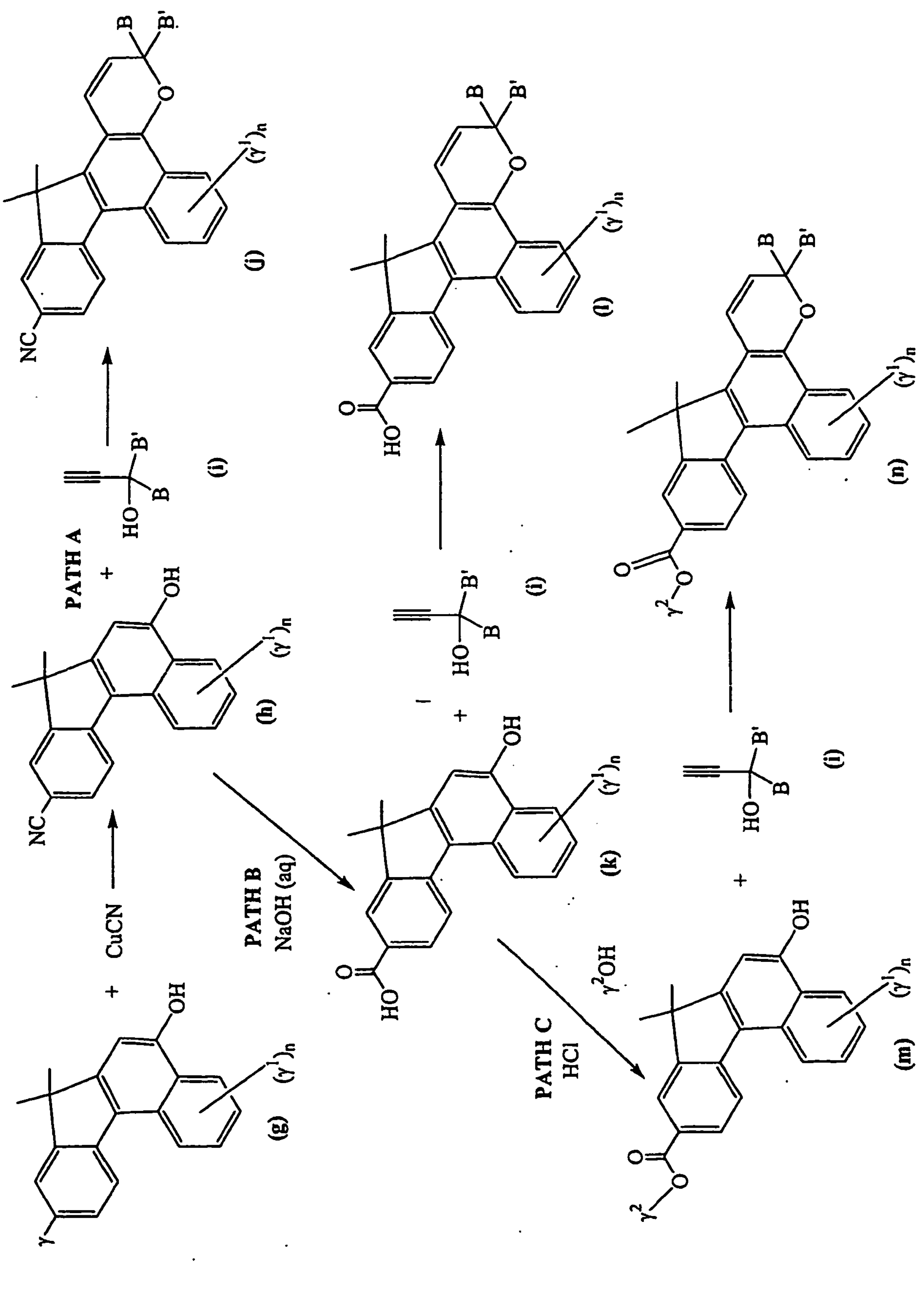

Fig. 6

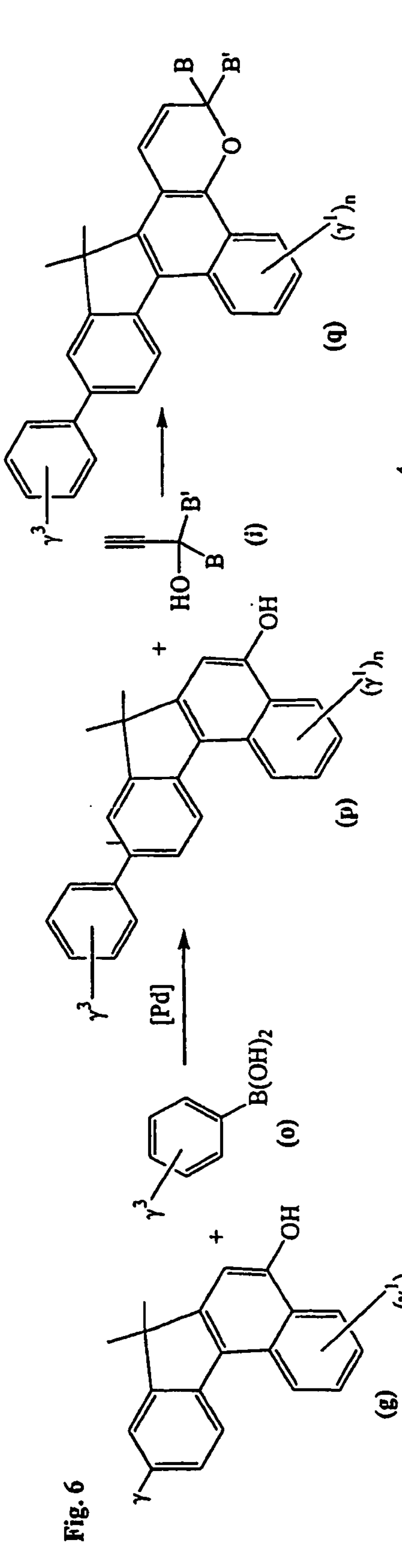


Fig. 7

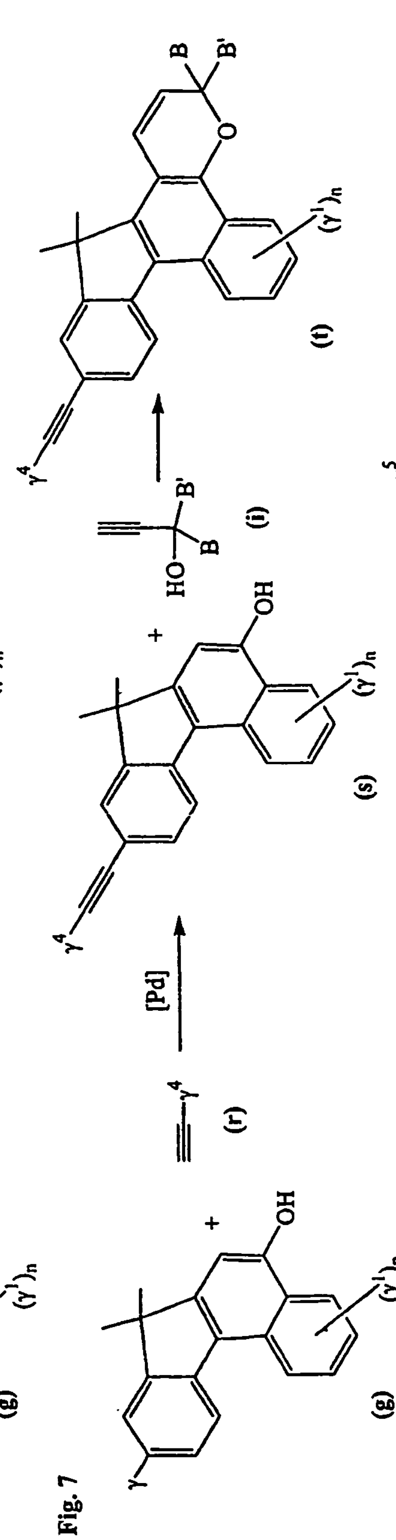


Fig. 8

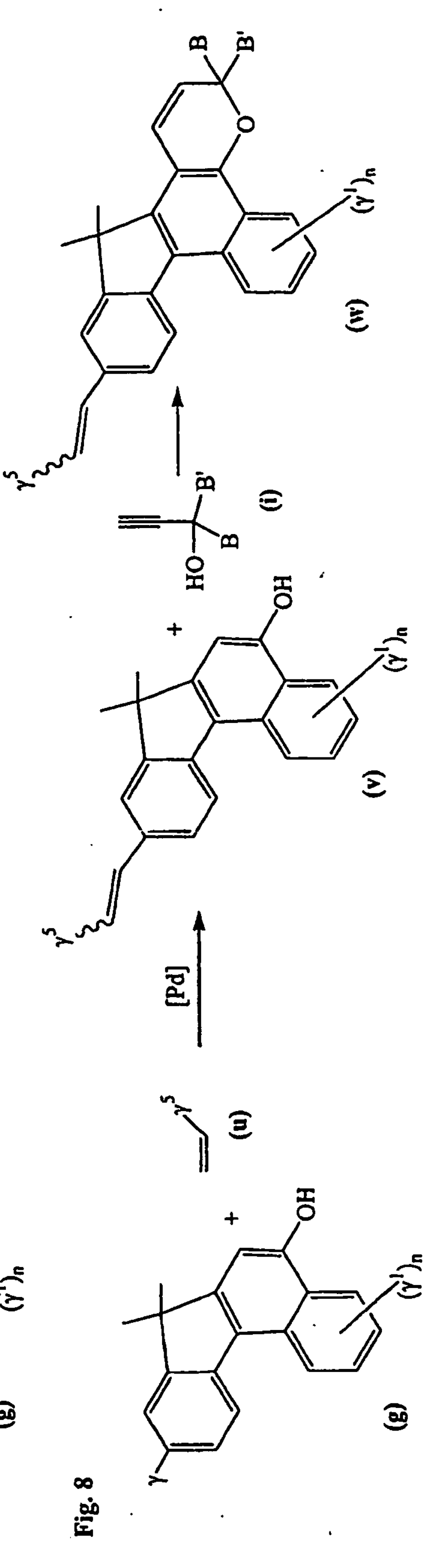

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6555028 B
- US 6113814 A
- US 5651923 A
- WO 9828289 A1
- WO 9923071 A1
- US 5645767 A
- US 6025026 A
- US 6150430 A
- US 20030165686 A
- US 6068797 A
- US 5458814 A